# EUROPEAN PATENT APPLICATION

(11) **EP 4 119 555 A1**
(43) Date of publication of application: **18.01.2023**
(21) Application number: 21771654.7
(22) Date of filing: 17.03.2021
(51) Int. Cl.: C07D 405/14, A61K 31/4439, A61P 3/00, A61P 9/00, A61P 1/16, A61P 25/28, C07D 401/14, C07D 413/14

(54) **GLP-1 RECEPTOR AGONIST, PHARMACEUTICAL COMPOSITION COMPRISING SAME, AND METHOD FOR PREPARING SAME**

(30) Priority: 18.03.2020 KR 20200033477
(71) Applicant: Lg Chem, Ltd., Seoul 07336 (KR)
(72) Inventor: KIM, Young Kwan, Daejeon 34122 (KR); JO, Min Mi, Daejeon 34122 (KR); PARK, Jun, Daejeon 34122 (KR)
(74) Representative: Hoffmann Eitle
(86) International application number: PCT/KR2021/003287
(87) International publication number: WO 2021/187886

(57) **Abstract**

The present invention relates to a novel compound useful as an agent for treatment or prophylaxis of various metabolic diseases such as obesity or diabetes and hyperlipidemia, by means of excellent GLP-1 agonist activity and an excellent DMPK profile, an isomer thereof or a pharmaceutically acceptable salt thereof, a pharmaceutical composition comprising the compound, and a method for preparing the compound.

## Description

### [Technical Field]

The present invention relates to a novel compound exhibiting GLP-1 receptor agonist activity, an isomer thereof, a pharmaceutically acceptable salt thereof, a pharmaceutical composition including the compound, and a method of preparing the compound.

### [Background Art]

Glucagon-like peptide-1 (GLP-1) is a polypeptide hormone secreted from intestinal L-cells after meals, and it may stimulate insulin secretion from pancreatic islet β cells, thereby stabilizing postprandial blood sugar levels. The GLP-1 binds to a GLP-1 receptor (GLP-1R). The GLP-1 receptor is a protein in the Class B receptor subclass of G protein-conjugated receptors (GPCR) regulating important physiological and pathophysiological processes. Its tertiary structure has not been identified, and since the GLP-1 receptor has a unique binding manner of determining affinity by binding the N-terminus thereof with a ligand, it is recognized as a drug target for which it is very difficult to develop a low-molecule ligand.

Exogenous administration of GLP-1 normalizes a blood sugar level in a Type 2 diabetes patient. Since the effect of GLP-1 on reduction in a blood sugar level varies according to a glucose concentration, the risk of hypoglycemia is greatly reduced while the blood sugar level is regulated. In addition, GLP-1-based drugs such as Byetta^{®} and Bydureon BCise^{®} (exenatide), Ozempic^{®} (semaglutide), Victoza^{®} (liraglutide), Adlyxin^{®} (lixisenatide); Tanzeum^{®} (albiglutide) and Trulicity^{®} (dulaglutide) are GLP-1 receptor agonists, which have been successfully sold in recent years, and it was identified that they provide blood sugar control which is effective for treating a type 2 diabetes patient in addition to providing weight loss effects, preservation of a beta-cell function, and relief of hypertension, hypoglycemia and/or hyperlipidemia.

However, since the above-described GLP-1 and GLP-1 receptor agonists may lack sufficient oral bioavailability that is required for a peptide-based oral drug, there is a need for a small-molecule agonist of the GLP-1 receptor with oral bioavailability.

### [Disclosure of Invention]

### [Technical Problem]

The present invention is directed to provide a novel compound having an activity as a GLP-1 agonist.

In addition, the present invention is directed to provide a pharmaceutical composition for preventing or treating a metabolic disease or neurodegenerative disease, which contains the novel compound as an active ingredient.

### [Solution to Problem]

The definition of each group used in the specification will be described in detail. Unless specified otherwise, each group has the following definition.

The "halo" used herein may be fluoro, chloro, bromo or iodo.

The "alkyl" used herein refers to a linear or branched aliphatic saturated hydrocarbon group, and specifically, may be C₁₋₆ alkyl, C₁₋₄ alkyl or C₁₋₃ alkyl. Examples of the alkyl may include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl or 2-ethylbutyl.

The "alkoxy" used herein refers to an oxygen group to which a single-bonded linear or branched saturated hydrocarbon is bonded to, and may be specifically C₁₋₆ alkoxy, C₁₋₄ alkoxy or C₁₋₃ alkoxy. Examples of the alkoxy may include methoxy, ethoxy, propoxy, n-butoxy, tert-butoxy or 1-methylpropoxy.

The "cycloalkyl" used herein refers to a cyclic single-bonded saturated hydrocarbon group, and is specifically C₃₋₈ cycloalkyl or C₃₋₆ cycloalkyl depending on the number of carbon atoms. Examples of the cycloalkyl may include cyclopropyl, cyclobutyl, cyclopentyl or cyclohexyl.

The "heterocycloalkyl" used herein refers to a cyclic single-bonded saturated hydrocarbon group including one or more heteroatoms such as N, O or S other than a carbon atom as a ring member, and may be monocyclic or a fused polycyclic ring. Specifically, the heterocycloalkyl may be 4- to 10-membered heterocycloalkyl, 4- to 7-membered heterocycloalkyl, or 4- to 6-membered heterocycloalkyl, which includes one or more, and preferably, one to three types of heteroatoms selected from the group consisting of N, O and S. Examples of heterocycloalkyl may include oxytanyl, aziridine, pyrrolidine, pyrrolidinyl, piperidinyl, piperazinyl, morpholinyl, tetrahydrofuranyl or tetrahydropyranyl.

The "aryl" used herein refers to an aromatic substituent having at least one ring, which has a covalent pi electron system, and may be monocyclic or a fused polycyclic ring (that is, rings each having adjacent pairs of carbon atoms). Specifically, such aryl may be C₄₋₁₀ aryl or C₆₋₁₀ aryl depending on the number of carbon atoms included in a ring, and for example, phenyl or naphthyl.

The "heteroaryl" used herein refers to an aromatic ring compound including one or more heteroatoms such as N, O or S, other than a carbon atom as a ring member, and may be monocyclic or a fused polycyclic ring. Specifically, the heteroaryl may be a 4- to 10-membered heteroaryl, a 4- to 7-membered heteroaryl, or a 4- to 6-membered heteroaryl having one or more, and preferably, one to three types of heteroatoms selected from the group consisting of N, O and S. Examples of heteroaryl may be furanyl, pyranyl, imidazoly, oxazolyl, isoxaazolyl, pyridyl, pyrazinyl, pyrimidyl, pyridazinyl, oxadiazolyl, thiadiazolyl, tetrazolyl, triazinyl, triazyl, or triazoyl, but the present invention is not limited thereto.

The "substituent" used herein may be one or more selected from the group consisting of a halo group, a nitrile group and a C₁₋₃ alkyl group.

Hereinafter, the present invention will be described in detail.

To achieve the above-described objects, the present invention provides a compound represented by Formula 1 below, an isomer thereof, or a pharmaceutically acceptable salt thereof:

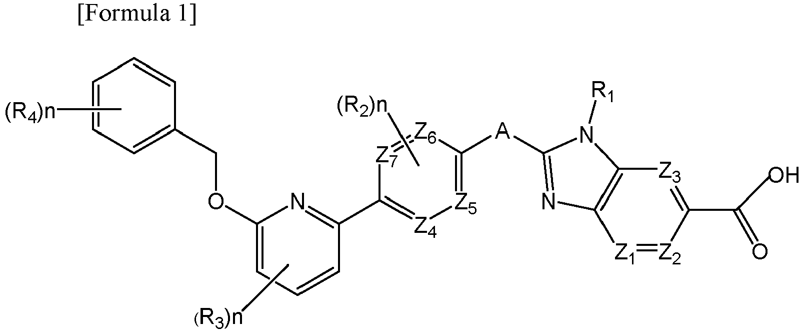

In this formula,
A is -(CH₂)ₘ-, -O- or -N(Rₐ)-, wherein m is an integer ranging from 1 to 3, and Rₐ is hydrogen or alkyl;
R₁ is (cycloalkyl)alkyl, (heterocycloalkyl)alkyl, (aryl)alkyl or (heteroaryl)alkyl;
R₂, R₃ or R₄ is each independently hydrogen, deuterium, halo, alkyl, alkoxy, alkylamine or a nitrile group;
n is an integer ranging from 1 to 4, wherein when n is an integer of 2 or more, each of R₂, R₃ and R₄ may be the same or different from each other;
Z₁, Z₂, Z₃, Z₄, Z₅, Z₆ or Z₇ each independently represents CH, CF, CCl, CBr, CI or N;
wherein, the alkyl, alkoxy, alkylamine, cycloalkyl, heterocycloalkyl, aryl, or heteroaryl is unsubstituted or substituted.

In an exemplary embodiment, A may be -CH₂-, -O- or -N(Rₐ)-.

In an exemplary embodiment, Rₐ may be hydrogen or C₁₋₃ alkyl.

In an exemplary embodiment, R₁ may be (C₃₋₈ cycloalkyl)C₁₋₃ alkyl, (4- to 10-membered heterocycloalkyl)C₁₋₃ alkyl, (C₆₋₁₀ aryl)alkyl or (4- to 10-membered heteroaryl)C₁₋₃ alkyl, wherein, the heterocycloalkyl or heteroaryl contains one to three types of heteroatoms selected from the group consisting of N, O and S.

In an exemplary embodiment, R₂, R₃ or R₄ is each independently hydrogen, deuterium, F, Cl, Br, I, C₁₋₃ alkyl, C₁₋₃ alkoxy, C₁₋₃ alkylamine or a nitrile group.

In an exemplary embodiment, n is an integer ranging from 1 to 3, wherein, when n is an integer of 2 or more, each of R₂, R₃ or R₄ may be the same or different from each other.

In an exemplary embodiment, Z₁, Z₂, Z₃, Z₄, Z₅, Z₆ or Z₇ each may be independently CH, CF or CCl.

In an exemplary embodiment, the alkyl, alkoxy, alkylamine, cycloalkyl, heterocycloalkyl, aryl, or heteroaryl may be unsubstituted, or halo- or C₁₋₃ alkylsubstituted.

In more exemplary embodiment, A may be -CH₂- or -O-.

In more exemplary embodiment, R₁ may be cyclopropylmethyl, cyclobutylmethyl, cyclopentylmethyl, cyclohexylmethyl, oxytanylmethyl, tetrahydrofuranylmethyl, tetrahydropyranylmethyl, oxazolylmethyl, benzyl, unsubstituted or propyl-substituted triazolylmethyl, or unsubstituted or ethyl-substituted imidazolylmethyl.

In more exemplary embodiment, R₂, R₃ or R₄ each is independently hydrogen, deuterium, F, Cl, or a nitrile group.

In more exemplary embodiment, n is 2, and each of R₂, R₃ or R₄ may be the same or different from each other.

Representative examples of compounds of Formula 1 according to the present invention may include the following compounds, but the present invention is not limited thereto:
1] 2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
2] (*S*)-2-(4-(6-((4-chloro-2-fluorobenzyl)oxy)pyridin-2-yl)benzyl)-1-(oxetan-2-ylmethyl)-1*H*-benzo[*d*]imidazole-6-carboxylic acid;
3] (*S*)-2-(4-(6-((4-cyano-2-fluorobenzyl)oxy)pyridin-2-yl)benzyl)-1-(oxetan-2-ylmethyl)-1*H*-benzo[d]imidazole-6-carboxylic acid;
4] 2-(4-(6-((4-cyano-2-fluorobenzyl)oxy)pyridin-2-yl)benzyl)-1-((1-ethyl-1*H*-imidazol-5-yl)methyl)-1*H*-benzo[*d*]imidazole-6-carboxylic acid;
5] 2-(4-(6-((4-cyano-2-fluorobenzyl)oxy)pyridin-2-yl)phenoxy)-1-((1-ethyl-1*H*-imidazol-5-yl)methyl)-1*H*-benzo[*d*]imidazole-6-carboxylic acid;
6] (*S*)-2-(4-(6-((4-cyano-2-fluorobenzyl)oxy)pyridin-2-yl)phenoxy)-1-(oxetan-2-ylmethyl)-1*H*-benzo[*d*]imidazole-6-carboxylic acid;
7] (*S*)-2-(4-(6-((4-chloro-2-fluorobenzyl)oxy)pyridin-2-yl)phenoxy)-1-(oxetan-2-ylmethyl)-1*H*-benzo[*d*]imidazole-6-carboxylic acid;
8] 2-(4-(6-((4-chloro-2-fluorobenzyl)oxy)pyridin-2-yl)benzyl)-1-(oxazol-5-ylmethyl)-1*H*-benzo[*d*]imidazole-6-carboxylic acid;
9] 2-(4-(6-((4-cyano-2-fluorobenzyl)oxy)pyridin-2-yl)phenoxy)-1-(oxazol-5-ylmethyl)-1*H*-benzo[*d*]imidazole-6-carboxylic acid;
10] (*S*)-2-(4-(6-((4-chloro-2-fluorobenzyl)oxy)pyridin-2-yl)-2-fluorobenzyl)-1-(oxetan-2-ylmethyl)-1*H*-benzo[*d*]imidazole-6-carboxylic acid;
11] 2-(4-(6-((4-chloro-2-fluorobenzyl)oxy)pyridin-2-yl)-2-fluorobenzyl)-1-(oxazol-5-ylmethyl)-1*H*-benzo[*d*]imidazole-6-carboxylic acid;
12] (*S*)-2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
13] (*R*)-2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
14] 2-(4-(6-((4-chloro-2-fluorobenzyl)oxy)pyridin-2-yl)benzyl)-l-((tetrahydro-2*H*-pyran-2-yl)methyl)-1*H*-benzo[*d*]imidazole-6-carboxylic acid;
15] (*S*)-2-(4-(6-((4-chloro-2-fluorobenzyl)oxy)pyridin-2-yl)-3-fluorobenzyl)-1-(oxetan-2-ylmethyl)-1*H*-benzo[*d*]imidazole-6-carboxylic acid;
16] (*S*)-2-(4-(6-((2-fluoro-4-(trifluoromethyl)benzyl)oxy)pyridin-2-yl)benzyl)-1-(oxetan-2-ylmethyl)-1*H*-benzo[*d*]imidazole-6-carboxylic acid;
17] (*S*)-2-(4-(6-((4-chloro-2-fluorobenzyl)oxy)pyridin-2-yl)-2-fluorophenoxy)-1-(oxetan-2-ylmethyl)-1*H*-benzo[*d*]imidazole-6-carboxylic acid;
18] (*S*)-2-(2-chloro-4-(6-((4-chloro-2-fluorobenzyl)oxy)pyridin-2-yl)phenoxy)-1-(oxetan-2-ylmethyl)-1*H*-benzo[*d*] imidazol-6-carboxylic acid;
19] (*S*)-2-(4-(6-((4-chloro-2-fluorobenzyl)oxy)pyridin-2-yl)-2,6-difluorophenoxy)-1-(oxetan-2-ylmethyl)-1*H*-benzo[*d*]imidazole-6-carboxylic acid;
20] (*S*)-2-(4-(6-((4-chloro-2-fluorobenzyl)oxy)pyridin-2-yl)-3-fluorophenoxy)-1-(oxetan-2-ylmethyl)-1*H*-benzo[*d*]imidazole-6-carboxylic acid;
21] 2-(4-(6-((2-fluoro-4-(trifluoromethyl)benzyl)oxy)pyridin-2-yl)benzyl)-1-(oxazol-5-ylmethyl)-1*H*-benzo[*d*]imidazole-6-carboxylic acid;
22] 2-(3-chloro-4-(6-((4-chloro-2-fluorobenzyl)oxy)pyridin-2-yl)benzyl)-1-(oxazol-5-ylmethyl)-1*H*-benzo[*d*]imidazole-6-carboxylic acid;
23] 2-(4-(6-((4-chloro-2-fluorobenzyl)oxy)pyridin-2-yl)-3-fluorobenzyl)-1-(oxazol-5-ylmethyl)-1*H*-benzo[*d*]imidazole-6-carboxylic acid;
24] 2-(4-(6-((4-chloro-2-fluorobenzyl)oxy)pyridin-2-yl)benzyl)-1-((4-propyl-4*H*-1,2,4-triazol-3-yl)methyl)-1*H*-benzo[*d*]imidazole-6-carboxylic acid;
25] 2-(4-(6-((2-fluoro-4-(trifluoromethyl)benzyl)oxy)pyridin-2-yl)benzyl)-1-((4-propyl-4*H*-1,2,4-triazol-3-yl)methyl)-1*H*-benzo[*d*]imidazole-6-carboxylic acid;
26] 2-(3-chloro-4-(6-((4-chloro-2-fluorobenzyl)oxy)pyridin-2-yl)benzyl)-1-((4-propyl-4*H*-1,2,4-triazol-3-yl)methyl)-1*H*-benzo[*d*]imidazole-6-carboxylic acid;
27] 2-(4-(6-((4-chloro-2-fluorobenzyl)oxy)pyridin-2-yl)-3-fluorobenzyl)-1-((4-propyl-4*H*-1,2,4-triazol-3-yl)methyl)-1*H*-benzo[*d*]imidazole-6-carboxylic acid;
28] 2-(4-(6-((4-chloro-2-fluorobenzyl)oxy)pyridin-2-yl)phenoxy)-1-((tetrahydrofuran-2-yl)methyl)-1*H*-benzo[*d*]imidazole-6-carboxylic acid;
29] (*S*)-2-(4-(6-((2-fluoro-4-(trifluoromethyl)benzyl)oxy)pyridin-2-yl)benzyl)-1-((tetrahydrofuran-2-yl)methyl)-1*H*-benzo[*d*]imidazole-6-carboxylic acid;
30] (*S*)-2-(4-(6-((4-chloro-2-fluorobenzyl)oxy)pyridin-2-yl)-2-fluorobenzyl)-1-((tetrahydrofuran-2-yl)methyl)-1H-benzo[*d*]imidazole-6-carboxylic acid;
31] (*S*)-2-(4-(6-((4-chloro-2-fluorobenzyl)oxy)pyridin-2-yl)-2-fluorophenoxy)-1-((tetrahydrofuran-2-yl)methyl)-1*H*-benzo[*d*]imidazole-6-carboxylic acid;
32] (*S*)-2-(2-chloro-4-(6-((4-chloro-2-fluorobenzyl)oxy)pyridin-2-yl)phenoxy)-1-((tetrahydrofuran-2-yl)methyl)-1*H*-benzo[*d*]imidazole-6-carboxylic acid;
33] (R)-2-(4-(6-((4-chloro-2-fluorobenzyl)oxy)pyridin-2-yl)benzyl)-1-((tetrahydrofuran-3-yl)methyl)-1*H*-benzo[*d*]imidazole-6-carboxylic acid;
34] (*S*)-2-(3-chloro-4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
35] (*S*)-2-(4-(6-((4-chloro-2-fluorobenzyl)oxy)pyridin-2-yl)benzyl)-1-((tetrahydrofuran-3-yl)methyl)-1*H*-benzo[*d*]imidazole-6-carboxylic acid;
36] (*S*)-2-(4-(6-((2-fluoro-4-(trifluoromethyl)benzyl)oxy)pyridin-2-yl)benzyl)-1-((tetrahydrofuran-3-yl)methyl)-1*H*-benzo[*d*]imidazole-6-carboxylic acid; and
37] (*S*)-2-(3-chloro-4-(6-((4-chloro-2-fluorobenzyl)oxy)pyridin-2-yl)benzyl)-1-((tetrahydrofuran-3-yl)methyl)-1*H*-benzo[*d*]imidazole-6-carboxylic acid.

The compounds included in the category of the Formula 1 described above may exhibit excellent GLP-1 receptor agonist activity, and thus, they exhibit a hypoglycemic effect and a positive effect on pancreatic beta cells so that they can be effectively used to treat various metabolic diseases.

Meanwhile, the compound represented by Formula 1 may have an asymmetric carbon center, and when it has an asymmetric carbon center, it may be present as an optical isomer, a partial optical isomer or a racemate thereof, and all types of isomers, including these, may be included in the category of the compounds according to one embodiment of the present invention. It is obvious that any type of isomer is also included in the category of the compounds of one embodiment. Hereinafter, the term "isomer" used herein may refer to the generic term for different compounds having the same molecular formula, the "optical isomer" may refer to the generic term for all stereoisomers that may exist for the compounds of one embodiment, including the same geometric isomers.

In the compound represented by Formula 1 according to one embodiment, it is understood that each substituent may be attached to a chiral center of the carbon atom. In addition, any asymmetric carbon atom of the compound of one embodiment may be present in any form of an (R)-, (S)- or (R, S)-configuration, and suitably, may be present in an (R)- or (S)-configuration, which are separate forms. In addition, the compound of one embodiment may be present in any form of possible isomers or mixtures thereof, for example, any form of pure geometric isomers, diastereomers, optical isomers, racemates, and mixtures thereof. In addition, when the compound of one embodiment has a double bond, each substituent binding to a double bond may be an E or Z configuration. In addition, when the compound of one embodiment contains a disubstituted cycloalkyl, each substituent of the cycloalkyl may have a cis or trans configuration.

Meanwhile, the term "pharmaceutically acceptable salt" used herein may be the generic term for salts preferred in terms of pharmaceutical, biological or other properties, which equivalently ensure the biological efficacy and properties of the compound of Formula 1 according to one embodiment. Non-limiting examples of the salt may include a salt to which an inorganic or organic base is added to the compound of Formula 1, or an acid addition salt. Examples of an organic acid that can form such an acid addition salt may include acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, or salicylic acid, and examples of an inorganic acid may include hydrochloric acid, hydrobromic acid, sulfuric acid, nitric acid or phosphoric acid.

The pharmaceutically acceptable salt of the compound of the above-described embodiment may be synthesized from a free base-type compound, or a basic or acidic residue derived therefrom by a conventional chemical method. In addition, a second pharmaceutically acceptable salt may be synthesized from a first pharmaceutically acceptable salt. As a specific example, the acid addition salt of the compound of one embodiment may be obtained by reacting the free base-type compound with a stoichiometric amount of an appropriate acid. Here, the reaction may be performed in water, an organic solvent or a mixture thereof, and specifically, in a non-aqueous medium such as ether, ethyl acetate, ethanol, isopropanol or acetonitrile. In addition, depending on the type of pharmaceutically acceptable salt, each type of salt may be obtained by a conventional reaction well known to those of ordinary skill in the art.

Meanwhile, according to another embodiment of the present invention, a pharmaceutical composition for preventing or treating a metabolic disease, which includes a compound represented by Formula 1 described above, an isomer thereof or a pharmaceutically acceptable salt thereof as an active ingredient is provided. As described above, since compounds represented by Formula 1 according to the present invention may exhibit a GLP-1 receptor (GLP-1R) activating activity, the pharmaceutical composition containing such a compound may have an effective blood sugar lowering action and a positive effect on pancreatic beta cells, and exhibit an effect of improving lipid metabolism, which is a chronic cardiovascular risk factor, and thus, they are effective in treating and/or preventing a disease associated with the activity of GLP-1 receptor, for example, a metabolic disease or neurodegenerative disease. The metabolic disease may be a disease selected from the group consisting of diabetes (preferably, type 2 diabetes), hypertension, hypoglycemia, hyperlipidemia (dyslipidemia), atherosclerosis, coronary artery disease, cardiovascular disorders, abnormal blood clotting, obesity, diabetic complications, diabetic retinopathy, liver disease, hepatobiliary disease, fatty liver, alcoholic steatohepatitis, chronic kidney disease, insulin resistance and impaired glucose tolerance. The neurodegenerative disease may be a disease selected from the group consisting of Parkinson's disease and Alzheimer's disease.

The pharmaceutical composition containing a compound represented by Formula 1 described above, an isomer thereof or a pharmaceutically acceptable salt thereof as an active ingredient may be used in the form of a conventional pharmaceutical preparation. That is, the pharmaceutical composition may be administered in various dosage forms such as oral or parenteral forms in actual clinical administration, and may be suitably administered in an oral form. In addition, the pharmaceutical composition may be formulated by further adding a pharmaceutically acceptable diluent or excipient such as a conventional filler, thickener, binder, wetting agent, disintegrant or surfactant depending on the dosage form.

A solid preparation for oral administration may be a tablet, a pill, powder, a granule or a capsule, and the solid preparation may be provided by being mixed with an active ingredient such as starch, calcium carbonate, sucrose, lactose or gelatin. In addition, other than an excipient, a lubricant such as magnesium stearate or talc may be used. In addition, a liquid preparation for oral administration may be a suspension, an oral liquid, an emulsion or a syrup, and the liquid preparation may contain various excipients, for example, a wetting agent, a sweetening agent, a flavoring agent or a preservative, in addition to a simple diluent such as water or a liquid paraffin. In addition, a preparation for parenteral administration may be a sterilized aqueous solution, a non-aqueous solvent, a suspension, an emulsion, a freeze-dried preparation or a suppository. The parenteral preparation may include a non-aqueous solvent, and as a suspending agent, propylene glycol, polyethylene glycol, a vegetable oil such as olive oil, or an injectable ester such as ethyl oleate may be used. As a base for the suppository, Witepsol, Macrogol, Tween 61, cacao butter, laurin or glycerogelatin may be used.

Moreover, the compound represented by Formula 1 of the pharmaceutical invention containing the present or pharmaceutically acceptable salt thereof as an active ingredient, and an isomer composition thereof may exhibit an effective amount in an administration range of approximately 0.1 to 1,000 mg. The composition may be administered at various doses and in various ways, for example, once a day or several times by dividing daily doses, depending on a patient's body weight, age, sex, health condition, a diet, administration time, an administration method, an excretion rate and the severity of a disease.

The present invention also provides a method of preparing the composition represented by Formula 1.

Hereinafter, to help understanding the present invention, the method of preparing the compound represented by Formula 1 will be described based on exemplary reaction schemes. However, those of ordinary skill in the art to which the present invention belongs may prepare the compound presented by Formula 1 by various methods based on the structure of Formula 1, and these methods will be construed as being included in the scope of the present invention. That is, the compound represented by Formula 1 may be prepared by a combination of various synthetic methods disclosed in the specification or in the related art, which will be understood as being included in the scope of the present invention, and the method of preparing the compound of Formula 1 is not limited to those to be described below.

In one exemplary embodiment, when A of the compound of Formula 1 according to the present invention is carbon, the compound of Formula 1 may be prepared by a preparation method including the following steps:
1) reacting a compound of Formula 2 below with a compound of Formula 3 below in the presence of a palladium catalyst to obtain a compound of Formula 4 below;
2) reacting the compound of Formula 4 below obtained in step 1) with a compound of Formula 5 below in the presence of a palladium catalyst, followed by hydrolyzing the resulting reaction product to obtain a compound of Formula 6 below; and
3) coupling the compound of Formula 6 below obtained in step 2) with a compound of Formula 7 below, followed by condensing and hydrolyzing the resulting reaction product to obtain the compound of Formula 1 below.

The bases used in steps 1) and 2) may be a material selected from the group consisting of a C₁₋₄ trialkylamine, diisopropylethylene amine (DIPEA, Hunig's base), pyridine, K₂CO₃, KOH, NaOH, Na₂CO₃, NaOAc, Ca(OH)₂, NaHCO₃, Cs₂CO₃ and LiOH, which may be used alone or in combination thereof, and ligands used in steps 1) and 2) may be triarylphosphines, trialkylphosphines, biaryl (dialkyl)phosphines, diphosphines, N-heterocyclic carbenes, cyclopentadienides, acetylacetonates, diamines, bipyridines, pyridines, DIOP, DiPAMP, BINAP, chiraphos or the like, but is not limited thereto.

The hydrolysis of step 2) may be performed using NaOH, KOH or LiOH at 0 to 80 °C, 10 to 70 °C, 20 to 60 °C, room temperature or 50 °C, but is not limited thereto. In addition, the reaction may be performed through stirring for a suitable time, which may be properly controlled.

The preparation method described above may be represented by Reaction Scheme 1:

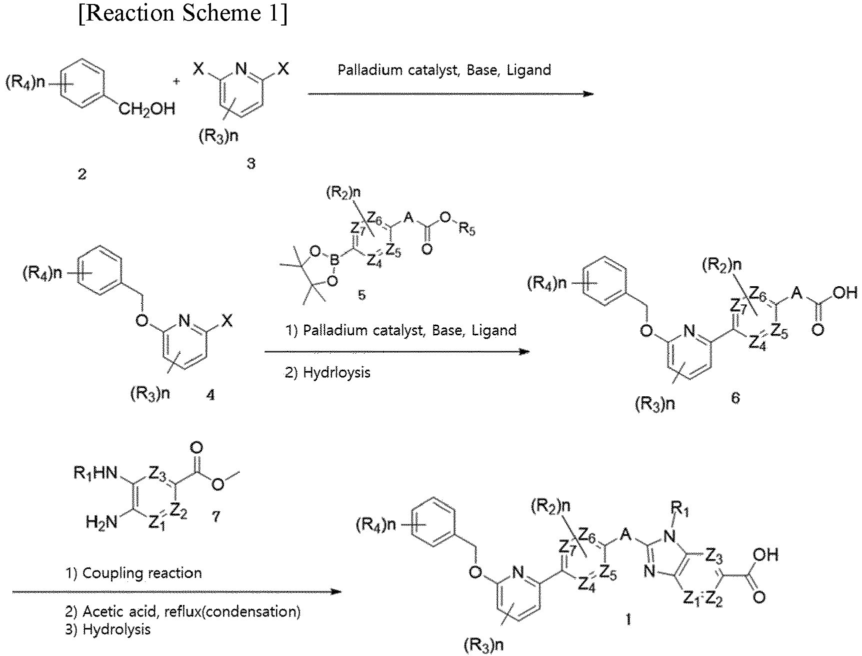

In an exemplary embodiment, when A of the compound of Formula 1 according to the present invention is oxygen, the compound of Formula 1 may be prepared by a preparation method including the following steps:
1') coupling a compound of Formula 4 below with a compound of Formula 8 below to obtain a compound of Formula 9 below; and
2') conducting a substitution reaction of the compound of Formula 9 below obtained in step 1') with a compound of Formula 10 below in the presence of a base, followed by hydrolyzing the resulting reaction product to obtain the compound of Formula 1 below.

All of bases, ligands and hydrolysis conditions used in the preparation method may be the same as those described in the preparation method used when A is carbon.

The preparation method described above may be represented by Reaction Scheme 2 below.

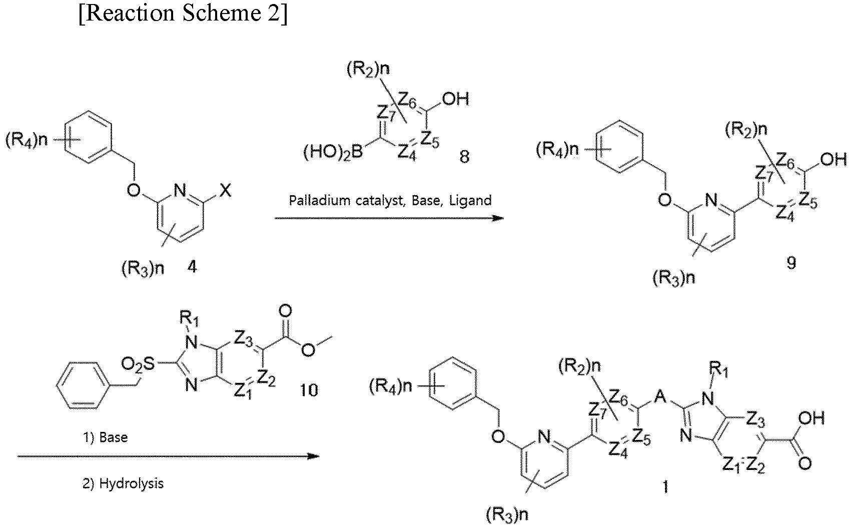

The compound of Formula 10 used in Reaction Scheme 2 may be prepared by a preparation method, which includes:
a) conducting a cyclization reaction of the compound of Formula 6 below, and then a substitution reaction with a benzyl halide to obtain a compound of Formula 11 below; and
b) conducting an oxidation reaction of the compound of Formula 11 obtained in step a) to obtain the compound of Formula 10.

The cyclization reaction of step a) may be performed using a suitable coupling agent. Examples of the coupling agent may include 1,1'-thiocarbonyldiimidzole (TCDI), 1,1'-carbonyldiimidzole (CDI) or the like, but is not limited thereto. In addition, the base used in step a) may be the same as described in the preparation method used when A is carbon.

In addition, the oxidation reaction of step b) may be performed using a suitable oxidant. Examples of the oxidant may include m-chloroperoxybenzoic acid (m-CPBA), hydrogen peroxide, potassium peroxymonosulfate, sodium periodate, sodium percarbonate, potassium permanganate, ruthenium oxide or the like, but is not limited thereto. The oxidant may be used in the presence of one or more additives, for example, KF, KHCO₃, NEt₃, AcONa, or the like. Those of ordinary skill in the art may recognize that an additive may be selected depending on the oxidant and reaction conditions to be used.

The preparation method described above may be represented by Reaction Scheme 3 below.

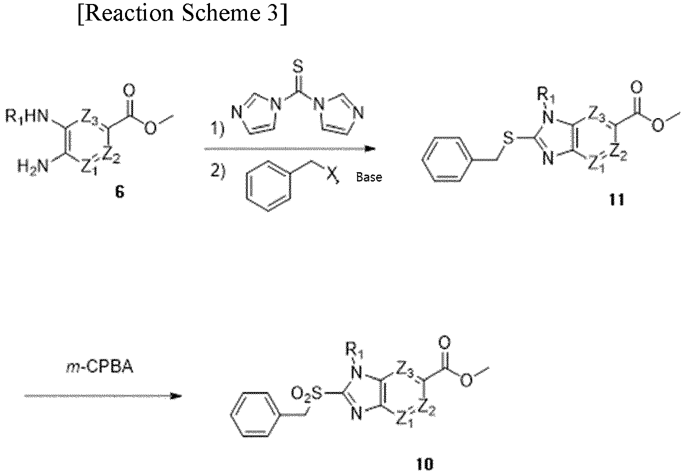

In an exemplary embodiment, when A of the compound of Formula 1 is nitrogen, the compound of Formula 1 may be prepared by a preparation method including the following steps:
1") coupling a compound of Formula 4 below with a compound of Formula 12 below to obtain a compound of Formula 13 below; and
2") conducting a substitution reaction of the compound of Formula 13 below obtained in step 1") with a compound of Formula 14 in the presence of acid catalyst, followed by hydrolyzing the resulting reaction product to obtain the compound of Formula 1 below.

The preparation method described above may be represented by Reaction Scheme 4 below.

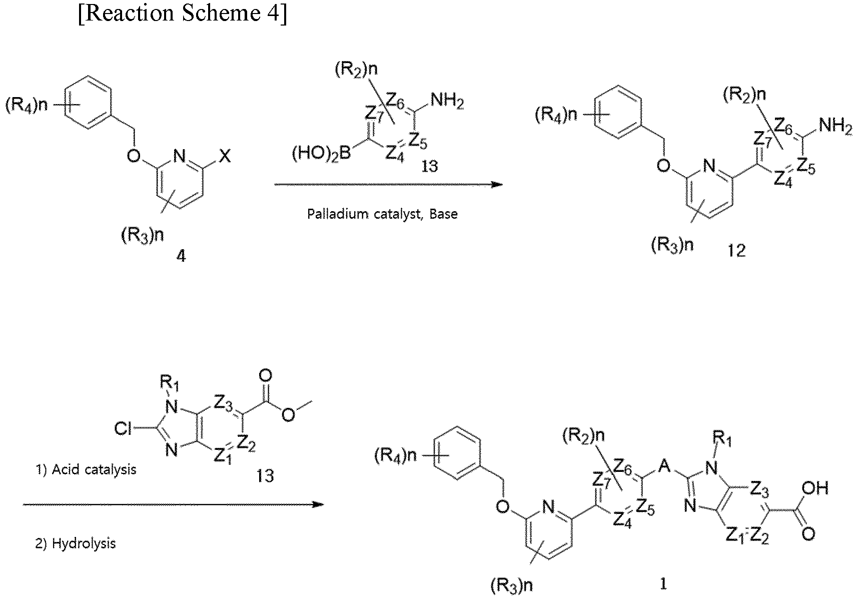

All of bases, ligands and hydrolysis conditions used in the preparation method may be the same as those described in the preparation method used when A is carbon.

In addition, the acid catalyst used in step 2") may be one selected from the group consisting of acetic acid, sulfuric acid, paratoluenesulfonic acid, hydrochloric acid, phosphoric acid and nitric acid, but is not limited thereto.

In Reaction Schemes 1 to 4, m, n, Rₐ, R₁, R₂, R₃, R₄, Z₁, Z₂, Z₃, Z₄, Z₅, Z₆ and Z₇ are as defined in Formula 1;
R₅ is alkyl; and
X is halo, and preferably, Cl, Br or I.

A compound not specifically described in the preparation method of the specification is a known compound, or a compound that can be easily synthesized from a known compound by a known synthetic method or a similar method thereto.

The compound of Formula 1 obtained through the above-described method may be isolated or purified by various methods including recrystallization, iontophoresis, silica gel column chromatography or ion exchange resin chromatography from a reaction product.

As described above, the compounds according to the present invention, and starting materials or intermediates for preparing the same may be synthesized by various methods, and the methods should be construed as being included in the scope of the present invention with respect to the preparation of the compound represented by Formula 1.

### [Advantageous Effects of Invention]

A novel compound according to the present invention is useful as an agent for treating or preventing obesity or various metabolic diseases such as diabetes and hyperlipidemia due to excellent GLP-1 agonist activity and an excellent DMPK profile.

### [Mode for the Invention]

Herein after, the present invention will be described in further detail with reference to the following examples and experimental examples. However, the following examples and experimental examples are only exemplary, and merely provided to more easily understand the present invention, and the present invention is not limited thereto.

In the examples, the following abbreviations are defined to represent the following materials.
DMF: dimethylformamide
THF: tetrahydrofuran
TEA: triethylamine
EtOAc: ethyl acetate
MgSO₄: magnesium sulfate
MPLC: Medium-Pressure Liquid Chromatography
Pd/C: palladium on carbon
AcOH: acetic acid
HCl: hydrochloric acid
CS₂: carbon disulfide
NaH: sodium hydride
DCM: dichloromethane
mCPBA: 3-chloroperbenzoic acid
NaHCO₃: sodium bicarbonate
*t*-BuOK: potassium *tert*-butoxide
Cs₂CO₃: cesium carbonate
K₂CO₃: potassium carbonate
BINAP: (2,2'-bis(diphenylphosphino)-1,1'-binaphthyl)
Pd₂(dba)₃: tris(dibenzylideneacetone)dipalladium
Fe: iron
NH₄Cl: ammonium chloride
CDI: carbodiimidazole
DCE: 1,2-dichloroethane
POCl₃: phosphoryl chloride
NaOH: sodium hydroxide
H₂O: water
MeOH: methanol
HOBt: hydroxybenzotriazole
EDC: 1-ethyl-3-(3-dimethylaminopropyl)carbodiimide
Dppf: 1,1'-bis(diphenylphosphino)ferrocene
Na₂SO₄: sodium sulfate
NMR: Nuclear Magnetic Resonance
HATU: (1-[bis(dimethylamino)methylidene]-1H-1,2,3-triazolo[4,5-b]pyridinium 3-oxide hexafluoro phosphate

### [Preparation Example 1: Preparation of 2-chloro-6-((4-chloro-2-fluorobenzyl)oxy)pyridine]

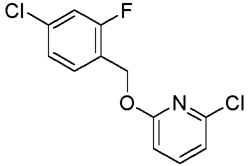

### 2-chloro-6-((4-chloro-2-fluorobenzyl)oxy)pyridine

(4-chloro-2-fluorophenyl)methanol (1236 mg, 7 mmol) was dissolved in 10 mL of DMF, and NaH (60% dispersion in mineral oil, 420 mg, 10.5 mmol) was added at 0 °C. After stirring at the same temperature for 10 minutes, 2,6-dichloropyridine (1036 mg, 7 mmol) was added and then stirred for 2 hours at room temperature. Water was added after the reaction, followed by extraction with EtOAc. An organic layer was dried with Na₂SO₄, concentrated under reduced pressure and purified by using MPLC, thereby obtaining 2-chloro-6-((4-chloro-2-fluorobenzyl)oxy)pyridine (1.06 g, 4.21 mmol, 60%).

¹H NMR (500 MHz, CDCl₃) δ 7.54 (t, *J* = 8.0 Hz, 1H), 7.46 (t, *J* = 8.0 Hz, 1H), 7.16 - 7.11 (m, 2H), 6.93 (d, *J* = 7.5 Hz, 1H), 6.70 (d, *J* = 8.0 Hz, 1H), 5.39 (s, 2H); LC-MS(ESI): 272.14 [M+H]⁺

### [Preparation Example 2: Preparation of 2-(4-(6-((4-chloro-2-fluorobenzyl)oxy)pyridin-2-yl)phenyl)acetic acid]

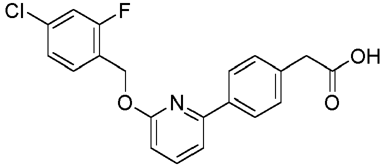

### 2-(4-(6-((4-chloro-2-fluorobenzyl)oxy)pyridin-2-yl)phenyl)acetic acid

The 2-chloro-6-((4-chloro-2-fluorobenzyl)oxy)pyridine (520 mg, 2.06 mmol) obtained in Preparation Example 1, Pd(dppf)Cl₂-DCM (163 mg, 0.2 mmol), Cs₂CO₃ (1879 mg, 5.77 mmol), and ethyl 2-(4-(4,4,5,5-tetramethyl-1,3,2-dioxabororan-2-yl)phenyl)acetate (707 mg, 2.43 mmol) were dissolved in 20 mL of THF/H₂O (9/1), followed by nitrogen substitution. The reactants were stirred for 5 hours at 85 °C. After the reaction was completed, water was added, and then extraction was performed using EtOAc.

An organic layer was dried with Na₂SO₄ and concentrated under reduced pressure, thereby obtaining an intermediate, ethyl 2-(4-(6-((4-chloro-2-fluorobenzyl)oxy)pyridin-2-yl)phenyl)acetate. The resulting intermediate was dissolved in 8 mL of MeOH without additional purification, and 8 mL of IN NaOH was added. The reactants were stirred for 22 hours at 50 °C. After the reaction was completed, the resulting solution was adjusted to pH 4 by using IN HCl, followed by extraction with EtOAc. An organic layer was dried with Na₂SO₄ and concentrated under reduced pressure, and purified by using MPLC, thereby obtaining 2-(4-(6-((4-chloro-2-fluorobenzyl)oxy)pyridin-2-yl)phenyl)acetic acid (358 mg, 0.962 mmol, 47%).

¹H NMR (500 MHz, MeOD) δ 8.00 (d, *J* = 8.5 Hz, 2H), 7.72 (t, *J* = 8.0 Hz, 1H), 7.55 (t, *J* = 8.0 Hz, 1H), 7.47 (d, *J* = 7.5 Hz, 1H), 7.38 (d, *J* = 8.5 Hz, 2H), 7.25 - 7.17 (m, 2H), 6.76 (d, *J* = 8.5 Hz, 1H), 5.53 (s, 2H), 3.67 (s, 2H); LC-MS(ESI): 372.28 [M+H]⁺

### [Preparation Example 3: Preparation of methyl 4-nitro-3(((tetrahydrofuran-2-yl)methyl)amino)benzoate]

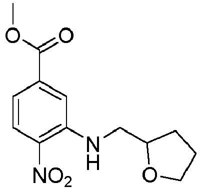

### methyl 4-nitro-3-(((tetrahydrofuran-2-yl)methyl)amino)benzoate

methyl 3-fluoro-4- nitrobenzoate (1 g, 5.02 mmol) was dissolved in DMF, (tetrahydrofuran-2-yl)methaneamine (660 mg, 6.53 mmol) and K₂CO₃ (693 mg, 5.02 mmol) were added at room temperature. The reactants were stirred for 4 hours at 50 °C. Water was added, and extraction was performed by using DCM. An organic layer was dried with Na₂SO₄, concentrated under reduced pressure, and purified by MPLC, thereby obtaining methyl 4-nitro-3(((tetrahydrofuran-2-yl)methyl)amino)benzoate (1.38 g, 4.92 mmol, 98%).

¹H NMR (500 MHz, CDCl₃) δ 8.25 - 8.12 (m, 2H), 7.57 (d, *J* = 1.5 Hz, 1H), 7.22 (dd, *J=* 9.0, 1.5 Hz, 1H), 4.25 - 4.18 (m, 1H), 4.00 - 3.91 (m, 4H), 3.87 - 3.77 (m, 1H), 3.56 - 3.47 (m, 1H), 3.44 - 3.34 (m, 1H), 2.16 - 2.04 (m, 1H), 2.02 - 1.91 (m, 2H), 1.77 - 1.64 (m, 1H).

### [Preparation Example 4: Preparation of methyl 4-amino-3-(((tetrahydrofuran-2-yl)methyl)amino)benzoate]

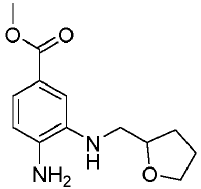

### methyl 4-amino-3-(((tetrahydrofuran-2-yl)methyl)amino)benzoate

The methyl 4-nitro-3(((tetrahydrofuran-2-yl)methyl)amino)benzoate (1.38 g, 4.92 mmol) obtained in Preparation Example 3 was dissolved in 50 mL of MeOH/DCM (1/1), 10% Pd/C (138 mg) was added and stirred for 17.5 hours under hydrogen at room temperature. After the reaction was completed, Pd/C was filtered through Celite and then concentration under reduced pressure was performed, thereby obtaining methyl 4-amino-3-(((tetrahydrofuran-2-yl)methyl)amino)benzoate (1.23 g, 4.92 mmol, 100%).

¹H NMR (400 MHz, CDCl₃) δ 7.46 (dd, *J* = 7.8, 1.8 Hz, 1H), 7.34 (d, *J* = 1.8 Hz, 1H), 6.67 (d, *J* = 8.4 Hz, 1H), 4.20 (qd, *J* = 7.3, 3.3 Hz, 1H), 3.92 - 3.77 (m, 7H), 3.26 (dd, *J* = 11.9, 3.2 Hz, 1H), 3.09 (dd, *J* = 11.9, 8.2 Hz, 1H), 2.10 - 2.03 (m, 1H), 1.98 - 1.91 (m, 2H), 1.74 - 1.67 (m, 1H); LC-MS(ESI): 251.28 [M+H]⁺

### [Preparation Example 5: Preparation of methyl 2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate]

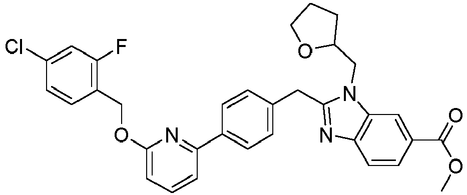

### methyl 2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate

The methyl 4-amino-3-(((tetrahydrofuran-2-yl)methyl)amino)benzoate (121 mg, 0.48 mmol) obtained in Preparation Example 4 and the 2-(4-(6-((4-chloro-2-fluorobenzyl)oxy)pyridin-2-yl)phenyl)acetic acid (150 mg, 0.40 mmol) obtained in Preparation Example 2 were dissolved in 2 mL of DMF, and EDC (155 mg, 0.81 mmol) and HOBt (108.9 mg, 0.81 mmol) were added and then stirred for 15.5 hours at room temperature. After the reaction was completed, water was added, and extraction was performed with EtOAc. An organic layer was dried with Na₂SO₄ and concentrated under reduced pressure, thereby obtaining an intermediate, methyl 4-(2-(4-(6-((4-chloro-2-fluorobenzyl)oxy)pyridin-2-yl)phenyl)acetamido)-3-(((tetrahydrofuran-2-yl)methyl)amino)benzoate. The resulting intermediate was dissolved in 20 mL of acetic acid without additional purification, and stirred for 2 hours at 120 °C. Water was added, and extraction was performed with EtOAc. An organic layer was dried with Na₂SO₄, concentrated under reduced pressure and purified by using MPLC, thereby obtaining methyl 2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate (135 mg, 0.23 mmol, 48%).

¹H NMR (500 MHz, CDCl₃) δ 8.08 (s, 1H), 7.98 - 7.94 (m, 3H), 7.76 (d, *J*= 8.5 Hz, 1H), 7.63 (t, *J* = 8.0 Hz, 1H), 7.46 (t, *J* = 8.2 Hz, 1H), 7.35 - 7.31 (m, 3H), 7.13 - 7.11 (m, 2H), 6.73 (d, *J* = 8.2 Hz, 1H), 5.51 (s, 2H), 4.51 (dd, *J* = 36.6, 15.9 Hz, 2H), 4.21 (dd, *J* = 14.2, 2.3 Hz, 1H), 4.17 - 4.07 (m, 2H), 3.94 (s, 3H), 3.86 (dd, *J* = 15.1, 6.9 Hz, 1H), 3.75 - 3.71 (m, 1H), 2.04 - 1.99 (m, 1H), 1.89 - 1.82 (m, 2H), 1.57 - 1.50 (m, 1H); LC-MS(ESI): 586.32 [M+H]⁺

### [Preparation Example 6: Preparation of methyl (S)-4-nitro-3-((oxetan-2-ylmethyl)amino)benzoate]

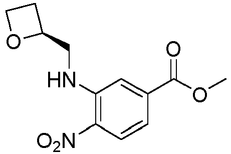

### methyl (S)-4-nitro-3-((oxetan-2-ylmethyl)amino)benzoate

(*S*)-oxetan-2-ylmethylamine (539 mg, 6.19 mmol) was dissolved in DMF (7 mL) and THF (~10 mL), and TEA (2.59 mL, 18.56 mmol) and methyl 3-fluoro-4-nitrobenzoate (1.23 g, 6.19 mmol) were added, followed by stirring for 16 hours under nitrogen at room temperature. After the reaction was completed, a reaction solvent was concentrated under reduced pressure. Water was added, extraction was performed by using EtOAc, and drying was performed with MgSO₄, followed by concentration under reduced pressure. The resulting concentrate was purified by using MPLC, thereby obtaining methyl (S)-4-nitro-3-((oxetan-2-ylmethyl)amino)benzoate (813 mg, 3.05 mmol, 49%).

¹H NMR (500 MHz, CDCl₃) δ 8.36 (br s, 1H), 8.24 (d, *J=* 8.5 Hz, 1H), 7.63 (d, *J* = 1.5 Hz, 1H), 7.28 (d, *J* = 1.5 Hz, 1H), 5.16-5.20 (m, 1H), 4.73-4.76 (m, 1H), 4.61-4.65 (m, 1H), 3.94 (s, 3H), 3.62-3.65 (m, 2H), 2.76-2.80 (m, 1H), 2.59-2.75 (m, 1H); LC-MS(ESI): 267.26 [M+H]⁺

### [Preparation Example 7: Preparation of methyl (S)-4-amino-3-((oxetan-2-ylmethyl)amino)benzoate]

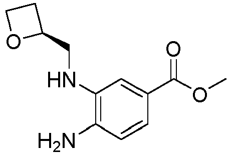

### methyl (S)-4-amino-3-((oxetan-2-ylmethyl)amino)benzoate

The methyl (*S*)-4-nitro-3-((oxetan-2-ylmethyl)amino)benzoate (813 mg, 3.05 mmol) obtained in Preparation Example 6 was dissolved in THF (13 mL), 10% Pd/C (325 mg) was added, and stirred for 4 hours under hydrogen at room temperature. After the reaction was completed, the resulting reaction product was filtered through Celite using EtOAc and concentrated under reduced pressure, thereby obtaining methyl (S)-4-amino-3-((oxetan-2-ylmethyl)amino)benzoate (720 mg, 3.05 mmol, 100%).

¹H NMR (500 MHz, CDCl₃) δ 7.48 (dd, *J* = 8.0, 1.5 Hz, 1H), 7.38 (d, *J* = 1.5 Hz, 1H), 6.68 (d, *J* = 8.0 Hz, 1H), 5.12-5.10 (m, 1H), 4.77-4.73 (m, 1H), 4.64-4.59 (m, 1H), 3.86 (s, 5H), 3.53 (br s, 1H), 3.46-3.42 (m, 1H), 3.37-3.34 (m, 1H), 2.78-2.74 (m, 1H), 2.61-2.57 (m, 1H); LC-MS(ESI): 237.27 [M+H]⁺

### [Preparation Example 8: Preparation of 4-(((6-chloropyridin-2-yl)oxy)methyl)-3-fluorobenzonitrile]

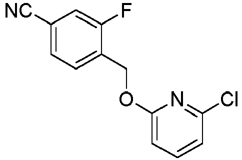

### 4-(((6-chloropyridin-2-yl)oxy)methyl)-3-fluorobenzonitrile

2,6-dichloropyridine (1.18 g, 7.94 mmol) was dissolved in 1,4-dioxane (20 mL), (2-fluoro-4-isocyanophenyl)methanol (600 mg, 3.97 mmol) and Cs₂CO₃ (2.59 g, 7.94 mmol) were added, and then nitrogen substitution was sufficiently performed. BINAP (124 mg, 0.198 mmol) and Pd₂(dba)₃ (109 mg, 0.119 mmol) were added, and nitrogen substitution was performed again, followed by stirring for 24 hours at 90 °C. After the reaction was completed, the resulting reaction product was cooled to room temperature, filtered through Celite by using EtOAc, and then concentrated under reduced pressure. The resulting concentrate was purified by using MPLC, thereby obtaining 4-(((6-chloropyridin-2-yl)oxy)methyl)-3-fluorobenzonitrile (678 mg, 2.58 mmol, 65%).

¹H NMR (500 MHz, CDCl₃) δ 7.65 (t, *J* = 7.3 Hz, 1H), 7.57 (t, *J* = 8.6 Hz, 1H), 7.48-7.47 (m, 1H), 7.40-7.38 (m, 1H), 6.96 (d, *J* = 7.5 Hz, 1H), 6.74 (d, *J* = 8.5 Hz, 1H), 5.49 (s, 2H).

### [Preparation Example 9: Preparation of ethyl 2-(4-(6-((4-cyano-2-fluorobenzyl)oxy)pyridin-2-yl)phenyl)acetate]

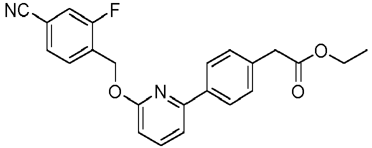

### ethyl 2-(4-(6-((4-cyano-2-fluorobenzyl)oxy)pyridin-2-yl)phenyl)acetate

The 4-(((6-chloropyridin-2-yl)oxy)methyl)-3-fluorobenzonitrile (160 mg, 0.609 mmol) obtained in Preparation Example 8 was dissolved in 1.4-dioxane/water (2 mL/2 mL), and ethyl 2-(4-(4,4,5,5-tetramethyl-1,3,2-dioxabororan-2-yl)phenyl)acetate (194 mg, 0.670 mmol) and Na₂CO₃ (129 mg, 1.218 mmol) were added, followed by sufficient nitrogen substitution. Pd(PPh₃)₄ (36 mg, 0.030 mmol) was added, and nitrogen substitution was performed again, followed by stirring for 24 hours at 90 °C. After the reaction was completed, the resulting reaction product was cooled to room temperature, filtered through Celite by using EtOAc, and concentrated under reduced pressure. The resulting concentrate was purified by using MPLC, thereby obtaining ethyl 2-(4-(6-((4-cyano-2-fluorobenzyl)oxy)pyridin-2-yl)phenyl)acetate (132 mg, 0.338 mmol, 55%).

¹H NMR (500 MHz, CDCl₃) δ 7.94 (d, *J* = 8.5 Hz, 2H), 7.67-7.65 (m, 2H), 7.45 (d, *J* = 8.5 Hz, 1H), 7.40-7.36 (m, 4H), 6.78 (d, *J* = 8.5 Hz, 1H), 5.63 (s, 2H), 4.17 (q, *J* = 7.3 Hz, 2H), 3.66 (s, 2H), 1.27 (t, *J* = 7.0 Hz, 3H); LC-MS(ESI): 391.34 [M+H]⁺

### [Preparation Example 10: Preparation of methyl 3-(((1-ethyl-1H-imidazol-5-yl)methyl)amino)-4-nitrobenzoate]

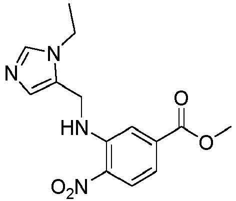

### methyl 3-(((1-ethyl-1H-imidazol-5-yl)methyl)amino)-4-nitrobenzoate

Methyl 3-fluoro-4-nitrobenzoate (700 mg, 3.52 mmol) was dissolved in THF (12 mL), and (1-ethyl-1*H*-imidazol-5-yl)methaneamine dihydrochloride (696 mg, 3.52 mmol) and TEA (1.47 mL, 10.55 mmol) were added, followed by stirring for 24 hours at 80 °C under nitrogen. After the reaction was completed, the resulting reaction product was cooled to room temperature, and then a reaction solvent was concentrated under reduced pressure. Water was added, extraction was performed by using EtOAc, and drying was performed with MgSO₄, followed by concentration under reduced pressure. The resulting concentrate was purified by using MPLC, thereby obtaining methyl 3-(((1-ethyl-1*H*-imidazol-5-yl)methyl)amino)-4-nitrobenzoate (442 mg, 1.452 mmol, 41%).

¹H NMR (500 MHz, CDCl₃) δ 8.25 (d, *J* = 9.0 Hz, 1H), 7.94 (br s, 1H), 7.69 (d, *J* = 1.5 Hz, 1H), 7.57 (s, 1H), 7.34 (dd, *J* = 9.0, 1.5 Hz, 1H), 7.11 (s, 1H), 4.54 (d, *J* = 5.0 Hz, 2H), 3.99 (m, 5H), 1.48 (t, *J* = 7.5 Hz, 3H); LC-MS(ESI): 305.1 [M+H]⁺

### [Preparation Example 11: Preparation of methyl 4-amino-3-(((1-ethyl-1H-imidazol-5-yl)methyl)amino)benzoate]

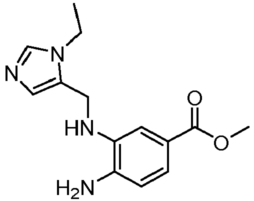

### methyl 4-amino-3-(((1-ethyl-1H-imidazol-5-yl)methyl)amino)benzoate

The methyl 3-(((1-ethyl-1*H*-imidazol-5-yl)methyl)amino)-4-nitrobenzoate (440 mg, 1.446 mmol) obtained in Preparation Example 10 was dissolved in MeOH/H₂O (6 mL/2 mL), and Fe (242 mg, 4.34 mmol) and NH₄Cl (1.55 g, 28.9 mmol) were added, and then stirring was performed for 3 hours at 80 °C under nitrogen. After the reaction was completed, the resulting reaction product was cooled to room temperature, extraction was performed by using EtOAc, drying was performed with MgSO₄, and concentration was performed under reduced pressure. The resulting concentrate was purified by using MPLC, thereby obtaining methyl 4-amino-3-(((1-ethyl-1*H*-imidazol-5-yl)methyl)amino)benzoate (254 mg, 0.925 mmol, 64%).

¹H NMR (400 MHz, CDCl₃) δ 7.53-7.50 (m, 2H), 7.46 (d, *J* = 1.6 Hz, 1H), 7.03 (s, 1H), 6.70 (d, *J* = 8.4 Hz, 1H), 4.27 (s, 2H), 4.01 (q, *J* = 7.4 Hz, 2H), 3.87 (s, 3H), 1.45 (t, *J* = 7.2 Hz, 3H); LC-MS(ESI): 275.1 [M+H]⁺

### [Preparation Example 12: Preparation of methyl 3-((1-ethyl-1H-imidazol-5-yl)methyl)-2-oxo-2,3-dihydro-1H-benzo[d]imidazole-5-carboxylate]

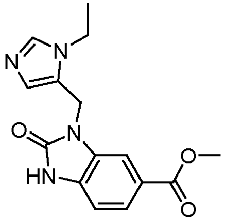

### methyl 3-((1-ethyl-1H-imidazol-5-yl)methyl)-2-oxo-2,3-dihydro-1H-benzo[d]imidazole-5-carboxylate

The methyl 4-amino-3-(((1-ethyl-1*H*-imidazol-5-yl)methyl)amino)benzoate (80 mg, 0.292 mmol) obtained in Preparation Example 11 was dissolved in THF (1 mL), and CDI (118 mg, 0.729 mmol) was added, followed by stirring for 24 hours at room temperature. After the reaction was completed, water was added, extraction was performed with EtOAc, and drying was performed with MgSO₄, followed by concentration under reduced pressure. The resulting concentrate was purified by using MPLC, thereby obtaining methyl 3-((1-ethyl-1*H*-imidazol-5-yl)methyl)-2-oxo-2,3-dihydro-1*H*-benzo[*d*]imidazole-5-carboxylate (53 mg, 0.176 mmol, 61%).

¹H NMR (400 MHz, CDCl₃) δ 8.19 (br s, 1H), 7.84 (dd, *J* = 1.6, 1.6 Hz, 1H), 7.75 (s, 1H), 7.49 (s, 1H), 7.22 (s, 1H), 7.06 (d, *J* = 8.0 Hz, 1H), 5.08 (s, 2H), 4.08 (q, *J* = 7.3 Hz, 2H), 3.90 (s, 3H), 1.33 (t, *J* = 7.3 Hz, 3H).

### [Preparation Example 13: Preparation of methyl 2-chloro-1-((1-ethyl-1H-imidazol-5-yl)methyl)-1H-benzo[d]imidazole-6-carboxylate]

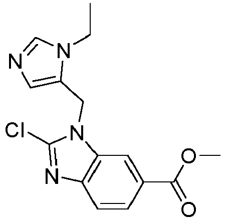

### methyl 2-chloro-1-((1-ethyl-1H-imidazol-5-yl)methyl)-1H-benzo[d]imidazole-6-carboxylate

The methyl 3-((1-ethyl-1*H*-imidazol-5-yl)methyl)-2-oxo-2,3-dihydro-1*H-*benzo[*d*]imidazole-5-carboxylate (62 mg, 0.206 mmol) obtained in Preparation Example 12 was dissolved in DCE (1 mL), and TEA (2 mL, 14.35 mmol) and POCl₃ (1.35 mL, 14.45 mmol) were added, followed by stirring for 24 hours at 80 °C. After the reaction was completed, the resulting reaction product was cooled to room temperature, and the reaction solvent was concentrated under reduced pressure. MPLC purification was performed, thereby obtaining methyl 2-chloro-1-((1-ethyl-1H-imidazol-5-yl)methyl)-1H-benzo[d]imidazole-6-carboxylate (30 mg, 0.094 mmol, 46%).

¹H NMR (500 MHz, CDCl₃) δ 8.04 (d, *J* = 1.0 Hz, 1H), 8.00 (dd, *J* = 8.5, 1.5 Hz, 1H), 7.73 (d, *J* = 9.0 Hz, 1H), 7.53 (s, 1H), 7.08 (s, 1H), 5.44 (s, 2H), 3.94-3.90 (m, 5H), 1.30 (t, *J* = 7.3 Hz, 3H).

### [Preparation Example 14: Preparation of 3-fluoro-4-(((6-(4-hydroxyphenyl)pyridin-2-yl)oxy)methyl)benzonitrile]

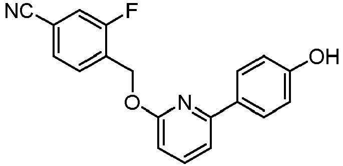

### 3-fluoro-4-(((6-(4-hydroxyphenyl)pyridin-2-yl)oxy)methyl)benzonitrile

The 4-(((6-chloropyridin-2-yl)oxy)methyl)-3-fluorobenzonitrile (150 mg, 0.571 mmol) obtained in Preparation Example 8 was dissolved in 1.4-dioxane/water (1 mL/1 mL), and (4-hydroxyphenyl)boronic acid (95 mg, 0.685 mmol) and K₂CO₃ (95 mg, 0.685 mmol) were added, followed by sufficient nitrogen substitution. Pd(PPh₃)₄ (27 mg, 0.023 mmol) was added, and nitrogen substitution was performed again, followed by stirring for 24 hours at 90 °C. After the reaction was completed, the resulting reaction product was cooled to room temperature, filtered through Celite by using EtOAc, and then concentrated under reduced pressure. The resulting concentrate was purified by using MPLC, thereby obtaining 3-fluoro-4-(((6-(4-hydroxyphenyl)pyridin-2-yl)oxy)methyl)benzonitrile (65 mg, 0.203 mmol, 35%).

¹H NMR (500 MHz, CDCl₃) δ 7.39 (d, *J* = 8.5 Hz, 2H), 7.67-7.63 (m, 2H), 7.45 (d, *J* = 8.5 Hz, 1H), 7.39 (dd, *J* = 9.5, 1.5 Hz, 1H), 7.31 (d, *J* = 7.5 Hz, 1H), 6.91-6.89 (m, 2H), 6.73 (d, *J* = 8.5 Hz, 1H), 5.63 (s, 2H); LC-MS(ESI): 321.28 [M+H]⁺

### [Preparation Example 15: Preparation of methyl 2-(4-(6-((4-cyano-2-fluorobenzyl)oxy)pyridin-2-yl)phenoxy)-1-((1-ethyl-1H-imidazol-5-yl)methyl)-1H-benzo[d]imidazole-6-carboxylate]

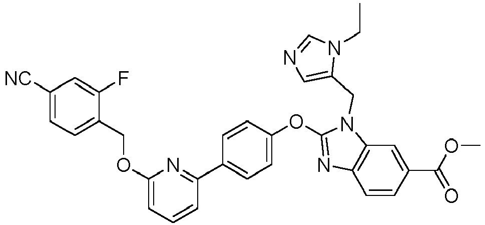

### methyl 2-(4-(6-((4-cyano-2-fluorobenzyl)oxy)pyridin-2-yl)phenoxy)-1-((1-ethyl-1H-imidazol-5-yl)methyl)-1H-benzo[d]imidazole-6-carboxylate

The 3-fluoro-4-(((6-(4-hydroxyphenyl)pyridin-2-yl)oxy)methyl)benzonitrile (20 mg, 0.062 mmol) obtained in Preparation Example 14 was dissolved in DCM (1 mL), and TEA (0.522 mL, 3.75 mmol) and the methyl 2-chloro-1-((1-ethyl-1*H-*imidazol-5-yl)methyl)-1*H*-benzo[*d*]imidazole-6-carboxylate (20 mg, 0.062 mmol) obtained in Preparation Example 13 were added, followed by stirring for 24 hours at 40 °C. After the reaction was completed, the resulting reaction product was cooled to room temperature, and the reaction solvent was concentrated under reduced pressure. MPLC purification was performed, thereby obtaining methyl 2-(4-(6-((4-cyano-2-fluorobenzyl)oxy)pyridin-2-yl)phenoxy)-1-((1-ethyl-1*H*-imidazol-5-yl)methyl)-1H-benzo[*d*]imidazole-6-carboxylate (10 mg, 0.017 mmol, 27%).

¹H NMR (500 MHz, MeOD) δ 7.90-7.66 (m, 8H), 7.37 (d, *J* = 7.5 Hz, 1H), 7.15 (d, *J* = 8.0 Hz, 1H), 7.12 (s, 1H), 7.03 (d, *J* = 7.5 Hz, 1H), 6.87-6.82 (m, 2H), 6.72 (d, *J* = 8.0 Hz, 1H), 5.62 (s, 2H), 5.20 (s, 2H), 4.16-4.13 (m, 2H), 3.90 (s, 3H), 1.29 (t, *J* = 7.0 Hz, 3H).

### [Preparation Example 16: Preparation of methyl (S)-2-mercapto-1-(oxetan-2-ylmethyl)-1H-benzo[d]imidazole-6-carboxylate]

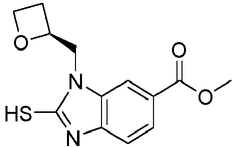

### methyl (S)-2-mercapto-1-(oxetan-2-ylmethyl)-1H-benzo[d]imidazole-6-carboxylate

The methyl (*S*)-4-amino-3-((oxetan-2-ylmethyl)amino)benzoate (300 mg, 1.270 mmol) obtained in Preparation Example 7 was dissolved in THF (5 mL), and TEA (0.354 mL, 2.54 mmol) was added, followed by stirring for 15 minutes under nitrogen at room temperature. CS₂ (0.115 mL, 1.905 mmol) was slowly added dropwise at 0 °C, and then stirred for 24 hours at 90 °C. After the reaction was completed, the resulting reaction product was cooled to room temperature. After water was added, the resulting product was extracted with EtOAc, dried with MgSO₄, and concentrated under reduced pressure. The resulting concentrate was purified by using MPLC, thereby obtaining methyl (*S*)-2-mercapto-1-(oxetan-2-ylmethyl)-1H-benzo[*d*]imidazole-6-carboxylate (235 mg, 0.844 mmol, 67%).

¹H NMR (500 MHz, CDCl₃) δ 9.70 (br s, 1H), 8.10 (s, 1H), 7.96 (dd, *J* = 8.5, 1.5 Hz, 1H), 7.21 (d, *J* = 8.0 Hz, 1H), 5.32-5.28 (m, 1H), 4.66-4.60 (m, 3H), 4.47-4.42 (m, 1H), 3.94 (s, 3H), 2.81-2.68 (m, 2H); LC-MS(ESI): 279.24 [M+H]⁺

### [Preparation Example 17: Preparation of methyl (S)-2-benzylthio-1-(oxetan-2-ylmethyl)-1H-benzo[d]imidazole-6-carboxylate]

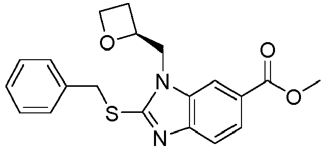

### methyl (S)-2-(benzylthio)-1-(oxetan-2-ylmethyl)-1H-benzo[d]imidazole-6-carboxylate

The methyl (*S*)-2-mercapto-1-(oxetan-2-ylmethyl)-1*H*-benzo[*d*]imidazole-6-carboxylate (235 mg, 0.844 mmol) obtained in Preparation Example 16 was dissolved in THF (5 mL), and NaH (60 % dispersion in mineral oil, 68 mg, 1.689 mmol) was added, followed by stirring for 15 minutes under nitrogen at room temperature. BnBr (0.131 mL, 1.098 mmol) was slowly added dropwise to the reaction mixture, and then stirred for 4 hours at room temperature. After the reaction was completed, water was added, extraction was performed with EtOAc, and drying was performed with MgSO₄, followed by concentration under reduced pressure. The resulting concentrate was purified by using MPLC, thereby obtaining methyl (*S*)-2-benzylthio-1-(oxetan-2-ylmethyl)-1*H*-benzo[*d*]imidazole-6-carboxylate (248 mg, 0.673 mmol, 80%).

¹H NMR (500 MHz, CDCl₃) δ 8.11 (d, *J* = 1.0 Hz, 1H), 7.97 (dd, *J* = 8.5, 1.5 Hz, 1H), 7.70 (d, *J* = 8.5 Hz, 1H), 7.43-7.41 (m, 2H), 7.31-7.27 (m, 3H), 5.14-5.11 (m, 1H), 4.66 (s, 2H), 4.59-4.57 (m, 1H), 4.38-4.32 (m, 3H), 3.94 (s, 3H), 2.71-2.66 (m, 1H), 2.47-2.43 (m, 1H); LC-MS(ESI): 369.32 [M+H]⁺

### [Preparation Example 18: Preparation of methyl (S)-2-benzylsulfonyl-1-(oxetan-2-ylmethyl)-1H-benzo[d]imidazole-6-carboxylate]

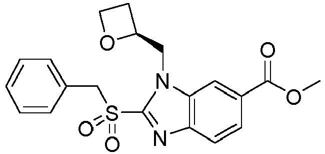

### methyl (S)-2-(benzylsulfonyl)-1-(oxetan-2-ylmethyl)-1H-benzo[d]imidazole-6-carboxylate

mCPBA (348 mg, 2.019 mmol) was dissolved in DCM (3 mL), and the methyl (*S*)-2-benzylthio-1-(oxetan-2-ylmethyl)-1H-benzo[*d*]imidazole-6-carboxylate (248 mg, 0.673 mmol) obtained in Preparation Example 17 was dissolved in DCM (3 mL) at 0 °C and slowly added dropwise to the reaction mixture, followed by stirring for 4 hours at room temperature under nitrogen. After the reaction was completed, water was added, and the resulting solution was neutralized to pH ~7 with NaHCO₃. The resulting product was extracted with EtOAc, dried with MgSO₄, and then concentrated under reduced pressure. The resulting concentrate was purified by using MPLC, thereby obtaining methyl (*S*)-2-benzylsulfonyl-1-(oxetan-2-ylmethyl)-1*H*-benzo[*d*]imidazole-6-carboxylate (250 mg, 0.624 mmol, 93%).

¹H NMR (400 MHz, CDCl₃) δ 8.30 (d, *J* = 0.8 Hz 1H), 8.07 (d, *J* = 1.5 Hz, 1H), 7.93 (d, *J* = 1.5 Hz, 1H), 7.39-7.20 (m, 5H), 4.95-4.91 (m, 1H), 4.90 (s, 2H), 4.60-4.51 (m, 2H), 4.42-4.32 (m, 2H), 3.95 (s, 3H), 2.60-2.69 (m, 1H), 2.31-2.39 (m, 1H); LC-MS(ESI): 401.31 [M+H]⁺

### [Preparation Example 19: Preparation of methyl (S)-2-(4-(6-((4-cyano-2-fluorobenzyl)oxy)pyridin-2-yl)phenoxy)-1-(oxetan-2-ylmethyl)-1H-benzo[d]imidazole-6-carboxylate]

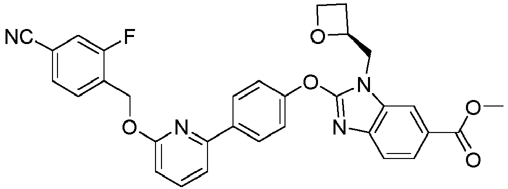

### methyl (S)-2-(4-(6-((4-cyano-2-fluorobenzyl)oxy)pyridin-2-yl)phenoxy)-1-(oxetan-2-ylmethyl)-1H-benzo[d]imidazole-6-carboxylate

The 3-fluoro-4-(((6-(4-hydroxyphenyl)pyridin-2-yl)oxy)methyl)benzonitrile (44 mg, 0.137 mmol) obtained in Preparation Example 14 was dissolved in DMF (2 mL), and *t*-BuOK (23 mg, 0.206 mmol) was added, followed by stirring for 15 minutes under nitrogen at room temperature. The methyl (S)-2-benzylsulfonyl-1-(oxetan-2-ylmethyl)-1*H*-benzo[*d*]imidazole-6-carboxylate (55 mg, 0.137 mmol) obtained in Preparation Example 18 was added, and then stirred for 3 hours at room temperature. After the reaction was completed, water was added, extraction was performed with EtOAc, and drying was performed with MgSO₄, followed by concentration under reduced pressure. The resulting concentrate was purified by using MPLC, thereby obtaining methyl (*S*)-2-(4-(6-((4-cyano-2-fluorobenzyl)oxy)pyridin-2-yl)phenoxy)-1-(oxetan-2-ylmethyl)-1*H-*benzo[*d*]imidazole-6-carboxylate (41 mg, 0.073 mmol, 53%).

¹H NMR (500 MHz, CDCl₃) δ 8.13 (s, 1H), 8.08 (m, 2H), 7.94 (dd, *J* = 8.5, 1.5 Hz, 1H), 7.70-7.55 (m, 5H), 7.47 (d, *J* = 2.0 Hz, 2H), 7.45 (d, *J* = 1.5 Hz, 1H), 7.37 (d, *J* = 7.5 Hz, 1H), 6.79 (d, *J* = 8.0 Hz, 1H), 5.63 (s, 2H), 5.27-5.25 (m, 1H), 4.68-4.65 (m, 1H), 4.50-4.12 (m, 3H), 3.94 (s, 3H), 2.82-2.81 (m, 1H), 2.63-2.61 (m, 1H).

### [Preparation Example 20: Preparation of 4-(6-((4-chloro-2-fluorobenzyl)oxy)pyridin-2-yl)phenol]

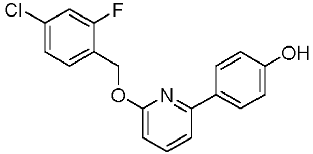

### 4-(6-((4-chloro-2-fluorobenzyl)oxy)pyridin-2-yl)phenol

The 2-chloro-6-((4-chloro-2-fluorobenzyl)oxy)pyridine (100 mg, 0.368 mmol) obtained in Preparation Example 1 was dissolved in THF/H₂O (3 mL/0.3 mL), and (4-hydroxyphenyl)boronic acid (56 mg, 0.404 mmol) and Cs₂CO₃ (300 mg, 0.919 mmol) were added, followed by sufficient nitrogen substitution. Pd(dppf)Cl₂-DCM (30 mg, 0.037 mmol) was added, and nitrogen substitution was performed again, followed by stirring for 6 hours at 90 °C. After the reaction was completed, the resulting reaction product was cooled to room temperature, filtered through Celite by using EtOAc, and then concentrated under reduced pressure. The resulting concentrate was purified by using MPLC, thereby obtaining 4-(6-((4-chloro-2-fluorobenzyl)oxy)pyridin-2-yl)phenol (30 mg, 0.091 mmol, 25%).

¹H NMR (500 MHz, CDCl₃) δ 7.93 (d, *J* = 8.5 Hz, 2H), 7.62 (t, *J* = 8.0 Hz, 1H), 7.48 (t, *J* = 8.3 Hz, 1H), 7.29 (d, *J* = 7.5 Hz, 1H), 7.14-7.12 (m, 2H), 6.91 (d, *J* = 8.5 Hz, 2H), 6.69 (d, *J* = 8.5 Hz, 1H), 5.53 (s, 2H), 4.83 (s, 1H)

### [Preparation Example 21: Preparation of methyl (S)-2-(4-(6-((4-chloro-2-fluorobenzyl)oxy)pyridin-2-yl)phenoxy)-1-(oxetan-2-ylmethyl)-1H-benzo[d]imidazole-6-carboxylate]

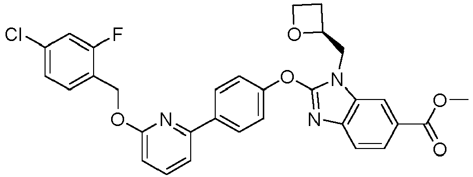

### methyl (S)-2-(4-(6-((4-chloro-2-fluorobenzyl)oxydin-2-yl)phenoxy)-1-(oxetan-2-ylmethyl)-1H-benzo[d]imidazole-6-carboxylate

The 4-(6-((4-chloro-2-fluorobenzyl)oxy)pyridin-2-yl)phenol (60 mg, 0.182 mmol) obtained in Preparation Example 20 was dissolved in DMF (2 mL), and *t-*BuOK (31 mg, 0.273 mmol) was added, followed by stirring for 15 minutes under nitrogen at room temperature. The methyl (*S*)-2-benzylsulfonyl-1-(oxetan-2-ylmethyl)-1*H*-benzo[*d*]imidazole-6-carboxylate (73 mg, 0.182 mmol) obtained in Preparation Example 18 was added and stirred for 3 hours at room temperature. After the reaction was completed, water was added, extraction was performed with EtOAc, and drying was performed with MgSO₄, followed by concentration under reduced pressure. The resulting concentrate was purified by using MPLC, thereby obtaining methyl (*S*)-2-(4-(6-((4-chloro-2-fluorobenzyl)oxy)pyridin-2-yl)phenoxy)-1-(oxetan-2-ylmethyl)-1*H*-benzo[*d*]imidazole-6-carboxylate (86 mg, 0.150 mmol, 82%).

¹H NMR (500 MHz, CDCl₃) δ 8.13-8.09 (m, 3H), 7.94 (dd, *J* = 8.5, 1.5 Hz, 1H), 7.67 (t, *J* = 8.0 Hz, 1H), 7.58 (d, *J* = 8.5 Hz, 1H), 7.50-7.46 (m, 3H), 7.36 (d, *J* = 7.5 Hz, 1H), 7.15-7.05 (m, 2H), 6.76 (d, *J* = 8.5 Hz, 1H), 5.54 (s, 2H), 5.28-5.25 (m, 1H), 4.69-4.65 (m, 1H), 4.52-4.43 (m, 3H), 3.94 (s, 3H), 2.84-2.81 (m, 1H), 2.63-2.59 (m, 1H)

### [Preparation Example 22: Preparation of methyl 4-nitro-3-((oxazol-5-ylmethyl)amino)benzoate]

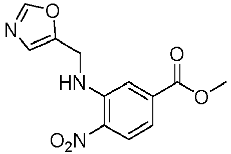

### methyl 4-nitro-3-((oxazol-5-ylmethyl)amino)benzoate

Methyl 3-fluoro-4-nitrobenzoate (904 mg, 4.54 mmol) was dissolved in THF (15 mL), and oxazol-5-ylmethaneamine hydrochloride (600 mg, 4.33 mmol) and TEA (1.81 mL, 12.98 mmol) were added, followed by stirring for 16 hours under nitrogen at room temperature. After the reaction was completed, a reaction solvent was concentrated under reduced pressure. Water was added, extraction was performed with EtOAc, and drying was performed with MgSO₄, followed by concentration under reduced pressure. The resulting concentrate was purified by using MPLC, thereby obtaining methyl 4-nitro-3-((oxazol-5-ylmethyl)amino)benzoate (682 mg, 2.46 mmol, 57%).

¹H NMR (400 MHz, CDCl₃) δ 8.25-8.23 (m, 2H), 7.87 (s, 1H), 7.64 (d, *J* = 1.2 Hz, 1H), 7.33 (dd, *J* = 2.0, 1.2 Hz, 1H), 7.09 (s, 1H), 4.66 (d, *J* = 5.6 Hz, 2H), 3.94 (s, 3H)

### [Preparation Example 23: Preparation of methyl 4-amino-3-((oxazol-5-ylmethyl)amino)benzoate]

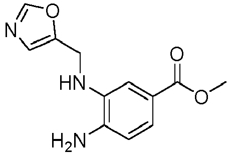

### methyl 4-amino-3-((oxazol-5-ylmethyl)amino)benzoate

The methyl 4-nitro-3-((oxazol-5-ylmethyl)amino)benzoate (682 mg, 2.46 mmol) obtained in Preparation Example 22 was dissolved in THF (12 mL), and 10% Pd/C (262 mg) was added, followed by stirring for 24 hours under hydrogen at room temperature. After the reaction was completed, the resulting reaction product was filtered through Celite by using EtOAc, and concentrated under reduced pressure, thereby obtaining methyl 4-amino-3-((oxazol-5-ylmethyl)amino)benzoate (600 mg, 2.43 mmol, 99%).

¹H NMR (500 MHz, CDCl₃) δ 7.86 (s, 1H), 7.52 (dd, *J* = 8.0, 1.5 Hz, 1H), 7.43 (d, *J* = 2.0 Hz, 1H), 7.04 (s, 1H), 6.72 (d, *J* = 8.0 Hz, 1H), 4.41 (d, *J* = 5.5 Hz, 2H), 3.86 (s, 3H), 3.83 (s, 2H), 3.46 (s,1H); LC-MS(ESI): 248.25 [M+H]⁺

### [Preparation Example 24: Preparation of methyl 2-(4-(6-((4-chloro-2-fluorobenzyl)oxy)pyridin-2-yl)benzyl)-1-(oxazol-5-ylmethyl)-1H-benzo [d]imidazole-6-carboxylate]

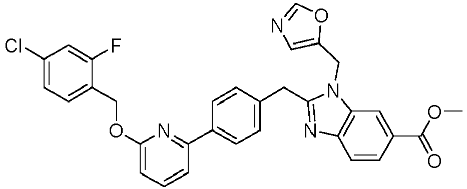

### methyl 2-(4-(6-((4-chloro-2-fluorobenzyl)oxy)pyridin-2-yl)benzyl)-1-(oxazol-5-ylmethyl)-1H-benzo[d]imidazole-6-carboxylate

The 2-(4-(6-((4-chloro-2-fluorobenzyl)oxy)pyridin-2-yl)phenyl)acetic acid (40 mg, 0.108 mmol) obtained in Preparation Example 2 was dissolved in DMF (1 mL), and the methyl 4-amino-3-((oxazol-5-ylmethyl)amino)benzoate (30 mg, 0.118 mmol) obtained in Preparation Example 23, HATU (82 mg, 0.215 mmol) and TEA (0.08 mL, 0.538 mmol) were added. The resulting reactants were stirred for 24 hours under nitrogen at room temperature. After the reaction was completed, water was added, extraction was performed with EtOAc, and drying was performed with MgSO₄, followed by concentration under reduced pressure. The resulting concentrate was purified by using MPLC, thereby obtaining methyl 4-(2-(4-(6-((4-chloro-2-fluorobenzyl)oxy)pyridin-2-yl)phenyl)acetamido)-3-((oxazol-5-ylmethyl)amino)benzoate. The methyl 4-(2-(4-(6-((4-chloro-2-fluorobenzyl)oxy)pyridin-2-yl)phenyl)acetamido)-3-((oxazol-5-ylmethyl)amino)benzoate was dissolved in AcOH (1.5 mL, 26.6 mmol) without additional purification, and stirred for 3 hours at 120 °C. After the reaction was completed, concentration was performed under reduced pressure. Water was added, extraction was performed with EtOAc, and drying was performed with MgSO₄, followed by concentration under reduced pressure. The resulting concentrate was purified by using MPLC, thereby obtaining methyl 2-(4-(6-((4-chloro-2-fluorobenzyl)oxy)pyridin-2-yl)benzyl)-1-(oxazol-5-ylmethyl)-1H-benzo[*d*]imidazole-6-carboxylate (49 mg, 0.084 mmol, 63%).

¹H NMR (500 MHz, CDCl₃) δ 8.13 (s, 1H), 8.02 (dd, *J* = 8.5, 1.5 Hz, 1H), 7.81 (d, *J* = 8.5 Hz, 1H), 7.77 (s, 1H), 7.56-7.53 (m, 4H), 7.43 (t, *J* = 7.5 Hz, 2H), 7.36-7.31 (m, 4H), 6.84 (s, 1H), 5.28 (s, 2H), 4.48 (s, 2H), 3.95 (s, 5H).

### [Preparation Example 25: Preparation of methyl 2-(benzylthio)-1-(oxazol-5-ylmethyl)-1H-benzo[d]imidazole-6-carboxylate]

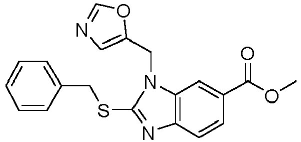

### methyl 2-(benzylthio)-1-(oxazol-5-ylmethyl)-1H-benzo[d]imidazole-6-carboxylate

The methyl 4-amino-3-((oxazol-5-ylmethyl)amino)benzoate (300 mg, 1.213 mmol) obtained in Preparation Example 23 was dissolved in THF (12 mL), and TCDI (360 mg, 1.820 mmol) was added, followed by stirring for 24 hours under nitrogen at room temperature. Water was added, extraction was performed with EtOAc, and drying was performed with MgSO₄, followed by concentration under reduced pressure. The resulting concentrate was performed by using MPLC, thereby obtaining methyl 2-mercapto-1-(oxazol-5-ylmethyl)-1*H*-benzo[*d*]imidazole-6-carboxylate (350 mg, 1.210 mmol, 100%). The methyl 2-mercapto-1-(oxazol-5-ylmethyl)-1*H*-benzo[*d*]imidazole-6-carboxylate was dissolved in THF (7 mL) without additional purification, and NaH (60 % dispersion in mineral oil, 97 mg, 2.420 mmol) was added, followed by stirring for 15 minutes under nitrogen at room temperature. BnBr (0.187 mL, 1.573 mmol) was slowly added dropwise, and then stirred for 4 hours at room temperature. After the reaction was completed, water was added, extraction was performed with EtOAc, and drying was performed with MgSO₄, followed by concentration under reduced pressure. The resulting concentrate was purified by using MPLC, thereby obtaining methyl 2-(benzylthio)-1-(oxazol-5-ylmethyl)-1*H*-benzo[*d*]imidazole-6-carboxylate (450 mg, 1.186 mmol, 98%).

¹H NMR (500 MHz, CDCl₃) δ 8.09 (d, *J* = 9.5 Hz, 1H), 7.99 (dd, *J* = 8.5, 1.5 Hz, 1H), 7.79 (s, 1H), 7.71 (d, *J* = 8.5 Hz, 1H), 7.42 (d, *J* = 6.5 Hz, 2H), 7.34-7.29 (m, 3H), 7.05 (s, 1H), 5.30 (s, 2H), 4.66 (s, 2H), 3.95 (s, 3H)

### [Preparation Example 26: Preparation of methyl 2-(benzylsulfonyl)-1-(oxazol-5-ylmethyl)-1H-benzo[d]imidazole-6-carboxylate]

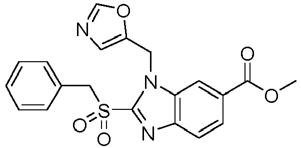

### methyl 2-(benzylsulfonyl)-1-(oxazol-5-ylmethyl)-1H-benzo[d]imidazole-6-carboxylate

mCPBA (877 mg, 3.56 mmol) was dissolved in DCM (5 mL), and then the methyl 2-(benzylthio)-1-(oxazol-5-ylmethyl)-1H-benzo[d]imidazole-6-carboxylate (450 mg, 1.186 mmol) obtained in Preparation Example 25 was dissolved in DCM (5 mL) and slowly added dropwise at 0 °C, followed by stirring for 4 hours under nitrogen at room temperature. After the reaction was completed, water was added, and the resulting solution was neutralized to pH ~7 with NaHCO₃. The resulting product was extracted with EtOAc, dried with MgSO₄, and then concentrated under reduced pressure. The resulting concentrate was purified by using MPLC, thereby obtaining methyl 2-(benzylsulfonyl)-1-(oxazol-5-ylmethyl)-1H-benzo[d]imidazole-6-carboxylate (285 mg, 0.693 mmol, 58%).

¹H NMR (500 MHz, CDCl₃) δ 8.30 (s, 1H), 8.13 (d, *J* = 8.5 Hz, 1H), 7.96 (d, *J=* 8.5 Hz, 1H), 7.73 (s, 1H), 7.35 (t, *J=* 6.8 Hz, 1H), 7.28-7.25 (m, 2H), 7.20 (d, *J* = 7.0 Hz, 2H), 7.05 (s, 1H), 5.41 (s, 2H), 4.84 (s, 2H), 3.99 (s, 3H); LC-MS(ESI): 412.28 [M+H]⁺

### [Preparation Example 27: Preparation of methyl 2-(4-(6-((4-cyano-2-fluorobenzyl)oxy)pyridin-2-yl)phenoxy)-1-(oxazol-5-ylmethyl)-1H-benzo[d]imidazole-6-carboxylate]

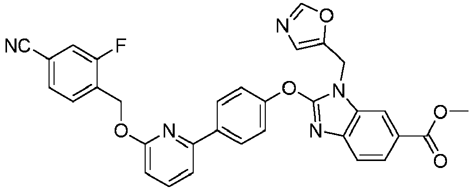

### methyl 2-(4-(6-((4-cyano-2-fluorobenzyl)oxy)pyridin-2-yl)phenoxy)-1-(oxazol-5-ylmethyl)-1H-benzo[d]imidazole-6-carboxylate

The 3-fluoro-4-(((6-(4-hydroxyphenyl)pyridin-2-yl)oxy)methyl)benzonitrile (70 mg, 0.219 mmol) obtained in Preparation Example 14 was dissolved in DMF (2 mL), and *t*-BuOK (37 mg, 0.328 mmol) was added, followed by stirring for 15 minutes under nitrogen at room temperature. The methyl 2-(benzylsulfonyl)-1-(oxazol-5-ylmethyl)-1*H*-benzo[*d*]imidazole-6-carboxylate (90 mg, 0.219 mmol) obtained in Preparation Example 26 was added, and then stirred for 3 hours at room temperature. After the reaction was completed, water was added, and the resulting product was extracted with EtOAc, dried with MgSO₄, and then concentrated under reduced pressure. The resulting concentrate was purified by using MPLC, thereby obtaining methyl 2-(4-(6-((4-cyano-2-fluorobenzyl)oxy)pyridin-2-yl)phenoxy)-1-(oxazol-5-ylmethyl)-1*H*-benzo[*d*]imidazole-6-carboxylate (64 mg, 0.111 mmol, 51%).

¹H NMR (500 MHz, CDCl₃) δ 8.09-8.05 (m, 3H), 7.96 (dd, *J* = 8.5, 1.5 Hz, 1H), 7.87 (s, 1H), 7.72-7.67 (m, 2H), 7.58 (d, *J* = 8.0 Hz, 1H), 7.47 (d, *J* = 9.0 Hz, 3H), 7.41-7.38 (m, 2H), 7.20 (s, 1H), 6.81 (d, *J* = 8.0 Hz, 1H), 5.64 (s, 2H), 5.45 (s, 2H), 3.96 (s, 3H).

### [Preparation Example 28: Preparation of 2-(4-(6-((4-chloro-2-fluorobenzyl)oxy)pyridin-2-yl)-2-fluorophenyl)acetic acid]

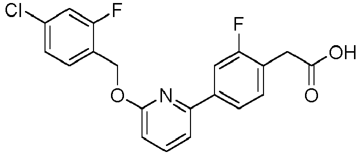

### 2-(4-(6-((4-chloro-2-fluorobenzyl)oxy)pyridin-2-yl)-2-fluorophenyl)acetic acid

The 2-chloro-6-((4-chloro-2-fluorobenzyl)oxy)pyridine (908 mg, 3.34 mmol) obtained in Preparation Example 1, and Pd(dppf)Cl₂-DCM (273 mg, 0.334 mmol), Cs₂CO₃ (2718 mg, 8.34 mmol), and (4-(2-ethoxy-2-oxoethyl)-3-fluorophenyl)boronic acid (830 mg, 3.67 mmol) were dissolved in 13 mL of THF/H₂O (9/1) and subjected to nitrogen substitution. The reactants were stirred for 5 hours at 85 °C. After the reaction was completed, water was added, and extraction was performed using EtOAc. An organic layer was dried with Na₂SO₄, and concentrated under reduced pressure, thereby obtaining an intermediate, ethyl 2-(4-(6-((4-chloro-2-fluorobenzyl)oxy)pyridin-2-yl)-2-fluorophenyl)acetate. The intermediate was dissolved in 10 mL of THF/H₂O (1/1) without additional purification, and NaOH (210 mg, 5.24 mmol) was added. The reactant was stirred for 24 hours at room temperature. After the reaction was completed, the resulting solution was adjusted to pH ~2 using IN HCl, and extraction was performed using EtOAc. An organic layer was dried with Na₂SO₄, concentrated under reduced pressure, and purified by using MPLC, thereby obtaining 2-(4-(6-((4-chloro-2-fluorobenzyl)oxy)pyridin-2-yl)-2-fluorophenyl)acetic acid (650 mg, 1.668 mmol, 95%).

¹H NMR (400 MHz, CDCl₃) δ 7.75-7.74 (m, 2H), 7.65 (t, *J* = 8.4 Hz, 1H), 7.46 (t, *J* = 8.0 Hz, 1H), 7.34-7.32 (m, 2H), 7.13 (d, *J* = 8.8 Hz, 2H), 6.76 (d, *J* = 8.0 Hz, 1H), 5.51 (s, 2H), 3.77 (s, 2H); LC-MS(ESI): 390.26 [M+H]⁺

### [Preparation Example 29: Preparation of methyl (S)-4-nitro-3(((tetrahydrofuran-2-yl)methyl)amino)benzoate]

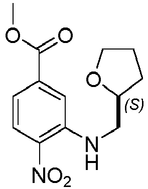

### methyl (S)-4-nitro-3-(((tetrahydrofuran-2-yl)methyl)amino)benzoate

Methyl 3-fluoro-4-nitrobenzoate (1.39 g, 7.0 mmol) was dissolved in DMF (2.5 mL), and (S)-(tetrahydrofuran-2-yl)methaneamine (0.868 mL) and K₂CO₃ (967 mg) were added at room temperature. The reactants were stirred for 4 hours at 50 °C. Water was added, and extraction was performed using EtOAc. An organic layer was dried with Na₂SO₄, concentrated under reduced pressure, and purified by using MPLC, thereby obtaining methyl (*S*)-4-nitro-3(((tetrahydrofuran-2-yl)methyl)amino)benzoate (1.69 g, 6.86 mmol, 86%).

¹H NMR (400 MHz, CDCl₃) δ 8.26 - 8.12 (m, 2H), 7.59 (d, *J* = 1.8 Hz, 1H), 7.24 (dd, *J* = 9.1, 1.8 Hz, 1H), 4.22 (qd, *J* = 6.9, 4.1 Hz, 1H), 4.02 - 3.91 (m, 4H), 3.87 - 3.77 (m, 1H), 3.53 (td, *J* = 8.7, 4.3 Hz, 1H), 3.46 - 3.34 (m, 1H), 2.18 - 2.04 (m, 1H), 2.05 - 1.90 (m, 2H), 1.82 - 1.61 (m, 1H); LC-MS(ESI): 281.28 [M+H]⁺

### [Preparation Example 30: Preparation of methyl (S)-4-amino-3-(((tetrahydrofuran-2-yl)methyl)amino)benzoate]

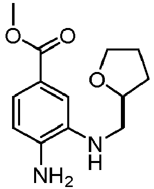

### methyl (S)-4-amino-3-(((tetrahydrofuran-2-yl)methyl)amino)benzoate

The methyl (*S*)-4-nitro-3(((tetrahydrofuran-2-yl)methyl)amino)benzoate (1.69 g, 6.86 mmol) obtained in Preparation Example 29 was dissolved in THF (50 mL), and 10% Pd/C (169 mg) was added, followed by stirring for 17.5 hours under hydrogen at room temperature. After the reaction was completed, Pd/C was filtered through Celite, and then concentration under reduced pressure was performed, thereby obtaining methyl (S)-4-amino-3-(((tetrahydrofuran-2-yl)methyl)amino)benzoate (1.68 g, 6.72 mmol, 98%).

¹H NMR (500 MHz, CDCl₃) δ 7.46 (dd, *J* = 8.1, 1.7 Hz, 1H), 7.34 (d, *J* = 1.5 Hz, 1H), 6.67 (d, *J* = 7.9 Hz, 1H), 4.20 (qd, *J* = 7.3, 3.2 Hz, 1H), 3.96 - 3.76 (m, 5H), 3.53 (brs, 1H), 3.26 (dd, *J* = 11.9, 3.1 Hz, 1H), 3.15 - 3.03 (m, 1H), 2.14 - 2.04 (m, 1H), 2.00 - 1.90 (m, 2H), 1.77 - 1.63 (m, 1H); LC-MS(ESI): 251.29 [M+H]⁺

### [Preparation Example 31: Preparation methyl (R)-4-nitro-3(((tetrahydrofuran-2-yl)methyl)amino)benzoate]

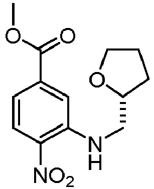

### methyl (R)-4-nitro-3-(((tetrahydrofuran-2-yl)methyl)amino)benzoate

Methyl 3-fluoro-4-nitrobenzoate (1.39 g, 7.0 mmol) was dissolved in DMF (2.5 mL), and (*R*)-(tetrahydrofuran-2-yl)methaneamine (0.868 mL) and K₂CO₃ (967 mg) were added at room temperature. The reactants were stirred for 4 hours at 50 °C. Water was added, and then extraction was performed by using EtOAc. An organic layer was dried with Na₂SO₄, concentrated under reduced pressure, and purified by using MPLC, thereby obtaining methyl (*R*)-4-nitro-3(((tetrahydrofuran-2-yl)methyl)amino)benzoate (1.82 g, 6.47 mmol, 92%).

¹H NMR (400 MHz, CDCl₃) δ 8.30 - 8.14 (m, 2H), 7.58 (d, *J* = 1.8 Hz, 1H), 7.24 (dd, *J* = 8.9, 1.6 Hz, 1H), 4.22 (qd , *J* = 6.9, 4.1 Hz, 1H), 4.06 - 3.90 (m, 4H), 3.88 - 3.78 (m, 1H), 3.53 (dt, *J* = 12.8, 4.7 Hz, 1H), 3.46 - 3.30 (m, 1H), 2.17 - 2.04 (m, 1H), 2.05 - 1.85 (m, 2H), 1.78 - 1.61 (m, 1H); LC-MS(ESI): 281.28 [M+H]⁺

### [Preparation Example 32: Preparation of methyl (R)-4-amino-3-(((tetrahydrofuran-2-yl)methyl)amino)benzoate]

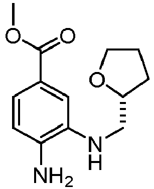

### methyl (R)-4-amino-3-(((tetrahydrofuran-2-yl)methyl)amino)benzoate

The methyl (*R*)-4-nitro-3(((tetrahydrofuran-2-yl)methyl)amino)benzoate (1.82 g, 6.47 mmol) obtained in Preparation Example 31 was dissolved in THF (50 mL), and 10% Pd/C (182 mg) was added, followed by stirring for 12 hours under hydrogen at room temperature. After the reaction was completed, Pd/C was filtered through Celite, and then concentration under reduced pressure was performed, thereby obtaining methyl (*R*)-4-amino-3-(((tetrahydrofuran-2-yl)methyl)amino)benzoate (1.58 g, 6.34 mmol, 98%).

¹H NMR (500 MHz, CDCl₃) δ 7.48 (dd, *J* = 7.9, 1.8 Hz, 1H), 7.36 (s, 1H), 6.69 (d, *J* = 7.9 Hz, 1H), 4.27 - 4.18 (m, 1H), 4.00 - 3.79 (m, 5H), 3.56 (brs, 1H), 3.27 (dd, *J* = 11.9, 3.1 Hz, 1H), 3.11 (dd, *J* = 11.9, 8.2 Hz, 1H), 2.16 - 2.06 (m, 1H), 2.02 - 1.90 (m, 2H), 1.79 - 1.66 (m, 1H); LC-MS(ESI): 251.29 [M+H]⁺

### [Preparation Example 33: Preparation of methyl (R)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate]

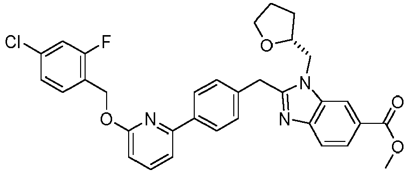

### methyl (R)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate

The methyl (*R*)-4-amino-3-(((tetrahydrofuran-2-yl)methyl)amino)benzoate (150 mg, 0.6 mmol) obtained in Preparation Example 32 and the 2-(4-(6-((4-chloro-2-fluorobenzyl)oxy)pyridin-2-yl)phenyl)acetic acid (186 mg, 0.5 mmol) obtained in Preparation Example 2 were dissolved in 2 mL of DMF, and EDC (192 mg, 1.0 mmol) and HOBt (135 mg, 1.0 mmol) were added, followed by stirring for 24 hours at room temperature. After the reaction was completed, water was added, and extraction was performed by using EtOAc. An organic layer was dried with Na₂SO₄, and concentrated under reduced pressure, thereby obtaining an intermediate, methyl (*R*)-4-(2-(4-(6-((4-chloro-2-fluorobenzyl)oxy)pyridin-2-yl)phenyl)acetamido)-3-(((tetrahydrofuran-2-yl)methyl)amino)benzoate. The intermediate was dissolved in 10 mL of acetic acid without additional purification, and then stirred for 2 hours at 120 °C. Water was added, and extraction was performed by using EtOAc. The organic layer was dried with Na₂SO₄, concentrated under reduced pressure, and purified by using MPLC, thereby obtaining methyl (*R*)-2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylate (144 mg, 0.25 mmol, 49%).

¹H NMR (500 MHz, CDCl₃) δ 8.08 (s, 1H), 7.98 (d, *J* = 8.5 Hz, 1H) , 7.93 (d, *J* = 8.0 Hz, 2H), 7.75 (d, *J* = 8.5 Hz, 1H), 7.64 (t, *J* = 7.5 Hz, 1H), 7.46 (t, *J* = 8.1 Hz, 1H), 7.35 - 7.28 (m, 3H), 7.17 - 7.08 (m, 2H), 6.73 (d, *J* = 8.2 Hz, 1H), 5.54 - 5.43 (m, 2H), 4.51 (dd, *J* = 38.1, 15.9 Hz, 2H), 4.25 - 4.17 (m, 1H), 4.15 - 4.06 (m, 2H), 3.94 (s, 3H), 3.86 (dd, *J* = 15.1, 6.9 Hz, 1H), 3.75 - 3.69 (m, 1H), 2.04 - 1.97 (m, 1H), 1.92 - 1.80 (m, 2H), 1.59 - 1.47 (m, 1H); LC-MS(ESI): 586.33 [M+H]⁺

### [Preparation Example 34: Preparation of methyl 4-nitro-3-(((tetrahydro-2H-pyran-2-yl)methyl)amino)benzoate]

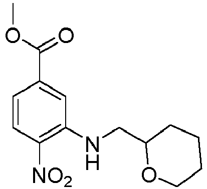

### methyl 4-nitro-3-(((tetrahydro-2H-pyran-2-yl)methyl)amino)benzoate

Methyl 3-fluoro-4-nitrobenzoate (1.0 g, 5.0 mmol) was dissolved in DMF (1.8 mL), and (tetrahydro-2*H*-pyran-2-yl)methaneamine (0.868 mL) and K₂CO₃ (693 mg) were added at room temperature. The reactants were stirred for 4 hours at 50 °C. Water was added, and extraction was performed by using EtOAc. An organic layer was dried with Na₂SO₄, concentrated under reduced pressure, and purified by using MPLC, thereby obtaining methyl 4-nitro-3-(((tetrahydro-2*H-*pyran-2-yl)methyl)amino)benzoate (1.37 g, 4.9 mmol, 98%).

¹H MR (500 MHz, CDCl₃) δ 8.29 - 8.15 (m, 2H), 7.54 (d, *J* = 1.5 Hz, 1H), 7.22 (dd, *J=* 8.8, 1.8 Hz, 1H), 4.07 - 4.04 (m, 1H), 3.94 (s, 3H), 3.68 - 3.58 (m, 1H), 3.50 (td, *J* = 11.6, 2.4 Hz, 1H), 3.42 (ddd, *J* = 12.7, 5.7, 3.9 Hz, 1H), 3.37 - 3.27 (m, 1H), 2.00 - 1.84 (m, 1H), 1.72 - 1.40 (m, 5H); LC-MS(ESI): 295.31 [M+H]⁺

### [Preparation Example 35: Preparation of methyl 4-amino-3-(((tetrahydro-2H-pyran-2-yl)methyl)amino)benzoate]

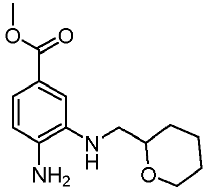

### methyl 4-amino-3-(((tetrahydro-2H-pyran-2-yl)methyl)amino)benzoate

The 4-nitro-3-(((tetrahydro-2*H*-pyran-2-yl)methyl)amino)benzoate (1.45 g, 4.94 mmol) obtained in Preparation Example 34 was dissolved in MeOH (50 mL) and DCM (30 mL), and 10% Pd/C (145 mg) was added, followed by stirring for 15 hours under hydrogen at room temperature. After the reaction was completed, Pd/C was filtered through Celite, and then concentration under reduced pressure was performed, thereby obtaining methyl 4-amino-3-(((tetrahydro-2*H*-pyran-2-yl)methyl)amino)benzoate (1.28 g, 4.84 mmol, 98%) .

¹H NMR (500 MHz, CDCl₃) δ 7.45 (dd, *J* = 8.1, 1.7 Hz, 1H), 7.33 (d*, J* = 1.8 Hz, 1H), 6.67 (d*, J* = 8.2 Hz, 1H), 4.09 - 3.97 (m, 1H), 3.93 - 3.69 (m, 5H), 3.62 - 3.55 (m, 1H), 3.53 - 3.42 (m, 1H), 3.19 (dd, *J=* 12.2, 3.1 Hz, 1H), 3.09 (dd, *J=* 12.2, 8.2 Hz, 1H), 1.91 - 1.80 (m, 1H), 1.68 - 1.39 (m, 5H); LC-MS(ESI): 265.32 [M+H]⁺

### [Preparation Example 36: Preparation of methyl 2-(4-(6-((4-chloro-2-fluorobenzyl)oxy)pyridin-2-yl)benzyl)-1-((tetrahydro-2H-pyran-2-yl)methyl)-1H-benzo[d]imidazole-6-carboxylate]

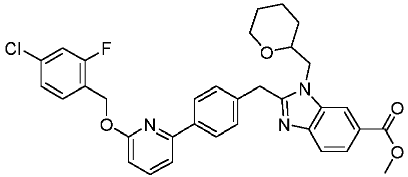

### methyl 2-(4-(6-((4-chloro-2-fluorobenzyl)oxy)pyridin-2-yl)benzyl)-1-((tetrahydro-2H-pyran-2-yl)methyl)-1H-benzo[d]imidazole-6-carboxylate

The methyl 4-amino-3-(((tetrahydro-2*H*-pyran-2-yl)methyl)amino)benzoate (158 mg, 0.6 mmol) obtained in Preparation Example 35 and the 2-(4-(6-((4-chloro-2-fluorobenzyl)oxy)pyridin-2-yl)phenyl)acetic acid (186 mg, 0.5 mmol) obtained in Preparation Example 2 were dissolved in 2 mL of DMF, and EDC (192 mg, 1.0 mmol) and HOBt (135 mg, 1.0 mmol) were added, followed by stirring for 24 hours at room temperature. After the reaction was completed, water was added, and extraction was performed with EtOAc. An organic layer was dried with Na₂SO₄ and concentrated under reduced pressure, thereby obtaining an intermediate, methyl 4-(2-(4-(6-((4-chloro-2-fluorobenzyl)oxy)pyridin-2-yl)phenyl)acetamido)-3-(((tetrahydro-2*H*-pyran-2-yl)methyl)amino)benzoate. The intermediate was dissolved in 10 mL of acetic acid without additional purification, and stirred for 2 hours at 120 °C. Water was added, and extraction was performed by using EtOAc. The organic layer was dried with Na₂SO₄, concentrated under reduced pressure, and purified by using MPLC, thereby obtaining methyl 2-(4-(6-((4-chloro-2-fluorobenzyl)oxy)pyridin-2-yl)benzyl)-1-((tetrahydro-2*H*-pyran-2-yl)methyl)-1*H-*benzo[d]imidazole-6-carboxylate (156 mg, 0.26 mmol, 52%).

¹H NMR (500 MHz, CDCl₃) δ 8.05 (s, 1H), 8.00 - 7.91 (m, 3H), 7.75 (d, *J*= 8.5 Hz, 1H), 7.63 (t, *J* = 7.5 Hz, 1H), 7.46 (t, *J* = 8.0 Hz, 1H), 7.35 - 7.29 (m, 3H), 7.15 - 7.06 (m, 2H), 6.72 (d, *J=* 8.2 Hz, 1H), 5.58 - 5.42 (m, 2H), 4.49 (s, 2H), 4.09 (d, *J=* 9.2 Hz, 1H), 4.06 - 4.00 (m, 1H), 3.94 (s, 3H), 3.93 - 3.86 (m, 1H), 3.55 - 3.44 (m, 1H), 3.25 - 3.16 (m, 1H), 1.84 (d, *J* = 11.9 Hz, 1H), 1.66-1.29 (5H); LC-MS(ESI): 600.36 [M+H]⁺

### [Preparation Example 37: Preparation of 4-(6-((4-chloro-2-fluorobenzyl)oxy)pyridin-2-yl)-3-fluorophenol]

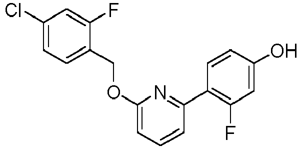

### 4-(6-((4-chloro-2-fluorobenzyl)oxy)pyridin-2-yl)-3-fluorophenol

The 2-chloro-6-((4-chloro-2-fluorobenzyl)oxy)pyridine (2.00 g, 7.35 mmol) obtained in Preparation Example 1, Pd(dppf)Cl₂-DCM (600 mg, 0.735 mmol), Cs₂CO₃ (5.99 g, 18.38 mmol), and 3-fluoro-4-(4,4,5,5-tetramethyl-1,3,2-dioxabororan-2-yl)phenol (1.84 g, 7.72 mmol) were dissolved in 22 mL of THF/H₂O (9/1), and subjected to nitrogen substitution. The reactants were stirred for 24 hours at 85 °C. After the reaction was completed, the resulting reaction product was cooled to room temperature, filtered through Celite using EtOAc, and concentrated under reduced pressure. The resulting concentrate was purified by MPLC, thereby obtaining 4-(6-((4-chloro-2-fluorobenzyl)oxy)pyridin-2-yl)-3-fluorophenol (2.12 g, 6.10 mmol, 83%).

¹H NMR (500 MHz, MeOD) δ 7.89 (t, *J* = 9.0 Hz, 1H), 7.69 (q, *J* = 7.7 Hz, 1H), 7.55 (t, *J=* 8.1 Hz, 1H), 7.39 (dd, *J* = 7.6, 1.5 Hz, 1H), 7.21-7.28 (m, 2H), 6.70-6.74 (m, 2H), 6.60 (td, *J* = 6.8, 2.2 Hz, 1H), 5.50 (d, *J* = 5.2 Hz, 2H).

### [Preparation Example 38: Preparation of methyl 2-(4-(6-((4-chloro-2-fluorobenzyl)oxy)pyridin-2-yl)-3-fluorophenyl)acetate]

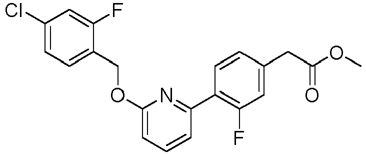

### methyl 2-(4-(6-((4-chloro-2-fluorobenzyl)oxy)pyridin-2-yl)-3-fluorophenyl)acetate

The 2-chloro-6-((4-chloro-2-fluorobenzyl)oxy)pyridine (673 mg, 2.47 mmol) obtained in Preparation Example 1, Pd(dppf)Cl₂-DCM (202 mg, 0.247 mmol), Cs₂CO₃ (2.01 g, 6.18 mmol), and methyl 2-(3-fluoro-4-(4,4,5,5-tetramethyl-1,3,2-dioxabororan-2-yl)phenyl)acetate (800 mg, 2.72 mmol) were dissolved in 8 mL of THF/H₂O (9/1), and subjected to nitrogen substitution. The reactants were stirred for 24 hours at 85 °C. After the reaction was completed, the resulting reaction product was cooled to room temperature, filtered through Celite using EtOAc, and concentrated under reduced pressure. The resulting concentrate was purified by using MPLC, thereby obtaining methyl 2-(4-(6-((4-chloro-2-fluorobenzyl)oxy)pyridin-2-yl)-3-fluorophenyl)acetate (720 mg, 1.78 mmol, 72%).

¹H NMR (500 MHz, MeOD) δ 7.99 (t, *J* = 8.1 Hz, 1H), 7.76 (t, *J* = 7.9 Hz, 1H), 7.47-7.57 (m, 2H), 7.16-7.27 (m, 4H), 6.83 (d, *J* = 8.2 Hz, 1H), 5.52 (s, 2H), 3.74 (d*, J* = 8.5 Hz, 5H).

### [Preparation Example 39: Preparation of 2-chloro-4-(6-((4-chloro-2-fluorobenzyl)oxy)pyridin-2-yl)phenol]

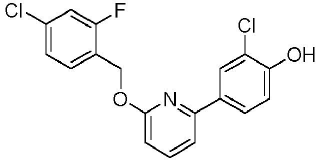

### 2-chloro-4-(6-((4-chloro-2-fluorobenzyl)oxy)pyridin-2-yl)phenol

The 2-chloro-6-((4-chloro-2-fluorobenzyl)oxy)pyridine (1.20 g, 4.41 mmol) obtained in Preparation Example 1, Pd(dppf)Cl₂-DCM (360 mg, 0.441 mmol), Cs₂CO₃ (3.59 g, 11.03 mmol), and (3-chloro-4-hydroxyphenyl)boronic acid (798 mg, 4.63 mmol) were dissolved in 18 mL of THF/H₂O (9/1), and subjected to nitrogen substitution. The reactants were stirred for 24 hours at 85 °C. After the reaction was completed, the resulting reaction product was cooled to room temperature, filtered through Celite by using EtOAc, and concentrated under reduced pressure. The resulting concentrate was purified by using MPLC, thereby obtaining 2-chloro-4-(6-((4-chloro-2-fluorobenzyl)oxy)pyridin-2-yl)phenol (250 mg, 0.686 mmol, 16%).

¹H NMR (500 MHz, CDCl₃) δ 8.04 (dd, *J* = 6.4, 2.1 Hz, 1H), 7.85 (dd, *J* = 8.5, 2.1 Hz, 1H), 7.63-7.67 (m, 1H), 7.43-7.58 (m, 2H), 7.16-7.22 (m, 2H), 7.11 (d, *J* = 8.5 Hz, 1H), 6.72 (dd, *J* = 14.2, 8.1 Hz, 1H), 5.54-5.58 (m, 2H).

### [Preparation Example 40: Preparation of 4-(6-((4-chloro-2-fluorobenzyl)oxy)pyridin-2-yl)-2-fluorophenol]

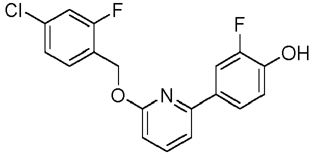

### 4-(6-((4-chloro-2-fluorobenzyl)oxy)pyridin-2-yl)-2-fluorophenol

The 2-chloro-6-((4-chloro-2-fluorobenzyl)oxy)pyridine (1.00 g, 3.68 mmol) obtained in Preparation Example 1, Pd(dppf)Cl₂-DCM (300 mg, 0.368 mmol), Cs₂CO₃ (2.99 g, 9.19 mmol) and (3-fluoro-4-hydroxyphenyl)boronic acid (602 mg, 3.86 mmol) were dissolved in 14 mL of THF/H₂O (9/1), and subjected to nitrogen substitution. The reactants were stirred for 24 hours at 85 °C. After the reaction was completed, the resulting reaction product was cooled to room temperature and filtered through Celite by using EtOAc, followed by concentration under reduced pressure. MPLC was performed, thereby obtaining 4-(6-((4-chloro-2-fluorobenzyl)oxy)pyridin-2-yl)-2-fluorophenol (190 mg, 0.546 mmol, 15%).

¹H NMR (500 MHz, MeOD) δ 7.79 (dd, *J=* 13.0, 2.0 Hz, 1H), 7.71 (t, *J* = 7.8 Hz, 1H), 7.54-7.57 (m, 1H), 7.37-7.42 (m, 2H), 7.22-7.30 (m, 2H), 6.97-7.01 (m, 1H), 6.73 (d, *J=* 8.2 Hz, 1H), 5.55 (d, *J=* 10.4 Hz, 2H).

### [Preparation Example 41: Preparation of methyl 2-(4-(6-((2-fluoro-4-(trifluoromethyl)benzyl)oxy)pyridin-2-yl)phenyl)acetate]

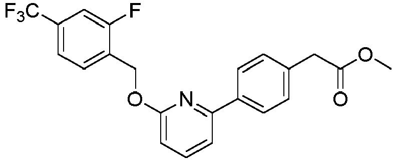

### methyl 2-(4-(6-((2-fluoro-4-(trifluoromethyl)benzyl)oxy)pyridin-2-yl)phenyl)acetate

2-chloro-6-((2-fluoro-4-(trifluoromethyl)benzyl)oxy)pyridine (1.78 g, 5.82 mmol), Pd(dppf)Cl₂-DCM (476 mg, 0.582 mmol), Cs₂CO₃ (4.74 g, 14.56 mmol), and methyl 2-(4-(4,4,5,5-tetramethyl-1,3,2-dioxaborolane-2-yl)phenyl)acetate (1.77 g, 6.41 mmol) were dissolved in 15 mL of THF/H₂O (9/1), and subjected to nitrogen substitution. The reactants were stirred for 24 hours at 85 °C. After the reaction was completed, the resulting reaction product was cooled to room temperature, filtered through Celite by using EtOAc, and concentrated under reduced pressure. The resulting concentrate was purified by using MPLC, thereby obtaining methyl 2-(4-(6-((2-fluoro-4-(trifluoromethyl)benzyl)oxy)pyridin-2-yl)phenyl)acetate (1.71 g, 4.07 mmol, 70%).

¹H NMR (400 MHz,CDCl₃) δ 7.94 (dd, *J* = 6.4, 1.8 Hz, 2H), 7.73-7.59 (m, 2H), 7.46-7.31 (m, 5H), 6.76 (d*, J* = 8.2 Hz, 1H), 5.68-5.53 (2H), 3.70 (s, 3H), 3.67 (d, *J=* 2.7 Hz, 2H).

### [Preparation Example 42: Preparation of methyl 4-nitro-3-(((4-propyl-4H-1,2,4-triazol-3-yl)methyl)amino)benzoate]

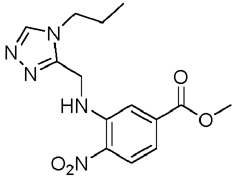

### methyl 4-nitro-3-(((4-propyl-4H-1,2,4-triazol-3-yl)methyl)amino)benzoate

Methyl 3-fluoro-4-nitrobenzoate (934 mg, 4.69 mmol) was dissolved in THF (10 mL)/MeOH (6.67 mL), and (4-propyl-4*H*-1,2,4-triazol-3-yl)methaneamine dihydrochloride (696 mg, 4.69 mmol) and TEA (3.27 mL, 23.46 mmol) were added, followed by stirring for 24 hours at room temperature under nitrogen. After the reaction was completed, the resulting reaction product was cooled to room temperature, and then the reaction solvent was concentrated under reduced pressure. Water was added, extraction was performed by using EtOAc, and drying was performed with MgSO₄, followed by concentration under reduced pressure. The resulting concentrate was purified by using MPLC, thereby obtaining methyl 4-nitro-3-(((4-propyl-4*H*-1,2,4-triazol-3-yl)methyl)amino)benzoate (918 mg, 2.87 mmol, 61%).

¹H NMR (500 MHz, MeOD) δ 8.54 (s, 1H), 8.27 (d, *J=* 8.8 Hz, 1H), 7.76 (d, *J* = 1.5 Hz, 1H), 7.34 (dd, *J* = 8.5, 1.5 Hz, 1H), 4.91 (d, *J* = 3.4 Hz, 2H), 4.15 (t, *J* = 7.5 Hz, 2H), 3.93 (s, 3H), 1.81-1.91 (m, 2H), 0.98 (t, *J=* 7.3 Hz, 3H).

### [Preparation Example 43: Preparation of methyl 4-amino-3-(((4-propyl-4H-1,2,4-triazol-3-yl)methyl)amino)benzoate]

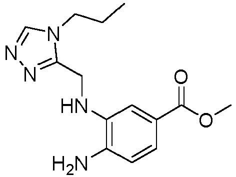

### methyl 4-amino-3-(((4-propyl-4H-1,2,4-triazol-3-yl)methyl)amino)benzoate

The methyl 4-nitro-3-(((4-propyl-4*H*-1,2,4-triazol-3-yl)methyl)amino)benzoate (918 mg, 2.87 mmol) obtained in Preparation Example 42 was dissolved in THF (10 mL), and 10% Pd/C (922 mg) was added, followed by stirring for 24 hours under hydrogen at room temperature. After the reaction was completed, the resulting reaction product was filtered through Celite by using EtOAc, and concentrated under reduced pressure. The resulting concentrate was purified by using MPLC, thereby obtaining methyl 4-amino-3-(((4-propyl-4*H*-1,2,4-triazol-3-yl)methyl)amino)benzoate (500 mg, 1.73 mmol, 60%).

¹H NMR (500 MHz, MeOD) δ 8.55 (d, *J* = 25.0 Hz, 1H), 7.39 (dd, *J* = 8.1, 1.7 Hz, 1H), 7.32-7.35 (m, 1H), 6.70 (d, *J=* 8.2 Hz, 1H), 4.62 (d*, J* = 18.3 Hz, 2H), 4.12 (t, *J=* 7.5 Hz, 2H), 3.80-3.88 (m, 3H), 1.82-1.91 (m, 2H), 0.92-1.02 (m, 3H).

### [Preparation Example 44: Preparation of methyl 2-mercapto-1-((tetrahydrofuran-2-yl)methyl)-1H-benzo[d]imidazole-6-carboxylate]

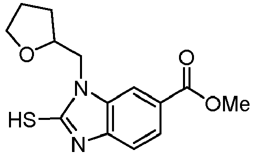

### methyl 2-mercapto-1-((tetrahydrofuran-2-yl)methyl)-1H-benzo[d]imidazole-6-carboxylate

The methyl 4-amino-3-(((tetrahydrofuran-2-yl)methyl)amino)benzoate (322 mg, 1.213 mmol) obtained in Preparation Example 4 was dissolved in THF (6.4 mL), and TCDI (344 mg, 1.930 mmol) was added, followed by stirring for 4.5 hours under nitrogen at room temperature. Water was added, extraction was performed with EtOAc, and drying was performed with Na₂SO₄, followed by concentration under reduced pressure. The resulting concentrate was performed by using MPLC, thereby obtaining methyl 2-mercapto-1-((tetrahydrofuran-2-yl)methyl)-1*H-*benzo[d]imidazole-6-carboxylate (321 mg, 1.098 mmol, 85%).

¹H NMR (500 MHz, CDCl₃) δ 11.34 (brs, 1H), 8.04 (s, 1H), 7.93 (d, *J* = 8.5 Hz, 1H), 7.24 (d*, J* = 8.2 Hz, 1H), 4.54 (dd, *J* = 14.3, 3.7 Hz, 1H), 4.38 - 4.48 (m, 1H), 4.22 - 4.33 (m, 1H), 3.94 (s, 3H), 3.89 (dd, *J=* 14.8, 6.6 Hz, 1H), 3.72 - 3.76 (m, 1H), 2.06 - 2.16 (m, 1H), 1.89 - 1.95 (m, 2H), 1.80 - 1.88 (m, 1H).

### [Preparation Example 45: Preparation of methyl 2-(benzylthio)-1-((tetrahydrofuran-2-yl)methyl)-1H-benzo[d]imidazole-6-carboxylate]

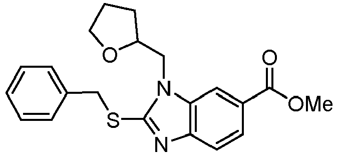

### methyl 2-(benzylthio)-1-((tetrahydrofuran-2-yl)methyl)-1H-benzo[d]imidazole-6-carboxylate

The methyl 2-mercapto-1-((tetrahydrofuran-2-yl)methyl)-1*H-*benzo[d]imidazole-6-carboxylate (320 mg, 1.095 mmol) obtained in Preparation Example 44 was dissolved in THF (6.8 mL), and NaH (60% dispersion in mineral oil, 88 mg, 2.189 mmol) was added at 0 °C, followed by stirring for 10 minutes under nitrogen at room temperature. BnBr (0.169 mL, 1.423 mmol) was slowly added dropwise, and stirring for 4 hours at room temperature. After the reaction was completed, water was added, extraction was performed with EtOAc, and drying was performed with Na₂SO₄, followed by concentration under reduced pressure. The resulting concentrate was purified by using MPLC, thereby obtaining methyl 2-(benzylthio)-1-((tetrahydrofuran-2-yl)methyl)-1*H*-benzo[*d*]imidazole-6-carboxylate (389 mg, 1.023 mmol, 93%).

¹H NMR (400 MHz, CDCl₃) δ 8.08 (d*, J* = 1.4 Hz, 1H), 7.96 (dd, *J* = 8.2, 1.4 Hz, 1H), 7.69 (d, *J* = 8.7 Hz, 1H), 7.27 - 7.44 (m, 5H), 4.66 (s, 2H), 4.22 - 4.29 (m, 1H), 4.10 - 4.19 (m, 2H), 3.94 (s, 3H), 3.82 - 3.87 (m, 1H), 3.69 - 3.75 (m, 1H), 1.99 (dt*, J* = 18.9, 6.7 Hz, 1H), 1.81 - 1.88 (m, 2H), 1.59 - 1.67 (m, 1H).

### [Preparation Example 46: Preparation of methyl 2-(benzylsulfonyl)-1-((tetrahydrofuran-2-yl)methyl)-1H-benzo[d]imidazole-6-carboxylate]

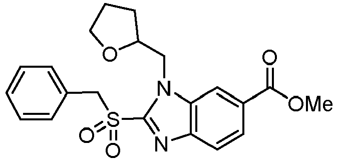

### methyl 2-(benzylsulfonyl)-1-((tetrahydrofuran-2-yl)methyl)-1H-benzo[d]imidazole-6-carboxylate

The methyl 2-(benzylthio)-1-((tetrahydrofuran-2-yl)methyl)-1*H-*benzo[d]imidazole-6-carboxylate (410 mg, 1.072 mmol) obtained in Preparation Example 45 was dissolved in CH₂Cl₂, and m-CPBA (555 mg, 3.22 mmol) was added at 0 °C, followed by stirring for 2 hours under nitrogen at room temperature. After the reaction was completed, the resulting reaction product was neutralized to pH 7 by adding NaHCO₃(aq). Extraction was performed with CH₂Cl₂, and drying was performed with Na₂SO₄, followed by concentration under reduced pressure. The resulting concentrate was purified by using MPLC, thereby obtaining methyl 2-(benzylsulfonyl)-1-((tetrahydrofuran-2-yl)methyl)-1*H*-benzo[*d*]imidazole-6-carboxylate (322 mg, 0.777 mmol, 72.5%).

¹H NMR (500 MHz, CDCl₃) δ 8.26 (s, 1H), 8.08 (dd, *J* = 8.5, 1.5 Hz, 1H), 7.92 (d, *J=* 8.5 Hz, 1H), 7.23 - 7.35 (m, 5H), 4.87 (dd, *J=* 19.8, 14.0 Hz, 2H), 4.27 - 4.38 (m, 2H), 4.06 (qd, *J* = 7.3, 3.4 Hz, 1H), 3.97 (s, 3H), 3.84 (q, *J* = 7.3 Hz, 1H), 3.66 (dd, *J* = 14.2, 7.8 Hz, 1H), 1.97 - 2.02 (m, 1H), 1.80 - 1.90 (m, 2H), 1.50 - 1.58 (m, 1H).

### [Preparation Example 47: Preparation of methyl 2-(4-(6-((4-chloro-2-fluorobenzyl)oxy)pyridin-2-yl)phenoxy)-1-((tetrahydrofuran-2-yl)methyl)-1H-benzo[d]imidazole-6-carboxylate]

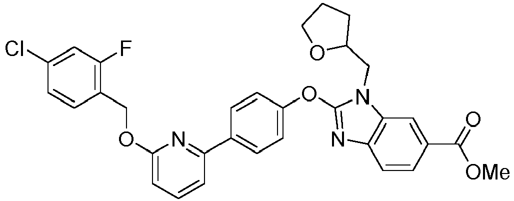

### methyl 2-(4-(6-((4-chloro-2-fluorobenzyl)oxy)pyridin-2-yl)phenoxy)-1-((tetrahydrofuran-2-yl)methyl)-1H-benzo[d]imidazole-6-carboxylate

The 4-(6-((4-chloro-2-fluorobenzyl)oxy)pyridin-2-yl)phenol (66 mg, 0.2 mmol) obtained in Preparation Example 20 was dissolved in DMF (2.94 mL), and t-BuOK (33.7 mg, 0.3 mmol) was added, followed by stirring for 10 minutes under nitrogen at room temperature. The methyl 2-(benzylsulfonyl)-1-((tetrahydrofuran-2-yl)methyl)-1*H*-benzo[*d*]imidazole-6-carboxylate (82.9 mg, 0.20 mmol) obtained in Preparation Example 46 was added, followed by stirring for 2 hours at room temperature. After the reaction was completed, water was added, the resulting solution was extracted with EtOAc, dried with Na₂SO₄, and concentrated under reduced pressure. The resulting concentrate was purified by using MPLC, thereby obtaining methyl 2-(4-(6-((4-chloro-2-fluorobenzyl)oxy)pyridin-2-yl)phenoxy)-1-((tetrahydrofuran-2-yl)methyl)-1*H*-benzo[*d*]imidazole-6-carboxylate (94.4 mg, 0.161 mmol, 80%).

¹H NMR (500 MHz, CDCl₃) δ 8.07 - 8.10 (m, 3H), 7.92 (dd, *J=* 8.2, 1.5 Hz, 1H), 7.67 (t, *J=* 7.8 Hz, 1H), 7.56 (d, *J=* 8.2 Hz, 1H), 7.46 - 7.50 (m, 3H), 7.36 (d, *J* = 7.6 Hz, 1H), 7.12 - 7.15 (m, 2H), 6.76 (d*, J* = 8.2 Hz, 1H), 5.54 (s, 2H), 4.37 - 4.42 (m, 1H), 4.28 (d*, J* = 5.8 Hz, 2H), 3.95 (s, 3H), 3.89 (dd, *J* = 15.1, 6.9 Hz, 1H), 3.79 (dd, *J* = 15.1, 6.9 Hz, 1H), 2.11 (dt, *J* = 19.2, 6.9 Hz, 1H), 1.90 - 1.96 (m, 2H), 1.74 - 1.81 (m, 1H).

### [Preparation Example 48: Preparation of methyl (S)-2-mercapto-1-((tetrahydrofuran-2-yl)methyl)-1H-benzo[d]imidazole-6-carboxylate]

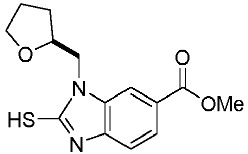

### methyl (S)-2-mercapto-1-((tetrahydrofuran-2-yl)methyl)-1H-benzo[d]imidazole-6-carboxylate

The methyl (*S*)-4-amino-3-(((tetrahydrofuran-2-yl)methyl)amino)benzoate (677 mg, 2.70 mmol) obtained in Preparation Example 30 was dissolved in THF (6.1 mL), and TCDI (723 mg, 4.06 mmol) was added, followed by stirring for 4 hours under nitrogen at room temperature. Water was added, extraction was performed with EtOAc, and drying was performed with Na₂SO₄, followed by concentration under reduced pressure. The resulting concentrate was purified by using MPLC, thereby obtaining methyl (*S*)-2-mercapto-1-((tetrahydrofuran-2-yl)methyl)-1*H-*benzo[d]imidazole-6-carboxylate (648 mg, 2.216 mmol, 82%).

¹H NMR (400 MHz, CDCl₃) δ 10.14 (brs, 1H), 8.03 (d, *J=* 1.4 Hz, 1H), 7.94 (dd, *J* = 8.5, 1.6 Hz, 1H), 7.21 (d, *J* = 8.2 Hz, 1H), 4.55 (dd, *J* = 14.2, 3.7 Hz, 1H), 4.44 (qd, *J* = 7.0, 3.7 Hz, 1H), 4.21 - 4.32 (m, 1H), 3.95 (s, 3H), 3.87 - 3.92 (m, 1H), 3.71 - 3.77 (m, 1H), 2.08 - 2.15 (m, 1H), 1.78 - 1.96 (m, 3H).

### [Preparation Example 49: Preparation of methyl (S)-2-(benzylthio)-1-((tetrahydrofuran-2-yl)methyl)-1H-benzo[d]imidazole-6-carboxylate]

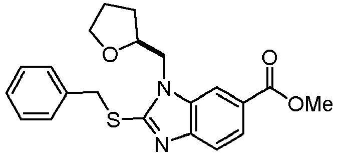

### methyl (S)-2-(benzylthio)-1-((tetrahydrofuran-2-yl)methyl)-1H-benzo[d]imidazole-6-carboxylate

The methyl (*S*)-2-mercapto-1-((tetrahydrofuran-2-yl)methyl)-1*H-*benzo[*d*]imidazole-6-carboxylate (458 mg, 2.2 mmol) obtained in Preparation Example 48 was dissolved in THF (10.0 mL), and NaH (60% dispersion in mineral oil, 176 mg, 4.4 mmol) was added at 0 °C, followed by stirring for 10 minutes under nitrogen at room temperature. BnBr (0.340 mL, 2.86 mmol) was slowly added dropwise, and stirred for 12 hours at room temperature. After the reaction was completed, water was added, extraction was performed with EtOAc, and drying was performed with Na₂SO₄, followed by concentration under reduced pressure. The resulting concentrate was purified by using MPLC, thereby obtaining methyl (S)-2-(benzylthio)-1-((tetrahydrofuran-2-yl)methyl)-1*H*-benzo[*d*]imidazole-6-carboxylate (590.8 mg, 1.98 mmol, 90%).

¹H NMR (500 MHz, CDCl₃) δ 8.07 (d*, J* = 0.6 Hz, 1H), 7.95 (dd, *J* = 8.4, 1.1 Hz, 1H), 7.68 (d, *J* = 8.2 Hz, 1H), 7.27 - 7.43 (m, 5H), 4.65 (s, 2H), 4.23 - 4.28 (m, 1H), 4.13 - 4.16 (m, 2H), 3.94 (s, 3H), 3.85 (q, *J=* 7.3 Hz, 1H), 3.70 - 3.76 (m, 1H), 1.98 (dt, *J=* 19.3, 6.8 Hz, 1H), 1.82 - 1.87 (m, 2H), 1.59 - 1.66 (m, 1H).

### [Preparation Example 50: Preparation of methyl (S)-2-(benzylsulfonyl)-1-((tetrahydrofuran-2-yl)methyl)-1H-benzo[d]imidazole-6-carboxylate]

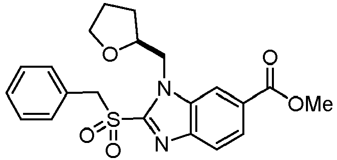

### methyl (S)-2-(benzylsulfonyl)-1-((tetrahydrofuran-2-yl)methyl)-1H-benzo[d]imidazole-6-carboxylate

The methyl (*S*)-2-(benzylthio)-1-((tetrahydrofuran-2-yl)methyl)-1*H-*benzo[*d*]imidazole-6-carboxylate (590 mg, 1.98 mmol) obtained in Preparation Example 49 was dissolved in CH₂Cl₂, and m-CPBA (1023 mg, 5.93 mmol) was added at 0 °C, followed by stirring for 5 hours under nitrogen at room temperature. After the reaction was completed, the resulting reaction product was neutralized to pH 7 by adding NaHCO₃(aq). Extraction was performed with CH₂Cl₂, and drying was performed with Na₂SO₄, followed by concentration under reduced pressure. The resulting concentrate was purified by using MPLC, thereby obtaining methyl (*S*)-2-(benzylsulfonyl)-1-((tetrahydrofuran-2-yl)methyl)-1*H*-benzo[*d*]imidazole-6-carboxylate (458 mg, 1.386 mmol, 70%).

¹H NMR (500 MHz, CDCl₃) δ 8.26 (s, 1H), 8.08 (d*, J* = 7.6 Hz, 1H), 7.92 (d, *J=* 8.5 Hz, 1H), 7.23 - 7.36 (m, 5H), 4.87 (dd*, J* = 20.1, 14.0 Hz, 2H), 4.27 - 4.37 (m, 2H), 4.06 (qd, *J* = 7.3, 3.4 Hz, 1H), 3.97 (s, 3H), 3.84 (dd, *J* = 15.0, 6.7 Hz, 1H), 3.66 (dd, *J* = 14.3, 7.6 Hz, 1H), 1.97 - 2.05 (m, 1H), 1.82 - 1.92 (m, 2H), 1.50 - 1.59 (m, 1H).

### [Preparation Example 51: Preparation of methyl (R)-4-nitro-3-(((tetrahydrofuran-3-yl)methyl)amino)benzoate]

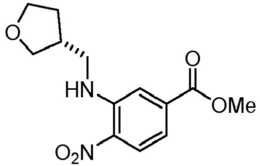

### methyl (R)-4-nitro-3-(((tetrahydrofuran-3-yl)methyl)amino)benzoate

Methyl 3-fluoro-4-nitrobenzoate (1315 mg, 6.60 mmol) was dissolved in DMF (5 mL), and (*R*)-(tetrahydrofuran-3-yl)methaneamine hydrochloride (1000 mg, 7.267 mmol) and K₂CO₃ (1004 mg, 7.267 mmol) were added at room temperature. The reactants were stirred for 4 hours at room temperature. Water was added, and extraction was performed with EtOAc. An organic layer was dried with Na₂SO₄, concentrated under reduced pressure and purified by using MPLC, thereby obtaining methyl (R)-4-nitro-3-(((tetrahydrofuran-3-yl)methyl)amino)benzoate (1.0 g, 3.57 mmol, 54%).

¹H NMR (500 MHz, CDCl₃) δ 8.20 (d*, J* = 8.8 Hz, 1H), 8.00 (s, 1H), 7.54 (s, 1H), 7.24 (dd, *J* = 8.8, 1.5 Hz, 1H), 3.88 - 3.98 (m, 5H), 3.79 (q, *J* = 7.8 Hz, 1H), 3.71 - 3.61 (m, 1H), 3.32 - 3.41 (m, 2H), 2.64 - 2.69 (m, 1H), 2.14 - 2.21 (m, 1H), 1.70 - 1.79 (m, 1H).

### [Preparation Example 52: Preparation of methyl (R)-4-amino-3-(((tetrahydrofuran-3-yl)methyl)amino)benzoate]

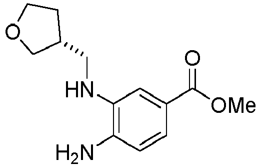

### methyl (R)-4-amino-3-(((tetrahydrofuran-3-yl)methyl)amino)benzoate

The methyl (R)-4-nitro-3-(((tetrahydrofuran-3-yl)methyl)amino)benzoate (1.0 g, 3.57 mmol) obtained in Preparation Example 51 was dissolved in 10 mL of THF, and 10% Pd/C (100 mg) was added, followed by stirring for 15 hours under hydrogen at room temperature. After the reaction was completed, Pd/C was filtered through Celite, and then concentration under reduced pressure was performed, thereby obtaining methyl (R)-4-amino-3-(((tetrahydrofuran-3-yl)methyl)amino)benzoate (879 mg, 3.51 mmol, 98%).

¹H NMR (500 MHz, CDCl₃) δ 7.60 (d*, J* = 7.9 Hz, 1H), 7.48 (s, 1H), 6.82 (d, *J* = 7.9 Hz, 1H), 4.02 - 4.11 (m, 2H), 3.99 (s, 3H), 3.84 - 3.97 (m, 3H), 3.79 (dd, *J* = 8.5, 5.5 Hz, 1H), 3.27 - 3.34 (m, 3H), 2.69 - 2.75 (m, 1H), 2.24 - 2.30 (m, 1H), 1.87 (dt*, J* = 19.4, 6.9 Hz, 1H).

### [Preparation Example 53: Preparation of methyl 2-(4-bromo-3-chlorophenyl)acetate]

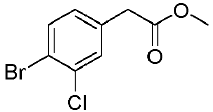

### methyl 2-(4-bromo-3-chlorophenyl)acetate

2-(4-bromo-3-chlorophenyl)acetic acid (5 g, 20.04 mmol) was dissolved in MeOH (100 mL), and sulfuric acid (1 mL) was added, followed by refluxing for 24 hours. NaHCO₃(aq) was added at 0 °C to neutralize the resulting product to pH 7, followed by concentration under reduced pressure. An organic material was extracted with CH₂Cl₂, and an organic layer was dried with Na₂SO₄, concentrated under reduced pressure and purified by using MPLC, thereby obtaining methyl 2-(4-bromo-3-chlorophenyl)acetate (4418 mg, 16.77 mmol, 84%).

¹H NMR (500 MHz, CDCl₃) δ 7.56 (d, *J* = 8.2 Hz, 1H), 7.39 (d, *J* = 1.8 Hz, 1H), 7.04 (dd, *J* = 8.1, 1.7 Hz, 1H), 3.71 (s, 3H), 3.57 (s, 2H).

### [Preparation Example 54: Preparation of methyl 2-(3-chloro-4-(4,4,5,5tetramethyl-1,3,2-dioxabororan-2-yl)phenyl)acetate]

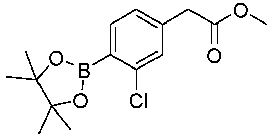

### methyl 2-(3-chloro-4-(4,4,5,5-tetramethyl-1,3,2-dioxaborolan-2-yl)phenyl)acetate

The methyl 2-(4-bromo-3-chlorophenyl)acetate (2108 mg, 8 mmol) obtained in Preparation Example 53 was dissolved in DMSO (47 mL), and 4,4,4',4',5,5,5',5'-octamethyl-2,2'-bi(1,3,2-dioxaborolane) (2437 mg, 9.6 mmol) and KOAc (2198 mg, 22.4 mmol) were added, followed by nitrogen substitution three times. Pd(dppf)Cl₂-DCM (653 mg, 0.8 mmol) was added, and nitrogen substitution was performed again, followed by stirring for 21 hours at 85 °C. After the reaction was completed, the resulting reaction product was cooled to room temperature and filtered through Celite by using EtOAc. Water was added, and extraction was performed with EtOAc. The extract was dried with Na₂SO₄, concentrated under reduced pressure and purified by using MPLC, thereby obtaining methyl 2-(3-chloro-4-(4,4,5,5-tetramethyl-1,3,2-dioxaborolane-2-yl)phenyl)acetate (1630 mg, 5.25 mmol, 65.6%).

¹H NMR (400 MHz, CDCl₃) δ 7.64 (d*, J* = 7.8 Hz, 1H), 7.27 (s, 1H), 7.14 (d, *J=* 7.8 Hz, 1H), 3.67 (s, 3H), 3.58 (s, 2H), 1.35 (brs, 12H).

### [Preparation Example 55: Preparation of methyl 2-(3-chloro-4-(6-((4-chloro-2-fluorobenzyl)oxy)pyridin-2-yl)phenyl)acetate]

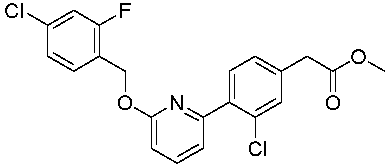

### methyl 2-(3-chloro-4-(6-((4-chloro-2-fluorobenzyl)oxy)pyridin-2-yl)phenyl)acetate

The 2-chloro-6-((4-chloro-2-fluorobenzyl)oxy)pyridine (1.19 g, 4.376 mmol) obtained in Preparation Example 1 was dissolved in THF/H₂O (38.7 mL/4.3 mL), and the methyl 2-(3-chloro-4-(4,4,5,5-tetramethyl-1,3,2-dioxaborolane-2-yl)phenyl)acetate (1.63 g, 5.25 mmol) obtained in Preparation Example 54 and Cs₂CO₃ (3.99 g, 12.25 mmol) were added, followed by nitrogen substitution three times. Pd(dppf)Cl₂-DCM (357 mg, 0.4376 mmol) was added, and nitrogen substitution was performed again, followed by stirring for 21 hours at 85 °C. After the reaction was completed, the resulting reaction product was cooled to room temperature, filtered through Celite by using EtOAc, and concentrated under reduced pressure. The resulting concentrate was purified by using MPLC, thereby obtaining methyl 2-(3-chloro-4-(6-((4-chloro-2-fluorobenzyl)oxy)pyridin-2-yl)phenyl)acetate (1198 mg, 2.85 mmol, 65.2%).

¹H NMR (400 MHz, CDCl₃) δ 7.65 (t, *J* = 8.0 Hz, 1H), 7.54 (d, *J* = 8.2 Hz, 1H), 7.45 (t, *J* = 8.2 Hz, 1H), 7.40 (d, *J* = 1.4 Hz, 1H), 7.23 - 7.27 (m, 2H), 7.09 - 7.13 (m, 2H), 6.78 (d, *J* = 8.2 Hz, 1H), 5.44 (s, 2H), 3.72 (s, 3H), 3.65 (s, 2H).

### [Preparation Example 56: Preparation of 2-(3-chloro-4-(6-((4-chloro-2-fluorobenzyl)oxy)pyridin-2-yl)phenyl)acetic acid]

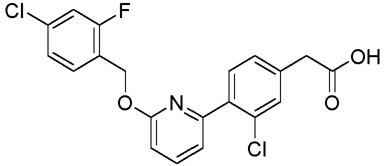

### 2-(3-chloro-4-(6-((4-chloro-2-fluorobenzyl)oxy)pyridin-2-yl)phenyl)acetic acid

The methyl 2-(3-chloro-4-(6-((4-chloro-2-fluorobenzyl)oxy)pyridin-2-yl)phenyl)acetate (1198 mg, 2.85 mmol) obtained in Preparation Example 55 was dissolved in 10 mL of MeOH, and 10 mL of IN NaOH was added. The reactants were stirred for 15 hours at 40 °C. After the reaction was completed, the pH was adjusted to pH 4 by using IN HCl, followed by extraction with EtOAc. The organic layer was dried with Na₂SO₄, concentrated under reduced pressure and purified by using MPLC, thereby obtaining 2-(3-chloro-4-(6-((4-chloro-2-fluorobenzyl)oxy)pyridin-2-yl)phenyl)acetic acid (1024 mg, 2.52 mmol, 88%).

¹H NMR (500 MHz, CDCl₃) δ 7.66 (t, *J* = 7.8 Hz, 1H), 7.56 (d, *J* = 7.9 Hz, 1H), 7.43 - 7.47 (m, 2H), 7.26 - 7.31 (m, 2H), 7.10 - 7.14 (m, 2H), 6.79 (d, *J* = 8.2 Hz, 1H), 5.45 (s, 2H), 3.70 (s, 2H).

### [Preparation Example 57: Preparation of methyl (S)-4-nitro-3-(((tetrahydrofuran-3-yl)methyl)amino)benzoate]

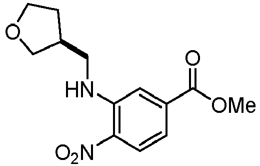

### methyl (S)-4-nitro-3-(((tetrahydrofuran-3-yl)methyl)amino)benzoate

Methyl 3-fluoro-4-nitrobenzoate (1 g, 5.02 mmol) was dissolved in DMF (2 mL), and (*S*)-(tetrahydrofuran-3-yl)methaneamine hydrochloride (760 mg, 5.522 mmol) and K₂CO₃ (762 mg, 5.52 mmol) were added at room temperature. The reactants were stirred for 19 hours at room temperature. Water was added, and extraction was performed by using EtOAc. An organic layer was dried with Na₂SO₄, concentrated under reduced pressure and purified by using MPLC, thereby obtaining methyl (*S*)-4-nitro-3-(((tetrahydrofuran-3-yl)methyl)amino)benzoate (1.198 g, 4.27 mmol, 85%).

¹H NMR (500 MHz, CDCl₃) δ 8.20 (d, *J* = 8.8 Hz, 1H), 8.05 (br s, 1H), 7.54 (s, 1H), 7.24 (dd, *J* = 8.8, 1.5 Hz, 1H), 3.89 - 3.98 (m, 5H), 3.79 (q, *J* = 7.8 Hz, 1H), 3.59 - 3.73 (m, 1H), 3.32 - 3.41 (m, 2H), 2.65 - 2.70 (m, 1H), 2.15 - 2.22 (m, 1H), 1.74 (td, *J=* 12.8, 7.3 Hz, 1H).

### [Preparation Example 58: Preparation of methyl (S)-4-amino-3-(((tetrahydrofuran-3-yl)methyl)amino)benzoate]

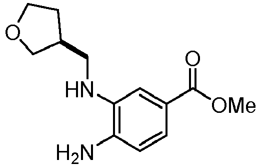

### methyl (S)-4-amino-3-(((tetrahydrofuran-3-yl)methyl)amino)benzoate

The methyl (*S*)-4-nitro-3-(((tetrahydrofuran-3-yl)methyl)amino)benzoate (1204 mg, 4.295 mmol) obtained in Preparation Example 57 was dissolved in 42 mL of THF, and 10% Pd/C (120 mg) was added, followed by stirring for 17 hours under hydrogen at room temperature. After the reaction was completed, Pd/C was filtered through Celite, and then concentration under reduced pressure was performed, thereby obtaining methyl (*S*)-4-amino-3-(((tetrahydrofuran-3-yl)methyl)amino)benzoate (1053 mg, 4.209 mmol, 98%).

¹H NMR (500 MHz, CDCl₃) δ 7.46 (dd, *J* = 7.9, 1.5 Hz, 1H), 7.35 (d*, J* = 1.5 Hz, 1H), 6.69 (d, *J=* 8.2 Hz, 1H), 3.90 - 3.94 (m, 2H), 3.86 (s, 3H), 3.71 - 3.81 (m, 3H), 3.66 (dd, *J* = 8.5, 5.5 Hz, 1H), 3.13 - 3.20 (m, 3H), 2.56 - 2.61 (m, 1H), 2.10 - 2.17 (m, 1H), 1.74 (td, *J=* 13.0, 7.1 Hz, 1H).

### [Preparation Example 59: Preparation of 4-(6-((4-chloro-2-fluorobenzyl)oxy)pyridin-2-yl)-2,6-difluorophenol]

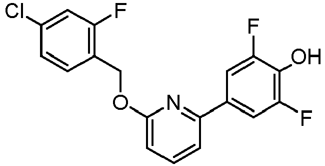

### 4-(6-((4-chloro-2-fluorobenzyl)oxy)pyridin-2-yl)-2,6-difluorophenol

The 2-chloro-6-((4-chloro-2-fluorobenzyl)oxy)pyridine (680 mg, 2.5 mmol) obtained in Preparation Example 1 was dissolved in THF/H₂O (15.8 mL/9.1 mL), and (3,5-difluoro-4-hydroxyphenyl)boronic acid (608.7 mg, 3.5 mmol) and Cs₂CO₃ (2280 mg, 7 mmol) were added, followed by nitrogen substitution three times. Pd(dppf)Cl₂-DCM (204 mg, 0.25 mmol) was added, nitrogen substitution was performed again, followed by stirring for 21 hours at 85 °C. After the reaction was completed, the resulting reaction product was cooled to room temperature, filtered through Celite by using EtOAc, and concentrated under reduced pressure. The resulting concentrate was purified by using MPLC, thereby obtaining 4-(6-((4-chloro-2-fluorobenzyl)oxy)pyridin-2-yl)-2,6-difluorophenol (586.3 mg, 1.603 mmol, 64.1%).

¹H NMR (500 MHz, CDCl₃) δ 7.57 - 7.65 (m, 3H), 7.46 (t, *J* = 7.9 Hz, 1H), 7.24 (d, *J=* 7.3 Hz, 1H), 7.14 (d, *J=* 8.5 Hz, 2H), 6.73 (d, *J=* 8.2 Hz, 1H), 5.50 (s, 2H).

### [Preparation Example 60: Preparation of methyl (S)-4-(2-(4-(6-((4-chloro-2-fluorobenzyl)oxy)pyridin-2-yl)phenyl)acetamido)-3-((oxetan-2-ylmethyl)amino)benzoate]

### methyl (S)-4-(2-(4-(6-((4-chloro-2-fluorobenzyl)oxy)pyridin-2-yl)phenyl)acetamido)-3-((oxetan-2-ylmethyl)amino)benzoate

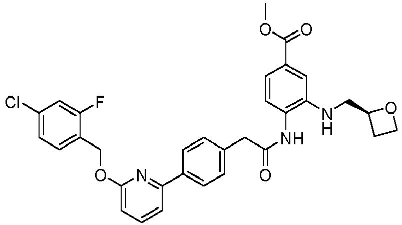

The methyl (*S*)-4-amino-3-((oxetan-2-ylmethyl)amino)benzoate (97 mg, 0.411 mmol) obtained in Preparation Example 7 was dissolved in DMF (3 mL), and 2-(4-(6-((4-chloro-2-fluorobenzyl)oxy)pyridin-2-yl)phenyl)acetic acid (139 mg, 0.374 mmol), EDC (143 mg, 0.748 mmol), and HOBt (28.6 mg, 0.187 mmol) were added. The resulting mixture was stirred for 24 hours under nitrogen at room temperature. After the reaction was completed, water was added, and the resulting solution was extracted with EtOAc, dried with MgSO₄ and concentrated under reduced pressure. The resulting concentrate was purified by using MPLC, thereby obtaining methyl (*S*)-4-(2-(4-(6-((4-chloro-2-fluorobenzyl)oxy)pyridin-2-yl)phenyl)acetamido)-3-((oxetan-2-ylmethyl)amino)benzoate (100 mg, 0.169 mmol, 45%).

¹H NMR (500 MHz, CDCl₃) δ 8.08 (d, *J* = 8.0 Hz, 2H), 7.80 (d, *J* = 8.5 Hz, 1H), 7.68 (t, *J=* 7.5 Hz, 1H), 7.57-7.55 (m, 2H), 7.47 (t, *J=* 7.3 Hz, 3H), 7.40 (d, *J* = 7.0 Hz, 1H), 7.15-7.13 (m, 2H), 6.77 (d, *J=* 8.0 Hz, 1H), 5.55 (s, 2H), 4.87-4.85 (m, 1H), 4.59-4.57 (m, 1H), 4.45-4.43 (m, 1H), 3.88-3.85 (m, 5H), 3.58 (br s, 1H), 3.28-3.25 (m, 1H), 3.17-3.14 (m, 1H), 2.59-2.55 (m, 1H), 2.40-2.36 (m, 1H).

### [Preparation Example 61: Preparation of methyl (S)-2-(4-(6-((4-chloro-2-fluorobenzyl)oxy)pyridin-2-yl)benzyl)-1-(oxetan-2-ylmethyl)-1H-benzo[d]imidazole-6-carboxylate]

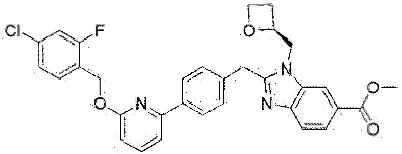

### methyl (S)-2-(4-(6-((4-chloro-2-fluorobenzyl)oxy)pyridine-2-yl)benzyl)-1-(oxetan-2-ylmethyl)-1H-benzo[d]imidazole-6-carboxylate

The methyl (*S*)-4-(2-(4-(6-((4-chloro-2-fluorobenzyl)oxy)pyridin-2-yl)phenyl)acetamido)-3-((oxetan-2-ylmethyl)amino)benzoate (100 mg, 0.169 mmol) obtained in Preparation Example 60 was dissolved in AcOH (2 mL, 34.9 mmol), and stirred for 3 hours at 120 °C. After the reaction was completed, the resulting reaction product was concentrated under reduced pressure. Water was added, extraction was performed by using EtOAc, and drying was performed with MgSO₄, followed by concentration under reduced pressure. The resulting concentrate was purified by using MPLC, thereby obtaining methyl (*S*)-2-(4-(6-((4-chloro-2-fluorobenzyl)oxy)pyridin-2-yl)benzyl)-1-(oxetan-2-ylmethyl)-1*H-*benzo[d]imidazole-6-carboxylate (89 mg, 0.156 mmol, 92%).

¹H NMR (500 MHz, CDCl₃) δ 8.07 (d, *J* = 1.0 Hz, 1H), 8.00-7.96 (m, 3H), 7.79 (d*, J* = 8.5 Hz, 1H), 7.64 (t, *J* = 7.8 Hz, 1H), 7.43-7.49 (m, 1H), 7.36-7.33 (m, 3H), 6.73-6.74 (m, 2H), 6.73 (d, *J* = 8.0 Hz, 1H), 5.52 (s, 2H), 5.09-5.07 (m, 1H), 4.62-4.50 (m, 3H), 4.40-4.35 (m, 2H), 4.33-4.27 (m, 1H), 3.94 (s, 3H), 2.66-2.64 (m, 1H), 2.36-2.32 (m, 1H).

### [Preparation Example 62: Preparation of methyl (S)-4-(2-(4-(6-((4-cyano-2-fluorobenzyl)oxy)pyridin-2-yl)phenyl)acetamido)-3-((oxetan-2-ylmethyl)amino)benzoate]

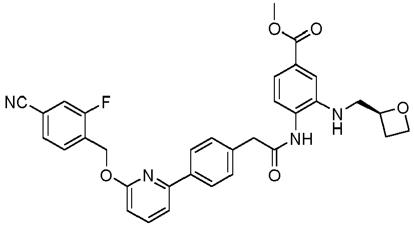

### methyl (S)-4-(2-(4-(6-((4-cyano-2-fluorobenzyl)oxy)pyridin-2-yl)phenyl)acetamido)-3-((oxetan-2-ylmethyl)amino)benzoate

The ethyl 2-(4-(6-((4-cyano-2-fluorobenzyl)oxy)pyridin-2-yl)phenyl)acetate (60 mg, 0.154 mmol) obtained in Preparation Example 9 was dissolved in EtOH/H₂O (1 mL/1 mL), and NaOH (18 mg, 0.461 mmol) was added, followed by stirring for 24 hours at room temperature. After the reaction was completed, water was added, and the resulting solution was neutralized to pH ~2 by using 1N-HCl. Extraction was performed by using EtOAc, drying was performed with MgSO₄, and concentration was performed under reduced pressure, thereby obtaining 2-(4-(6-((4-cyano-2-fluorobenzyl)oxy)pyridin-2-yl)phenyl)acetic acid (55 mg, 100%, 0.154 mmol) without purification .

Methyl (s)-4-amino-3-((oxetan-2-ylmethyl)amino)benzoate (88 mg, 0.373 mmol) was dissolved in DMF (2 mL), and 2-(4-(6-((4-cyano-2-fluorobenzyl)oxy)pyridin-2-yl)phenyl)acetic acid (123 mg, 0.339 mmol), EDC (195 mg, 1.018 mmol) and HOBt (26 mg, 0.170 mmol) were added. The resulting mixture was stirred for 24 hours under nitrogen at room temperature. After the reaction was completed, water was added, and the resulting solution was extracted with EtOAc, dried with MgSO₄ and concentrated under reduced pressure. The resulting concentrate was purified by using MPLC, thereby obtaining methyl (S)-4-(2-(4-(6-((4-cyano-2-fluorobenzyl)oxy)pyridin-2-yl)phenyl)acetamido)-3-((oxetan-2-ylmethyl)amino)benzoate (100 mg, 0.172 mmol, 51%).

¹H NMR (500 MHz, CDCl₃) δ 8.02 (m, 2H), 7.58-7.55 (m, 5H), 7.47-7.38 (m, 5H), 6.81 (d*, J* = 8.0 Hz, 1H), 5.64 (s, 2H), 4.89-4.88 (m, 1H), 4.62-4.59 (m, 1H), 4.47-4.44 (m, 1H), 3.88-3.84 (m, 5H), 3.28-3.27 (m, 1H), 3.19-3.16 (m, 1H), 2.63-2.56 (m, 1H), 2.44-2.37 (m, 1H).

### [Preparation Example 63: Preparation of methyl (S)-2-(4-(6-((4-cyano-2-fluorobenzyl)oxy)pyridin-2-yl)benzyl)-1-(oxetan-2-ylmethyl)-1H benzo[d]imidazole-6-carboxylate]

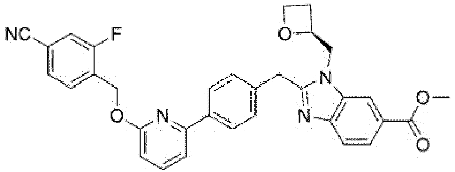

### methyl (S)-2-(4-(6-((4-cyano-2-fluorobenzyl)oxy)pyridine-2-yl)benzyl)-1-(oxetan-2-ylmethyl)-1H-benzo[d]imidazole-6-carboxylate

The methyl (*S*)-4-(2-(4-(6-((4-cyano-2-fluorobenzyl)oxy)pyridin-2-yl)phenyl)acetamido)-3-((oxetan-2-ylmethyl)amino)benzoate (100 mg, 0.172 mmol) obtained in Preparation Example 62 was dissolved in AcOH (2 mL, 34.9 mmol), and stirred for 3 hours at 120 °C. After the reaction was completed, concentration was performed under reduced pressure. Water was added, extraction was performed by using EtOAc, and drying was performed with MgSO₄, followed by concentration under reduced pressure. The resulting concentrate was purified by using MPLC, thereby obtaining methyl (*S*)-2-(4-(6-((4-cyano-2-fluorobenzyl)oxy)pyridin-2-yl)benzyl)-1-(oxetan-2-ylmethyl)-1*H*-benzo[*d*]imidazole-6-carboxylate (34 mg, 0.060 mmol, 35%).

¹H NMR (500 MHz, CDCl₃) δ 8.07 (brs, 1H), 7.99 (dd, *J* = 8.5, 1.5, 1H), 7.92 (d, 8.5 Hz, 2H), 7.79 (d, *J* = 8.5 Hz, 1H), 7.69 - 7.63 (m, 2H), 7.44 (d, *J* = 7.5 Hz, 1H), 7.41 - 7.30 (m, 4H), 6.77 (d, *J* = 8.0 Hz, 1H), 5.61 (s, 2H), 5.11 - 5.02 (m, 1H), 4.64 - 4.57 (m, 1H), 4.56 - 4.47 (m, 2H), 4.41 - 4.30 (m, 2H), 4.26 (dd, *J* = 15.5, 3.0 Hz, 1H), 3.96 (s, 3H), 2.70 - 2.61 (m, 1H), 2.41 - 2.28 (m, 1H).

### [Example 1: Preparation of 2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid]

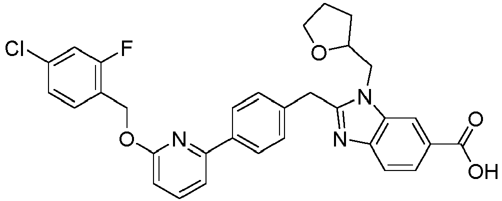

### 2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid

The methyl 2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylate (62.5 mg, 0.107 mmol) obtained in Preparation Example 5 was dissolved in 0.5 mL MeOH, and 0.5 mL of IN NaOH was added to the reactants and then stirred for 24 hours at 50 °C. IN HCl was added to adjust the pH to pH 4, followed by extraction with EtOAc. An organic layer was dried with Na₂SO₄, concentrated under reduced pressure and purified by using MPLC, thereby obtaining 2-(4-(6-((4-chloro-2-fluorobenzyl)oxy)pyridin-2-yl)benzyl)-1-((tetrahydrofuran-2-yl)methyl)-1*H-*benzo[d]imidazole-6-carboxylic acid (45.4 mg, 0.079 mmol, 74%).

¹H NMR (500 MHz, MeOD) δ 8.25 (s, 1H), 8.02 (d, *J=* 8.0 Hz, 2H), 7.97 (d, *J* = 8.5 Hz, 1H), 7.72 (t, *J* = 8.0 Hz, 1H), 7.67 (d, *J* = 8.5 Hz, 1H), 7.53 (t, *J* = 8.0 Hz, 1H), 7.46 (d*, J =* 7.5 Hz, 1H), 7.36 (d*, J* = 8.0 Hz, 2H), 7.27 - 7.14 (m, 2H), 6.76 (d, *J=* 8.5 Hz, 1H), 5.52 (s, 2H), 4.50 (s, 2H), 4.41 (dd, *J=* 15.5, 3.0 Hz, 1H), 4.25 (dd, *J* = 15.5, 8.5 Hz, 1H), 4.15 - 4.05 (m, 1H), 3.90 - 3.80 (m, 1H), 3.72 - 3.65 (m, 1H), 2.09 - 2.00 (m, 1H), 1.91 - 1.80 (m, 2H), 1.66 - 1.57 (m, 1H); LC-MS(ESI): 572.30 [M+H]⁺

### [Example 2: Preparation of (S)-2-(4-(6-((4-chloro-2-fluorobenzyl)oxy)pyridin-2-yl)benzyl)-1-(oxetan-2-ylmethyl)-1H benzo[d]imidazole-6-carboxylic acid]

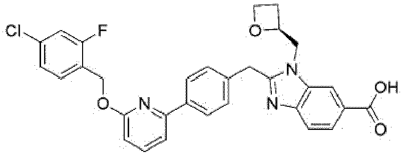

### (S)-2-(4-(6-((4-chloro-2-fluorobenzyl)oxy)pyridin-2-yl)benzyl)-1-(oxetan-2-ylmethly)-1H-benzo[d]imidazole-6-carboxylic acid

The methyl (*S*)-2-(4-(6-((4-chloro-2-fluorobenzyl)oxy)pyridin-2-yl)benzyl)-1-(oxetan-2-ylmethyl)-1*H*-benzo[*d*]imidazole-6-carboxylate (89 mg, 0.156 mmol) obtained in Preparation Example 61 was dissolved in MeOH/H₂O (2 mL/1 mL), and stirred for 24 hours at 50 °C. After the reaction was completed, water was added, and the resulting solution was acidified to pH ~2 using 1N-HCl. Extraction was performed using EtOAc, drying was performed with MgSO₄, and concentration was performed under reduced pressure. The resulting concentrate was purified by using MPLC, thereby obtaining (*S*)-2-(4-(6-((4-chloro-2-fluorobenzyl)oxy)pyridin-2-yl)benzyl)-1-(oxetan-2-ylmethyl)-1*H*-benzo[*d*]imidazole-6-carboxylic acid (30 mg, 0.054 mmol), 35%).

¹H NMR (500 MHz, CDCl₃) δ 8.17 (s, 1H), 8.07 (dd, *J* = 8.5, 1.5 Hz, 1H), 8.00 (d, *J* = 8.0 Hz, 2H), 7.86 (d, *J* = 8.5 Hz, 1H), 7.66 (t, *J* = 8.0 Hz, 1H), 7.48 (t, *J* = 8.0 Hz, 1H), 7.37 (m, 3H), 7.18 - 7.10 (m, 2H), 6.76 (d, *J* = 8.0 Hz, 1H), 5.54 (s, 2H), 5.14 - 5.08 (m, 1H), 4.67 - 4.54 (m, 3H), 4.43 - 4.36 (m, 2H), 4.30 (dd, *J=* 16.0, 3.0 Hz, 1H), 2.71 - 2.64 (m, 1H), 2.40 -2.33 (m, 1H); LC-MS(ESI): 558.27 [M+H]⁺

### [Example 3: Preparation of (S)-2-(4-(6-((4-cyano-2-fluorobenzyl)oxy)pyridin-2-yl)benzyl)-1-(oxetan-2-ylmethyl)-1H benzo[d]imidazole-6-carboxylic acid]

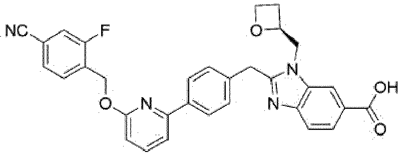

### (S)-2-(4-(6-((4-cyano-2-fluorobenzyl)oxy)pyridin-2-yl)benzyl)-1-(oxetan-2-ylmethyl)-1H-benzo[d]imidazole-6-carboxylic acid

The methyl (*S*)-2-(4-(6-((4-cyano-2-fluorobenzyl)oxy)pylidin-2-yl)benzyl)-1-(oxetan-2-ylmethyl)-1*H*-benzo[*d*]imidazole-6-carboxylate (34 mg, 0.060 mmol) obtained in Preparation Example 63 was dissolved in MeOH/H₂O (1 mL/1 mL), and stirred for 24 hours at 50 °C. After the reaction was completed, water was added, and the resulting solution was acidified to pH ~2 by using 1N-HCl. Extraction was performed by using EtOAc, and drying was performed with MgSO₄, followed by concentration under reduced pressure. The resulting concentrate was purified by using MPLC, thereby obtaining (*S*)-(2-(4-(6-((4-cyano-2-fluorobenzyl)oxy)pyridin-2-yl)benzyl)-1-(oxetan-2-ylmethyl)-1*H*-benzo[*d*]imidazole-6-carboxylic acid (4 mg, 0.007 mmol, 11%).

¹H NMR (500 MHz, MeOD) δ 8.17 (br s, 1H), 7.93 - 7.86 (m, 3H), 7.63 (t, *J* = 8.0 Hz, 1H), 7.59 - 7.51 (m, 4H), 7.37 (d, *J=* 7.5 Hz, 1H), 7.26 (d, *J=* 8.5 Hz, 2H), 6.70 (d, *J* = 8.0 Hz, 1H), 5.50 (s, 2H), 4.95 - 4.89 (m, 1H), 4.52 - 4.45 (m, 2H), 4.43 (s, 2H), 4.37 - 4.29 (m, 2H), 2.61 - 2.53 (m, 1H), 2.33 - 2.66 (m, 1H)

### [Example 4: Preparation of 2-(4-(6-((4-cyano-2-fluorobenzyl)oxy)pyridin-2-yl)benzyl)-1-((1-ethyl-1H-imidazol-5-yl)methyl)-1H-benzo[d]imidazole-6-carboxylic acid]

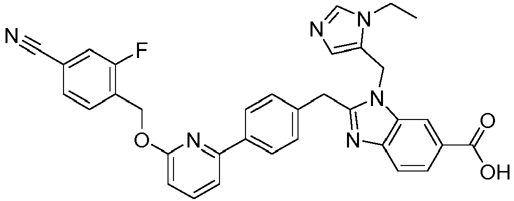

### 2-(4-(6-((4-cyano-2-fluorobenzyl)oxy)pyridin-2-yl)benzyl)-1-((1-ethyl-1H-imidazol-5-yl)methyl)-1H-benzo[d]imidazole-6-carboxylic acid

The methyl 4-amino-3-(((1-ethyl-1*H*-imidazol-5-yl)methyl)amino)benzoate (200 mg, 0.729 mmol) obtained in Preparation Example 11 and 2-(4-(6-((4-cyano-2-fluorobenzyl)oxy)pyridin-2-yl)phenyl)acetic acid (220 mg, 0.608 mmol) were dissolved in 3 mL of DMF, and EDC (232 mg, 1.215 mmol) and HOBt (164 mg, 1.215 mmol) were added, followed by stirring for 16 hours at room temperature. After the reaction was completed, water was added, followed by extraction with EtOAc. An organic layer was dried with Na₂SO₄, and concentrated under reduced pressure, thereby obtaining an intermediate, methyl 4-(2-(4-(6-((4-cyano-2-fluorobenzyl)oxy)pyridin-2-yl)phenyl)acetamido)-3-(((1-ethyl-1*H*-imidazol-5-yl)methyl)amino)benzoate. The intermediate was dissolved in 20 mL of acetic acid without additional purification, and stirred for 2 hours at 120 °C. Water was added, and extraction was performed with EtOAc. An organic layer was dried with Na₂SO₄, and concentrated under reduced pressure, thereby obtaining an intermediate, methyl 2-(4-(6-((4-cyano-2-fluorobenzyl)oxy)pyridin-2-yl)benzyl)-1-((1-ethyl-1*H-*imidazol-5-yl)methyl)-1*H*-benzo[*d*]imidazole-6-carboxylate. The intermediate was dissolved in 0.5 mL of MeOH without additional purification, 0.5 mL of IN NaOH was added to the reactants and stirred for 24 hours at 50 °C. 1N HCl was added to adjust the resulting solution to pH 4, followed by extraction with EtOAc. An organic layer was dried with Na₂SO₄, concentrated under reduced pressure and purified by using MPLC, thereby obtaining 2-(4-(6-((4-cyano-2-fluorobenzyl)oxy)pyridin-2-yl)benzyl)-1-((1-ethyl-1*H*-imidazol-5-yl)methyl)-1*H-*benzo[d]imidazole-6-carboxylic acid (10 mg, 0.017 mmol, 2.8%).

¹H NMR (500 MHz, MeOD) δ 8.09 (brs, 1H), 7.99 (brs, 1H), 7.91 (d, *J* = 8.0 Hz, 2H), 7.76 - 7.69 (m, 3H), 7.64 - 7.58 (m, 2H), 7.55 (d, *J* = 8.0 Hz, 1H), 7.45 (d, *J* = 7.5 Hz, 1H), 7.28 (d*, J* = 8.0 Hz, 2H), 6.81 (d*, J* = 8.0 Hz, 1H), 6.44 (s, 1H), 5.62 (s, 2H), 5.55 (s, 2H), 3.85 (q, *J* = 7.5 Hz, 2H), 1.17 (t, *J* = 7.5 Hz, 3H); LC-MS(ESI): 587.37 [M+H]⁺

### [Example 5: Preparation of 2-(4-(6-((4-cyano-2-fluorobenzyl)oxy)pyridin-2-yl)phenoxy)-1-((1-ethyl-1H-imidazol-5-yl)methyl)-1H-benzo[d]imidazole-6-carboxylic acid]

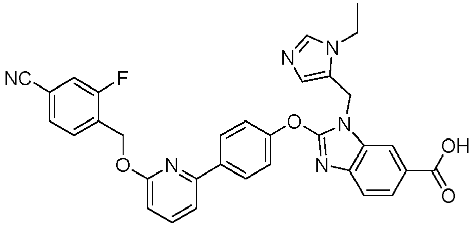

### 2-(4-(6-((4-cyano-2-fluorobenzyl)oxy)pyridin-2-yl)phenoxy)-1-((1-ethyl-1H-imidazol-5-yl)methyl)-1H-benzo[d]imidazole-6-carboxylic acid

The methyl 2-(4-(6-((4-cyano-2-fluorobenzyl)oxy)pyridin-2-yl)phenoxy)-1-((1-ethyl-1*H*-imidazol-5-yl)methyl)-1*H*-benzo[*d*]imidazole-6-carboxylate (10 mg, 0.017 mmol) obtained in Preparation Example 15 was dissolved in MeOH/H₂O (1 mL/1 mL), and stirred for 24 hours at 50 °C. After the reaction was completed, water was added, and 1N-HCl was used to acidify the resulting solution to pH ~2. Extraction was performed by using EtOAc and dried with MgSO₄, followed by concentration under reduced pressure. The resulting concentrate was purified by using MPLC, thereby obtaining 2-(4-(6-((4-cyano-2-fluorobenzyl)oxy)pyridin-2-yl)phenoxy)-1-((1-ethyl-1*H*-imidazol-5-yl)methyl)-1*H*-benzo[*d*]imidazole-6-carboxylic acid (5 mg, 0.008 mmol, 51%).

¹H NMR (400 MHz, CDCl₃) δ 8.75 (s, 1H), 8.18-8.14 (m, 1H), 7.97-7.91 (m, 4H), 7.87-7.82 (m, 1H), 7.70-7.64 (m, 1H), 7.58-7.50 (m, 4H), 7.38-7.35 (m, 1H), 6.93-6.91 (m, 1H), 6.71 (d, *J* = 8.4 Hz, 1H), 5.59 (s, 2H), 4.34 (s, 2H), 4.26 (q, *J* = 7.6 Hz, 2H), 1.52 (t, *J=* 7.0 Hz, 3H).

### [Example 6: Preparation of (S)-2-(4-(6-((4-cyano-2-fluorobenzyl)oxy)pyridin-2-yl)phenoxy)-1-(oxetan-2-ylmethyl)-1H-benzo[d]imidazole-6-carboxylic acid]

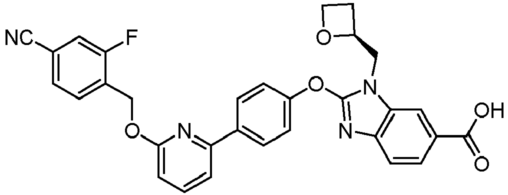

### (S)-2-(4-(6-((4-cyano-2-fluorobenzyl)oxy)pyridin-2-yl)phenoxy)-1-(oxetan-2-ylmethyl)-1H-benzo[d]imidazole-6-carboxylic acid

The methyl (*S*)-2-(4-(6-((4-cyano-2-fluorobenzyl)oxy)pyridin-2-yl)phenoxy)-1-(oxetan-2-ylmethyl)-1*H*-benzo[*d*]imidazole-6-carboxylate (40 mg, 0.071 mmol) obtained in Preparation Example 19 was dissolved in MeOH/H₂O (1 mL/1 mL), and NaOH (9 mg, 0.213 mmol) was added, followed by stirring for 24 hours at 50 °C. After the reaction was completed, water was added, and the resulting solution was acidified to pH ~2 by using 1N-HCl. Extraction was performed using EtOAc, and drying was performed with MgSO₄, followed by concentration under reduced pressure. The resulting concentrate was purified by using MPLC, thereby obtaining (*S*)-2-(4-(6-((4-cyano-2-fluorobenzyl)oxy)pyridin-2-yl)phenoxy)-1-(oxetan-2-ylmethyl)-1*H*-benzo[*d*]imidazole-6-carboxylic acid (20 mg, 0.036 mmol, 52%).

¹H NMR (500 MHz, MeOD) δ 8.24 (s, 1H), 8.19 (d, *J* = 8.8 Hz, 2H), 7.91-7.94 (m, 1H), 7.80 (t, *J* = 7.8 Hz, 1H), 7.66-7.71 (m, 3H), 7.55 (d, *J* = 7.6 Hz, 1H), 7.48 (dd, *J* = 8.4, 6.6 Hz, 3H), 6.86 (d, *J* = 8.2 Hz, 1H), 5.64 (d, *J* = 20.1 Hz, 2H), 5.28-5.31 (m, 1H), 4.60-4.70 (m, 2H), 4.47-4.54 (m, 2H), 2.86-2.91 (m, 1H), 2.62-2.68 (m, 1H).

### [Example 7: Preparation of (S)-2-(4-(6-((4-chloro-2-fluorobenzyl)oxy)pyridin-2-yl)phenoxy)-1-(oxetan-2-ylmethyl)-1H-benzo[d]imidazole-6-carboxylic acid]

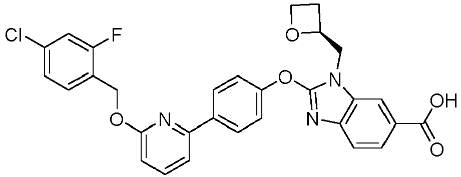

### (S)-2-(4-(6-((4-chloro-2-fluorobenzyl)oxy)pyridin-2-yl)phenoxy)-1-(oxetan-2-ylmethyl)-1H-benzo[d]imidazole-6-carboxylic acid

The methyl (*S*)-2-(4-(6-((4-chloro-2-fluorobenzyl)oxy)pyridin-2-yl)phenoxy)-1-(oxetan-2-ylmethyl)-1*H*-benzo[*d*]imidazole-6-carboxylate (86 mg, 0.150 mmol) obtained in Preparation Example 21 was dissolved in MeOH/H₂O (1 mL/1 mL), and NaOH (18 mg, 0.449 mmol) was added, followed by stirring for 24 hours at room temperature. After the reaction was completed, water was added, and the resulting solution was acidified to pH ~2 by using 1N-HCl. Extraction was performed by using EtOAc, and drying was performed with MgSO₄, followed by concentration under reduced pressure. The resulting concentrate was purified by using MPLC, thereby obtaining (*S*)-2-(4-(6-((4-chloro-2-fluorobenzyl)oxy)pyridin-2-yl)phenoxy)-1-(oxetan-2-ylmethyl)-1*H*-benzo[*d*]imidazole-6-carboxylic acid (62 mg, 0.111 mmol, 74%).

¹H NMR (500 MHz, CDCl₃) δ 8.23 (s, 1H), 8.11-8.14 (m, 2H), 8.01-8.03 (m, 1H), 7.69 (t, *J* = 7.8 Hz, 1H), 7.63 (d*, J* = 8.2 Hz, 1H), 7.45-7.52 (m, 3H), 7.34-7.39 (m, 1H), 7.15-7.17 (m, 2H), 6.79 (d, *J* = 8.2 Hz, 1H), 5.53 (d*, J* = 34.5 Hz, 2H), 5.29-5.33 (m, 1H), 4.72 (dd, *J=* 14.2, 7.8 Hz, 1H), 4.47-4.57 (m, 3H), 2.83-2.91 (m, 1H), 2.61-2.68 (m, 1H); LC-MS(ESI): 560.26 [M+H]⁺

### [Example 8: Preparation of 2-(4-(6-((4-chloro-2-fluorobenzyl)oxy)pyridin-2-yl)benzyl)-1-(oxazol-5-ylmethyl)-1H-benzo[d]imidazole-6-carboxylic acid]

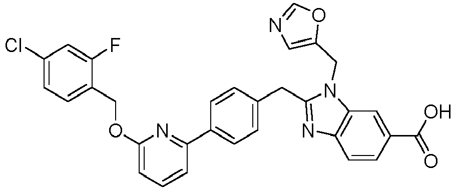

### 2-(4-(6-((4-chloro-2-fluorobenzyl)oxy)pyridin-2-yl)benzyl)-1-(oxazol-5-ylmethyl)-1H-benzo[d]imidazole-6-carboxylic acid

The methyl 2-(4-(6-((4-chloro-2-fluorobenzyl)oxy)pyridin-2-yl)benzyl)-1-(oxazol-5-ylmethyl)-1*H*-benzo[*d*]imidazole-6-carboxylate (49 mg, 0.084 mmol) obtained in Preparation Example 24 was dissolved in MeOH/H₂O (1 mL/1 mL), and NaOH (10 mg, 0.252 mmol) was added, followed by stirring for 24 hours at room temperature. After the reaction was completed, water was added, and the resulting solution was acidified to pH ~2 by using 1N-HCl. Extraction was performed by using EtOAc, and drying was performed with MgSO₄, followed by concentration with reduced pressure. The resulting concentrate was purified by using MPLC, thereby obtaining 2-(4-(6-((4-chloro-2-fluorobenzyl)oxy)pyridin-2-yl)benzyl)-1-(oxazol-5-ylmethyl)-1*H*-benzo[*d*]imidazole-6-carboxylic acid (27 mg, 0.047 mmol, 57%).

¹H NMR (500 MHz, MeOD) δ 8.50 (s, 1H), 8.24 (s, 1H), 8.03 (s, 1H), 7.98 (d, *J* = 8.5 Hz, 1H), 7.66 (d, *J* = 8.5 Hz, 1H), 7.59 - 7.5 5 (m, 4H), 7.41 (t, *J* = 7.8 Hz, 2H), 7.32 (d, *J* = 7.5 Hz, 3H), 6.88 (s, 1H), 5.58 (s, 2H), 4.52 (s, 2H); LC-MS(ESI): 569.26 [M+H]⁺

### [Example 9: Preparation of 2-(4-(6-((4-cyano-2-fluorobenzyl)oxy)pyridin-2-yl)phenoxy)-1-(oxazol-5-ylmethyl)-1H-benzo[d]imidazole-6-carboxylic acid]

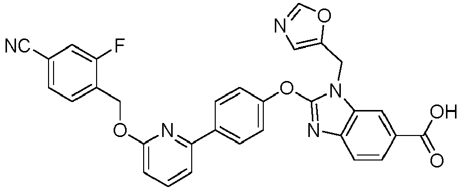

### 2-(4-(6-((4-cyano-2-fluorobenzyl)oxy)pyridin-2-yl)phenoxy)-1-(oxazol-5-ylmethyl)-1H-benzo[d]imidazole-6-carboxylic acid

The methyl 2-(4-(6-((4-cyano-2-fluorobenzyl)oxy)pyridin-2-yl)phenoxy)-1-(oxazol-5-ylmethyl)-1*H*-benzo[*d*]imidazole-6-carboxylate (64 mg, 0.111 mmol) obtained in Preparation Example 27 was dissolved in MeOH/H₂O (1 mL/1 mL), and NaOH (14 mg, 0.334 mmol) was added, followed by stirring for 24 hours at room temperature. After the reaction was completed, water was added, and the resulting solution was acidified to pH ~2 by using 1N-HCl. Extraction was performed by using EtOAc, and drying was performed with MgSO₄, followed by concentration under reduced pressure. The resulting concentrate was purified by using MPLC, thereby obtaining 2-(4-(6-((4-cyano-2-fluorobenzyl)oxy)pyridin-2-yl)phenoxy)-1-(oxazol-5-ylmethyl)-1*H*-benzo[*d*]imidazole-6-carboxylic acid (27 mg, 0.048 mmol, 43%).

¹H NMR (500 MHz, CDCl₃) δ 8.14 (s, 1H), 8.07 (d*, J* = 8.5 Hz, 2H), 8.01 (d, *J* = 8.0 Hz, 1H), 7.88 (s, 1H), 7.72-7.66 (m, 2H), 7.61 (d, *J* = 8.0 Hz, 1H), 7.48-7.47 (m, 3H), 7.41-7.38 (m, 2H), 7.22 (s, 1H), 6.81 (d*, J* = 8.5 Hz, 1H), 5.64 (s, 2H), 5.46 (s, 2H)

### [Example 10: Preparation of (S)-2-(4-(6-((4-chloro-2-fluorobenzyl)oxy)pyridin-2-yl)-2-fluorobenzyl)-1-(oxetan-2-ylmethyl)-1H-benzo[d]imidazole-6-carboxylic acid]

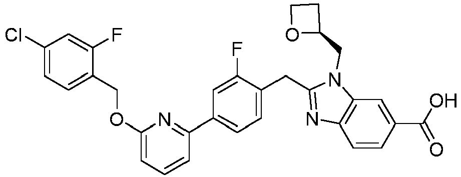

### (S)-2-(4-(6-((4-chloro-2-fluorobenzyl)oxy)pyridin-2-yl)-2-fluorobenzyl)-1-(oxetan-2-ylmethyl)-1H-benzo[d]imidazole-6-carboxylic acid

The 2-(4-(6-((4-chloro-2-fluorobenzyl)oxy)pyridin-2-yl)-2-fluorophenyl)acetic acid (150 mg, 0.385 mmol) obtained in Preparation Example 28 was dissolved in DMF (2.5 mL), and the methyl (*S*)-4-amino-3-((oxetan-2-ylmethyl)amino)benzoate (91 mg, 0.385 mmol) obtained in Preparation Example 7, HATU (439 mg, 1.154 mmol) and TEA (161 ml, 1.154 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl (*S*)-4-(2-(4-(6-((4-chloro-2-fluorobenzyl)oxy)pyridin-2-yl)-2-fluorophenyl)acetamido)-3-((oxetan-2-ylmethyl)amino)benzoate. The intermediate was dissolved in AcOH (4.4 mL, 77 mmol) without additional purification and stirred for 3 hours at 120 °C. After the reaction was completed, concentration was performed under reduced pressure, water was added, and then the resulting solution was extracted with EtOAc, dried with MgSO₄ concentrated under reduced pressure, thereby obtaining an intermediate, methyl (*S*)-2-(4-(6-((4-chloro-2-fluorobenzyl)oxy)pyridin-2-yl)-2-fluorobenzyl)-1-(oxetan-2-ylmethyl)-1*H*-benzo[*d*]imidazole-6-carboxylate without additional purification. Without additional purification, the intermediate was dissolved in THF/H₂O (1 mL/1 mL), and NaOH (34 mg, 0.859 mmol) was added, followed by stirring for 24 hours. After the reaction was completed, water was added, and the resulting solution was acidified to pH ~2 by using 1N-HCl. Extraction was performed by using EtOAc, and drying was performed with MgSO₄, followed by concentration under reduced pressure. The resulting concentrate was purified by using MPLC, thereby obtaining (*S*)-2-(4-(6-((4-chloro-2-fluorobenzyl)oxy)pyridin-2-yl)-2-fluorobenzyl)-1-(oxetan-2-ylmethyl)-1H-benzo[d]imidazole-6-carboxylic acid (50 mg, 0.087 mmol, 30%).

¹H NMR (500 MHz, MeOD) δ 8.29 (s, 1H), 7.97 (d, *J =* 8.5 Hz, 1H), 7.84-7.89 (m, 2H), 7.76 (t, *J =* 7.8 Hz, 1H), 7.65 (d, *J =* 8.5 Hz, 1H), 7.48-7.57 (m, 2H), 7.36 (q, *J* = 8.1 Hz, 1H), 7.21-7.26 (m, 2H), 6.82 (d, *J =* 8.2 Hz, 1H), 5.49-5.57 (m, 2H), 4.60 (d, *J =* 16.5 Hz, 1H), 4.41-4.50 (m, 2H), 4.29 (dd, *J =* 14.8, 9.3 Hz, 1H), 3.75-3.80 (m, 2H), 1.85-1.89 (m, 1H), 1.77 (td, *J=* 14.2, 5.5 Hz, 1H); LC-MS(ESI): 594.33 [M+H+H₂O]⁺

### [Example 11: Preparation of 2-(4-(6-((4-chloro-2-fluorobenzyl)oxy)pyridin-2-yl)-2-fluorobenzyl)-1-(oxazol-5-ylmethyl)-1H-benzo[d]imidazole-6-carboxylic acid]

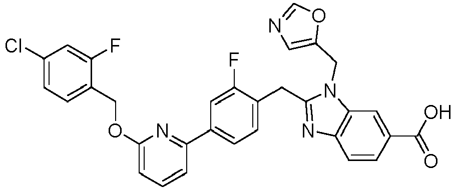

### 2-(4-(6-((4-chloro-2-fluorobenzyl)oxy)pyridin-2-yl)-2-fluorobenzyl)-1-(oxazol-5-ylmethyl)-1H-benzo[d]imidazole-6-carboxylic acid

The 2-(4-(6-((4-chloro-2-fluorobenzyl)oxy)pyridin-2-yl)-2-fluorophenyl)acetic acid (150 mg, 0.385 mmol) obtained in Preparation Example 28 was dissolved in DMF (2.5 mL), and the methyl 4-amino-3-((oxazol-5-ylmethyl)amino)benzoate (95 mg, 0.385 mmol) obtained in Preparation Example 23, HATU (439 mg, 1.154 mmol) and TEA (0.161 ml, 1.154 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(4-(6-((4-chloro-2-fluorobenzyl)oxy)pyridin-2-yl)-2-fluorophenyl)acetamido)-3-((oxazol-5-ylmethyl)amino)benzoate. The intermediate was dissolved in AcOH (4.4 mL, 77 mmol) without additional purification, and stirred for 3 hours at 120 °C. After the reaction, the intermediate was concentrated under reduced pressure, water was added, and the resulting solution was extracted with EtOAc, dried with MgSO₄ and concentrated under reduced pressure, thereby obtaining an intermediate, methyl 2-(4-(6-((4-chloro-2-fluorobenzyl)oxy)pyridin-2-yl)-2-fluorobenzyl)-1-(oxazol-5-ylmethyl)-1H-benzo[d]imidazole-6-carboxylate without additional purification. The intermediate was dissolved in THF/H₂O (1 mL/1 mL) without additional purification, and NaOH (17 mg, 0.424 mmol) was added, followed by stirring for 24 hours at room temperature. After the reaction was completed, water was added, and the resulting solution was acidified to pH ~2 using 1N-HCl. Extraction was performed by using EtOAc, and drying was performed with MgSO₄, followed by concentration under reduced pressure. The resulting concentrate was purified by using MPLC, thereby obtaining 2-(4-(6-((4-chloro-2-fluorobenzyl)oxy)pyridin-2-yl)-2-fluorobenzyl)-1-(oxazol-5-ylmethyl)-1*H*-benzo[*d*]imidazole-6-carboxylic acid (48 mg, 0.082 mmol, 58%).

¹H NMR (500 MHz, MeOD) δ 8.32 (s, 1H), 8.07 (s, 1H), 7.98 (dd, *J* = 8.5, 1.5 Hz, 1H), 7.85-7.79 (m, 2H), 7.74 (t, *J =* 8.0 Hz, 1H), 7.66 (d, *J =* 8.0 Hz, 1H), 7.55-7.48 (m, 3H), 7.29-7.19 (m, 3H), 7.07 (s, 1H), 6.80 (d, *J* = 8.0 Hz, 1H), 5.70 (s, 2H), 5.47 (s, 2H), 4.54 (s, 2H); LC-MS(ESI): 587.27 [M+H]⁺

### [Example 12: Preparation of (S)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid]

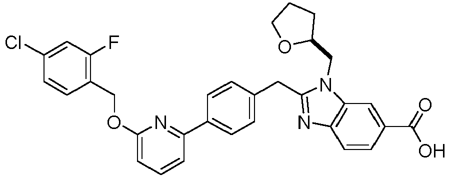

### (S)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid

The methyl (S)-4-amino-3-(((tetrahydrofuran-2-yl)methyl)amino)benzoate (150 mg, 0.6 mmol) obtained in Preparation Example 30 and the 2-(4-(6-((4-chloro-2-fluorobenzyl)oxy)pyridin-2-yl)phenyl)acetic acid (186 mg, 0.5 mmol) prepared in Preparation Example 2 were dissolved in 2 mL of DMF, and EDC (192 mg, 1.0 mmol) and HOBt (135 mg, 1.0 mmol) were added, followed by stirring for 24 hours at room temperature. After the reaction was completed, water was added, followed by extraction with EtOAc. The organic layer was dried with Na₂SO₄, and concentrated under reduced pressure, thereby obtaining an intermediate, methyl (S)-4-(2-(4-(6-((4-chloro-2-fluorobenzyl)oxy)pyridin-2-yl)phenyl)acetamido)-3-(((tetrahydrofuran-2-yl)methyl)amino)benzoate. The intermediate was dissolved in 10 mL of acetic acid without additional purification, followed by stirring for 2 hours at 120 °C. Water was added, followed by extraction with EtOAc. An organic layer was dried with Na₂SO₄, concentrated under reduced pressure and purified by using MPLC, thereby obtaining an intermediate, methyl (*S*)-2-(4-(6-((4-chloro-2-fluorobenzyl)oxy)pyridin-2-yl)benzyl)-1-((tetrahydrofuran-2-yl)methyl)-1*H-*benzo[d]imidazole-6-carboxylate. The intermediate was dissolved in 1.5 mL of MeOH, and 1.5 mL of IN NaOH was added to the reactants and then stirred for 24 hours at 50 °C. IN HCl was added, and the resulting solution was adjusted to pH 4, and extracted with EtOAc. An organic layer was dried with Na₂SO₄, concentrated under reduced pressure and purified by using MPLC, thereby obtaining (S)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid (60.4 mg, 0.105 mmol, 21%).

¹H NMR (500 MHz, CDCl₃) δ 8.17 (s, 1H), 8.10 - 8.05 (m, 1H), 7.95 (d, *J* = 8.5 Hz, 2H), 7.86 (d, *J =* 8.5 Hz, 1H), 7.62 (t, *J =* 7.9 Hz, 1H), 7.45 (t, *J =* 8.1 Hz, 1H), 7.35 (d, *J* = 8.0 Hz, 2H), 7.31 (d, *J* = 7.3 Hz, 1H), 7.15 - 7.08 (m, 2H), 6.72 (d, *J =* 8.5 Hz, 1H), 5.61 - 5.36 (m, 2H), 4.57 (dd, *J =* 48.5, 15.9 Hz, 2H), 4.22 (d, *J* = 12.5 Hz, 1H), 4.19 - 4.06 (m, 2H), 3.87 (dd, *J* = 15.1, 6.9 Hz, 1H), 3.73 (q, *J =* 7.4 Hz, 1H), 2.09 - 1.94 (m, 1H), 1.92 - 1.76 (m, 2H), 1.58 - 1.50 (m, 1H); LC-MS(ESI): 572.30 [M+H]⁺

### [Example 13: Preparation of (R)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid]

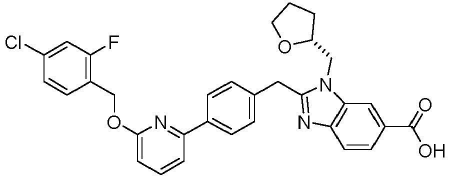

### (R)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid

The methyl (R)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylate (144 mg, 0.245 mmol) obtained in Preparation Example 33 was dissolved in 1.3 mL of MeOH and 1.3 mL of THF, and 1.3 mL of IN NaOH was added to the reactants, followed by stirring for 24 hours at 50 °C. IN HCl was added to adjust the resulting solution to pH 4, followed by extraction with EtOAc. An organic layer was dried with Na₂SO₄, concentrated under reduced pressure and purified by using MPLC, thereby obtaining (*R*)-2-(4-(6-((4-chloro-2-fluorobenzyl)oxy)pyridin-2-yl)benzyl)-1-((tetrahydrofuran-2-yl)methyl)-1H-benzo[d]imidazole-6-carboxylic acid (25.2 mg, 0.044 mmol, 18%).

¹H NMR (500 MHz, CDCl₃) δ 8.16 (s, 1H), 8.06 (d, *J =* 9.5 Hz, 1H), 7.96 (d, *J =* 8.2 Hz, 2H), 7.85 (d, *J =* 9.0 Hz, 1H), 7.62 (t, *J =* 7.8 Hz, 1H), 7.45 (t, *J =* 8.2 Hz, 1H), 7.36 (d, *J* = 8.0 Hz, 2H), 7.32 (d, *J* = 7.3 Hz, 1H), 7.11 (d, *J =* 9.5 Hz, 2H), 6.72 (d, *J* = 7.9 Hz, 1H), 5.61 - 5.41 (m, 2H), 4.70 - 4.47 (m, 2H), 4.30 - 4.06 (m, 3H), 3.88 (dd, *J =* 15.1, 6.9 Hz, 1H), 3.74 (q, *J =* 7.3 Hz, 1H), 2.08 - 1.97 (m, 1H), 1.96 - 1.81 (m, 2H), 1.62 - 1.48 (m, 1H); LC-MS(ESI): 572.31 [M+H]⁺

### [Example 14: Preparation of 2-(4-(6-((4-chloro-2-fluorobenzyl)oxy)pyridin-2-yl)benzyl)-1-((tetrahydro-2H-pyran-2-yl)methyl)-1H-benzo[d]imidazole-6-carboxylic acid]

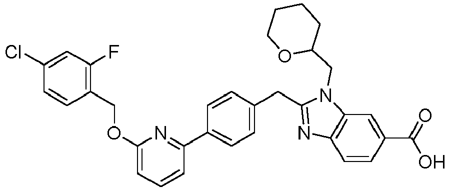

### 2-(4-(6-((4-chloro-2-fluorobenzyl)oxy)pyridin-2-yl)benzyl)-1-((tetrahydro-2H-pyran-2-yl)methyl)-1H-benzo[d]imidazole-6-carboxylic acid

The methyl 2-(4-(6-((4-chloro-2-fluorobenzyl)oxy)pyridin-2-yl)benzyl)-1-((tetrahydro-2H-pyran-2-yl)methyl)-1H-benzo[d]imidazole-6-carboxylate (156 mg, 0.26 mmol) obtained in Preparation Example 36 was dissolved in 1.3 mL of MeOH and 1.3 mL of THF, and 1.3 mL of IN NaOH was added to the reactants, followed by stirring for 24 hours at 50 °C. IN HCl was added to adjust the resulting solution to pH 4, followed by extraction with EtOAc. An organic layer was dried with Na₂SO₄, concentrated under reduced pressure and purified by using MPLC, thereby obtaining 2-(4-(6-((4-chloro-2-fluorobenzyl)oxy)pyridin-2-yl)benzyl)-1-((tetrahydro-2*H-*pyran-2-yl)methyl)-1H-benzo[d]imidazole-6-carboxylic acid (44.7 mg, 0.076 mmol, 29%).

¹H NMR (500 MHz, CDCl₃) δ 8.13 (s, 1H), 8.06 (d, *J* = 8.5 Hz, 1H), 7.96 (d, *J* = 8.2 Hz, 2H), 7.84 (d, *J =* 8.5 Hz, 1H), 7.63 (t, *J =* 7.8 Hz, 1H), 7.46 (t, *J =* 8.1 Hz, 1H), 7.36 (d, *J =* 8.2 Hz, 2H), 7.32 (d, *J* = 7.6 Hz, 1H), 7.12 (d, *J =* 9.2 Hz, 2H), 6.72 (d, *J* = 8.0 Hz, 1H), 5.61 - 5.41 (m, 2H), 4.53 (dd, *J* = 18.8, 16.0 Hz, 2H), 4.18 - 3.99 (m, 2H), 3.97 - 3.87 (m, 1H), 3.59 - 3.45 (m, 1H), 3.30 - 3.12 (m, 1H), 1.89-1.78 (m, 1H), 1.65 - 1.24 (m, 5H); LC-MS(ESI): 586.33 [M+H]⁺

### [Example 15: Preparation of (S)-2-(4-(6-((4-chloro-2-fluorobenzyl)oxy)pyridin-2-yl)-3-fluorobenzyl)-1-(oxetan-2-ylmethyl)-1H-benzo[d]imidazole-6-carboxylic acid]

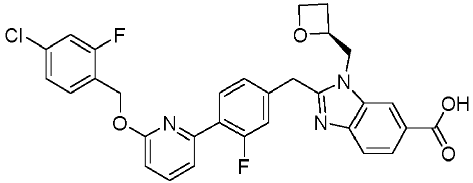

### (S)-2-(4-(6-((4-chloro-2-fluorobenzyl)oxy)pyridin-2-yl)-3-fluorobenzyl)-1-(oxetan-2-ylmethyl)-1H-benzo[d]imidazole-6-carboxylic acid

The methyl 2-(4-(6-((4-chloro-2-fluorobenzyl)oxy)pyridin-2-yl)-3-fluorophenyl)acetate (720 mg, 1.78 mmol) obtained in Preparation Example 38 was dissolved in 8 mL of THF/H₂O (1/1), and NaOH (214 mg, 5.35 mmol) was added. The reactants were stirred for 24 hours at room temperature. After the reaction was completed, IN HCl was added to adjust the resulting solution to pH ~2, followed by extraction with EtOAc. An organic layer was dried with Na₂SO₄, concentrated under reduced pressure and purified by using MPLC, thereby obtaining 2-(4-(6-((4-chloro-2-fluorobenzyl)oxy)pyridin-2-yl)-3-fluorophenyl)acetic acid (680 mg, 1.75 mmol, 98%).

¹H NMR (500 MHz, MeOD) δ 7.99 (t, *J =* 8.1 Hz, 1H), 7.76 (t, *J =* 7.8 Hz, 1H), 7.56 (t, *J* = 8.1 Hz, 1H), 7.48 (d, *J =* 7.6 Hz, 1H), 7.17-7.27 (m, 4H), 6.82 (d, *J* = 8.2 Hz, 1H), 5.52 (s, 2H), 3.70 (s, 2H).

The 2-(4-(6-((4-chloro-2-fluorobenzyl)oxy)pyridin-2-yl)-3-fluorophenyl)acetic acid (150 mg, 0.385 mmol) was dissolved in DMF (1.5 mL), and the methyl (S)-4-amino-3-((oxetan-2-ylmethyl)amino)benzoate (91 mg, 0.385 mmol) obtained in Preparation Example 7, EDC (148 mg, 0.770 mmol) and HOBt (118 mg, 0.770 mmol) were added. The reactants were stirred for 24 hours under nitrogen at room temperature. After the reaction was completed, water was added, the resulting solution was extracted with EtOAc, dried with MgSO₄ concentrated under reduced pressure, thereby obtaining an intermediate, methyl (*S*)-4-(2-(4-(6-((4-chloro-2-fluorobenzyl)oxy)pyridin-2-yl)-3-fluorophenyl)acetamido-3-((oxetan-2-ylmethyl)amino)benzoate (200 mg, 0.329 mmol). The intermediate was dissolved in AcOH (3.77 mL, 65.8 mmol) without additional purification, and then stirred for 3 hours at 120 °C. After the reaction, concentration was performed under reduced pressure. Water was added, and the resulting solution was extracted with EtOAc, dried with MgSO₄, and concentrated under reduced pressure, thereby obtaining an intermediate, methyl (*S*)-2-(4-(6-((4-chloro-2-fluorobenzyl)oxy)pyridin-2-yl)-3-fluorobenzyl)-1-(oxetan-2-ylmethyl)-1*H*-benzo[*d*]imidazole-6-carboxylate (150 mg, 0.254 mmol). The intermediate was dissolved in THF/H₂O (1 mL/1 mL) without additional purification, and NaOH (30 mg, 0.763 mmol) was added, followed by stirring for 24 hours at room temperature. After the reaction was completed, water was added, and IN HCl was added to acidify the resulting solution to pH ~2. Extraction was performed with EtOAc, and drying was performed with MgSO₄, followed by concentration under reduced pressure. The resulting concentrate was purified by using MPLC, thereby obtaining (S)-2-(4-(6-((4-chloro-2-fluorobenzyl)oxy)pyridin-2-yl)-3-fluorobenzyl)-1-(oxetan-2-ylmethyl)-1*H-*benzo[d]imidazole-6-carboxylic acid (70 mg, 0.122 mmol, 48%).

¹H NMR (500 MHz, MeOD) δ 8.30 (s, 1H), 8.01 (t, *J =* 7.9 Hz, 2H), 7.73 (dd, *J =* 20.8, 7.9 Hz, 2H), 7.54 (s, 1H), 7.47 (d, *J =* 7.6 Hz, 1H), 7.15-7.25 (m, 4H), 6.83 (d, *J* = 8.2 Hz, 1H), 5.51 (s, 2H), 5.19-5.04 (1H), 4.62-4.65 (m, 2H), 4.53-4.56 (m, 3H), 4.44 (d, *J=* 9.2 Hz, 1H), 2.81-2.69 (1H), 2.56-2.39 (1H).

### [Example 16: Preparation of (S)-2-(4-(6-((2-fluoro-4-(trifluoromethyl)benzyl)oxy)pyridin-2-yl)benzyl)-1-(oxetan-2-ylmethyl)-1H-benzo[d]imidazole-6-carboxylic acid]

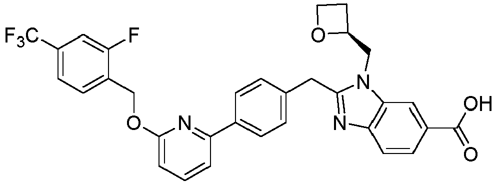

### (S)-2-(4-(6-((2-fluoro-4-(trifluoromethyl)benzyl)oxy)pyridin-2-yl)benzyl)-1-(oxetan-2-ylmethyl)-1H-benzo[d]imidazole-6-carboxylic acid

The methyl 2-(4-(6-((2-fluoro-4-(trifluoromethyl)benzyl)oxy)pyridin-2-yl)phenyl)acetate (1.71 g, 4.07 mmol) obtained in Preparation Example 41 was dissolved in 20 mL of THF/H₂O (1/1), and NaOH (488 mg, 12.2 mmol) was added. The reactants were stirred for 24 hours at room temperature. After the reaction was completed, IN HCl was added to adjust the resulting solution to pH ~2, followed by extraction with EtOAc. An organic layer was dried with Na₂SO₄, concentrated under reduced pressure, and purified by using MPLC, thereby obtaining 2-(4-(6-((2-fluoro-4-(trifluoromethyl)benzyl)oxy)pyridin-2-yl)phenyl)acetic acid (1.53 g, 3.77 mmol, 93%).

¹H NMR (400 MHz, CDCl₃) δ 7.96 (dd, *J* = 6.4, 1.8 Hz, 2H), 7.73-7.56 (m, 2H), 7.44-7.32 (m, 5H), 6.76 (d, *J=* 8.2 Hz, 1H), 5.62 (s, 2H), 3.71 (s, 2H).

The 2-(4-(6-((2-fluoro-4-(trifluoromethyl)benzyl)oxy)pyridin-2-yl)phenyl)acetic acid (150 mg, 0.370 mmol) was dissolved in DMF (2 mL), and the methyl (S)-4-amino-3-((oxetan-2-ylmethyl)amino)benzoate (87 mg, 0.370 mmol) obtained in Preparation Example 7, HATU (422 mg, 1.110 mmol) and TEA (0.155 mL, 1.110 mmol) were added. The reactants were stirred for 24 hours under nitrogen at room temperature. After the reaction was completed, water was added, and the resulting solution was extracted with EtOAc, dried with MgSO₄, and concentrated under reduced pressure, thereby obtaining an intermediate, methyl (S)-4-(2-(4-(6-((2-fluoro-4-(trifluoromethyl)benzyl)oxy)pyridin-2-yl)phenyl)acetamido)-3-((oxetan-2-ylmethyl)amino)benzoate (230 mg, 0.369 mmol). The intermediate was dissolved in AcOH (4.22 mL, 73.8 mmol) without additional purification, and stirred for 3 hours at 120 °C. After the reaction, concentration was performed under reduced pressure. Water was added, and the resulting solution was extracted with EtOAc, dried with MgSO₄, and concentrated under reduced pressure, thereby obtaining an intermediate, methyl (S)-2-(4-(6-((2-fluoro-4-(trifluoromethyl)benzyl)oxy)pyridin-2-yl)benzyl)-1-(oxetan-2-ylmethyl)-1*H-*benzo[*d*]imidazole-6-carboxylate (220 mg, 0.363 mmol). The intermediate was dissolved in THF/H₂O (1 mL/1 mL) without additional purification, and NaOH (44 mg, 1.090 mmol) was added, followed by stirring for 24 hours at room temperature. After the reaction was completed, water was added, and IN HCl was added to acidify the resulting solution to pH ~2. Extraction was performed with EtOAc, and drying was performed with MgSO₄, followed by concentration under reduced pressure. The resulting concentrate was purified by using MPLC, thereby obtaining (S)-2-(4-(6-((2-fluoro-4-(trifluoromethyl)benzyl)oxy)pyridin-2-yl)benzyl)-1-(oxetan-2-ylmethyl)-1H-benzo[d]imidazole-6-carboxylic acid (36 mg, 0.061 mmol, 17%).

¹H NMR (500 MHz, MeOD) δ 8.28 (s, 1H), 8.00 (q, *J =* 8.2 Hz, 3H), 7.74-7.78 (m, 2H), 7.69 (d, *J* = 8.5 Hz, 1H), 7.50 (t, *J=* 7.8 Hz, 3H), 7.39 (d, *J* = 8.2 Hz, 2H), 6.82 (d, *J=* 7.9 Hz, 1H), 5.64 (s, 2H), 4.54 (dd, *J=* 29.6, 16.2 Hz, 2H), 4.34 (dd, *J =* 14.6, 3.1 Hz, 1H), 4.20 (dd, *J =* 14.8, 9.3 Hz, 1H), 4.05-4.11 (m, 1H), 3.72-3.77 (m, 2H), 1.82 (d, *J=* 7.3 Hz, 1H), 1.73-1.76 (m, 1H).

### [Example 17: Preparation of (S)-2-(4-(6-((4-chloro-2-fluorobenzyl)oxy)pyridin-2-yl)-2-fluorophenoxy)-1-(oxetan-2-ylmethyl)-1H-benzo[d]imidazole-6-carboxylic acid]

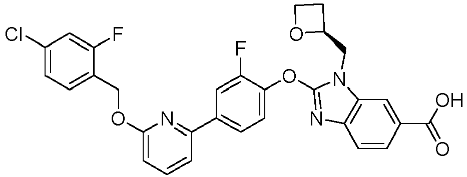

### (S)-2-(4-(6-((4-chloro-2-fluorobenzyl)oxy)pyridin-2-yl)-2-fluorophenoxy)-1-(oxetan-2-ylmethyl)-1H-benzo[d]imidazole-6-carboxylic acid

The 4-(6-((4-chloro-2-fluorobenzyl)oxy)pyridin-2-yl)-2-fluorophenol (76 mg, 0.219 mmol) obtained in Preparation Example 40 was dissolved in DMF (2 mL), and t-BuOK (45 mg, 0.397 mmol) was added, followed by stirring for 15 minutes under nitrogen at room temperature. The methyl (*S*)-2-benzylsulfonyl-1-(oxetan-2-ylmethyl)-1*H*-benzo[*d*]imidazole-6-carboxylate (80 mg, 0.199 mmol) obtained in Preparation Example 18 was added, and stirred for 3 hours at room temperature. After the reaction was completed, water was added, and the resulting solution was extracted with EtOAc, dried with MgSO₄, and concentrated under reduced pressure, thereby obtaining an intermediate, methyl (S)-2-(4-(6-((4-chloro-2-fluorobenzyl)oxy)pyridin-2-yl)-2-fluorophenoxy)-1-(oxetan-2-ylmethyl)-1*H-*benzo[d]imidazole-6-carboxylate (27 mg, 0.046 mmol). The intermediate was dissolved in THF/H₂O (1 mL/1 mL) without additional purification, and NaOH (5.5 mg, 1.137 mmol) was added, followed by stirring for 24 hours at room temperature. After the reaction was completed, water was added, and IN HCl was added to acidify the resulting solution to pH ~2. Extraction was performed by using EtOAc, and drying was performed with MgSO₄, followed by concentration under reduced pressure. The resulting concentrate was purified by using MPLC, thereby obtaining (*S*)-2-(4-(6-((4-chloro-2-fluorobenzyl)oxy)pyridin-2-yl)-2-fluorophenoxy)-1-(oxetan-2-ylmethyl)-1*H*-benzo[*d*]imidazole-6-carboxylic acid (10 mg, 0.017 mmol, 38%).

¹H NMR (400 MHz, CDCl₃) δ 8.20 (s, 1H), 7.90-7.98 (m, 2H), 7.85 *(d, J* = 9.1 Hz, 1H), 7.67 (t, *J =* 8.0 Hz, 1H), 7.56-7.60 (m, 2H), 7.46 (t, *J =* 8.0 Hz, 1H), 7.32-7.35 (m, 1H), 7.12-7.15 (m, 2H), 6.78 (d, *J=* 8.2 Hz, 1H), 5.49 (d, *J* = 26.1 Hz, 2H), 5.28 (d, *J* = 7.3 Hz, 1H), 4.67 (q, *J=* 7.2 Hz, 1H), 4.46-4.51 (m, 3H), 2.81 (d, *J* = 5.9 Hz, 1H), 2.65 (d, *J =* 11.9 Hz, 1H).

### [Example 18: Preparation of (S)-2-(2-chloro-4-(6-((4-chloro-2-fluorobenzyl)oxy)pyridin-2-yl)phenoxy)-1-(oxetan-2-ylmethyl)-1H-benzo[d] imidazol-6-carboxylic acid]

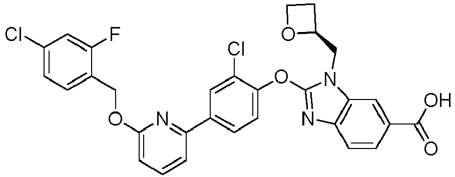

### (S)-2-(2-chloro-4-(6-((4-chloro-2-fluorobenzyl)oxy)pyridin-2-yl)phenoxy)-1-(oxetan-2-ylmethyl)-1H-benzo[d]imidazole-6-carboxylic acid

The 2-chloro-4-(6-((4-chloro-2-fluorobenzyl)oxy)pyridin-2-yl)phenol (77 mg, 0.211 mmol) obtained in Preparation Example 39 was dissolved in DMF (2 mL), and t-BuOK (32 mg, 0.288 mmol) was added, followed by stirring for 15 minutes under nitrogen at room temperature. The methyl (*S*)-2-benzylsulfonyl-1-(oxetan-2-ylmethyl)-1H-benzo[d]imidazole-6-carboxylate (77 mg, 0.192 mmol) obtained in Preparation Example 18 was added, followed by stirring for 3 hours at room temperature. After the reaction was completed, water was added, and the resulting solution was extracted with EtOAc, dried with MgSO₄ and concentrated under reduced pressure, thereby obtaining an intermediate, methyl (S)-2-(2-chloro-4-(6-((4-chloro-2-fluorobenzyl)oxy)pyridin-2-yl)phenoxy)-1-(oxetan-2-ylmethyl)-1*H-*benzo[d]imidazole-6-carboxylate (50 mg, 0.082 mmol). THF/H₂O (1 mL/1 mL) was dissolved without additional purification, and NaOH (10 mg, 0.247 mmol) was added, followed by stirring for 24 hours at room temperature. After the reaction, water was added, and IN HCl was added to acidify the resulting solution to pH ~2. Extraction was performed with EtOAc, and drying was performed with MgSO₄, followed by concentration under reduced pressure. The resulting concentrate was purified by using MPLC, thereby obtaining (S)-2-(2-chloro-4-(6-((4-chloro-2-fluorobenzyl)oxy)pyridin-2-yl)phenoxy)-1-(oxetan-2-ylmethyl)-1*H*-benzo[*d*] imidazol-6-carboxylic acid (23 mg, 0.039 mmol, 47%).

¹H NMR (500 MHz, CDCl₃) δ 8.28 (s, 1H), 8.17-8.19 (m, 1H), 7.98-8.03 (m, 2H), 7.60-7.71 (m, 3H), 7.44-7.51 (m, 1H), 7.34-7.40 (m, 1H), 7.16 (dd, *J=* 9.8, 7.0 Hz, 2H), 6.80 (dd, *J=* 14.6, 8.2 Hz, 1H), 5.48-5.60 (m, 2H), 5.34-5.39 (m, 1H), 4.66-4.75 (m, 1H), 4.48-4.61 (m, 3H), 2.84-2.92 (m, 1H), 2.63-2.78 (m, 1H).

### [Example 19: Preparation of (S)-2-(4-(6-((4-chloro-2-fluorobenzyl)oxy)pyridin-2-yl)-2,6-difluorophenoxy)-1-(oxetan-2-ylmethyl)-1H-benzo[d]imidazole-6-carboxylic acid]

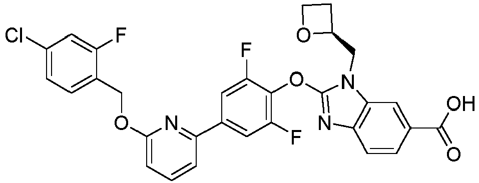

### (S)-2-(4-(6-((4-chloro-2-fluorobenzyl)oxy)pyridin-2-yl)-2,6-difluorophenoxy)-1-(oxetan-2-ylmethyl)-1H-benzo[d]imidazole-6-carboxylic acid

The 4-(6-((4-chloro-2-fluorobenzyl)oxy)pyridin-2-yl)-2,6-difluorophenol (200 mg, 0.547 mmol) obtained in Preparation Example 59 was dissolved in TEA (0.08 mL), and the methyl (*S*)-2-benzylsulfonyl-1-(oxetan-2-ylmethyl)-1*H-*benzo[d]imidazole-6-carboxylate (44 mg, 0.109 mmol) obtained in Preparation Example 18 was added, followed by stirring for 24 hours at 120 °C. After the reaction was completed, the resulting solution was concentrated under reduced pressure and purified by using MPLC, thereby obtaining an intermediate, methyl (S)-2-(4-(6-((4-chloro-2-fluorobenzyl)oxy)pyridin-2-yl)-2,6-difluorophenoxy)-1-(oxetan-2-ylmethyl)-1H-benzo[d]imidazole-6-carboxylate (22 mg, 0.036 mmol, 33%).

¹H NMR (500 MHz, MeOD) δ 8.30 (s, 1H), 7.93-7.96 (m, 3H), 7.83 (t, *J* = 7.8 Hz, 1H), 7.55-7.62 (m, 2H), 7.49 (d, *J* = 8.5 Hz, 1H), 7.23-7.28 (m, 2H), 6.91 (d, *J =* 8.2 Hz, 1H), 5.58 (s, 2H), 5.31-5.35 (m, 1H), 4.65-4.71 (m, 2H), 4.59 (dd, *J* = 15.4, 3.5 Hz, 1H), 4.40-4.44 (m, 1H), 3.94 (d, *J* = 6.1 Hz, 3H), 2.85-2.90 (m, 1H), 2.65-2.71 (m, 1H)

The intermediate, methyl (S)-2-(4-(6-((4-chloro-2-fluorobenzyl)oxy)pyridin-2-yl)-2,6-difluorophenoxy)-1-(oxetan-2-ylmethyl)-1*H*-benzo[*d*]imidazole-6-carboxylate (22 mg, 0.036 mmol), was dissolved in THF/H₂O (1 mL/1 mL), and NaOH (4 mg, 0.108 mmol) was added, followed by stirring for 24 hours at room temperature. After the reaction was completed, water was added, and IN HCl was added to acidify the resulting solution to pH ~2. Extraction was performed with EtOAc, and drying was performed with MgSO₄, followed by concentration under reduced pressure. The resulting concentrate was purified by using MPLC, thereby obtaining (S)-2-(4-(6-((4-chloro-2-fluorobenzyl)oxy)pyridin-2-yl)-2,6-difluorophenoxy)-1-(oxetan-2-ylmethyl)-1H-benzo[d]imidazole-6-carboxylic acid (8 mg, 0.013 mmol, 37%).

¹H NMR (500 MHz, MeOD) δ 8.28-8.30 (m, 1H), 7.88-8.01 (m, 3H), 7.77-7.85 (m, 1H), 7.54-7.64 (m, 2H), 7.44-7.53 (m, 1H), 7.18-7.30 (m, 2H), 6.88 (dd, *J* = 22.9, 8.2 Hz, 1H), 5.53-5.59 (m, 2H), 5.30-5.37 (m, 1H), 4.65-4.70 (m, 2H), 4.58 (dd, *J =* 15.3, 3.4 Hz, 1H), 4.41-4.48 (m, 1H), 2.84-2.91 (m, 1H), 2.63-2.70 (m, 1H).

### [Example 20: Preparation of (S)-2-(4-(6-((4-chloro-2-fluorobenzyl)oxy)pyridin-2-yl)-3-fluorophenoxy)-1-(oxetan-2-ylmethyl)-1H-benzo[d]imidazole-6-carboxylic acid]

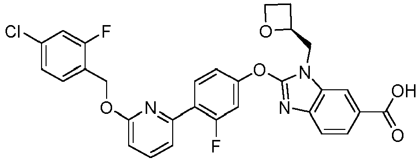

### (S)-2-(4-(6-((4-chloro-2-fluorobenzyl)oxy)pyridin-2-yl)-3-fluorophenoxy)-1-(oxetan-2-ylmethyl)-1H-benzo[d]imidazole-6-carboxylic acid

The 4-(6-((4-chloro-2-fluorobenzyl)oxy)pyridin-2-yl)-3-fluorophenol (150 mg, 0.431 mmol) obtained in Preparation Example 37 was dissolved in DMF (2 mL), and t-BuOK (73 mg, 0.647 mmol) was added, followed by stirring for 15 minutes under nitrogen at room temperature. The methyl (*S*)-2-benzylsulfonyl-1-(oxetan-2-ylmethyl)-1H-benzo[d]imidazole-6-carboxylate (173 mg, 0.431 mmol) obtained in Preparation Example 18 was added, followed by stirring for 3 hours at room temperature. After the reaction was completed, water was added, and resulting solution was extracted with EtOAc, dried with MgSO₄, and concentrated under reduced pressure, thereby obtaining an intermediate, methyl (*S*)-2-(4-(6-((4-chloro-2-fluorobenzyl)oxy)pyridin-2-yl)-3-fluorophenoxy)-1-(oxetan-2-ylmethyl)-1*H-*benzo[d]imidazole-6-carboxylate (125 mg, 0.211 mmol). The intermediate was dissolved in THF/H₂O (1 mL/1 mL) without additional purification, NaOH (25 mg, 0.633 mmol) was added, followed by stirring for 24 hours at room temperature. After the reaction, water was added, and IN HCl was added to acidify the resulting solution to pH ~2. Extraction was performed with EtOAc, and drying was performed with MgSO₄, followed by concentration under reduced pressure. The resulting concentrate was purified by using MPLC, thereby obtaining (S)-2-(4-(6-((4-chloro-2-fluorobenzyl)oxy)pyridin-2-yl)-3-fluorophenoxy)-1-(oxetan-2-ylmethyl)-1H-benzo[d]imidazole-6-carboxylic acid (64 mg, 0.111 mmol, 52%).

¹H NMR (500 MHz, MeOD) δ 8.25 (d, *J =* 9.2 Hz, 1H), 8.18 (t, *J=* 8.5 Hz, 1H), 7.96 (d, *J =* 8.5 Hz, 1H), 7.80 (t, *J* = 7.8 Hz, 1H), 7.48-7.59 (m, 3H), 7.37-7.41 (m, 2H), 7.23-7.27 (m, 2H), 6.87 (d, *J =* 8.2 Hz, 1H), 5.51-5.55 (m, 2H), 5.27-5.32 (m, 1H), 4.64-4.70 (m, 2H), 4.47-4.54 (m, 2H), 2.84-2.89 (m, 1H), 2.60-2.65 (m, 1H).

### [Example 21: Preparation of 2-(4-(6-((2-fluoro-4-(trifluoromethyl)benzyl)oxy)pyridin-2-yl)benzyl)-1-(oxazol-5-ylmethyl)-1H-benzo[d]imidazole-6-carboxylic acid]

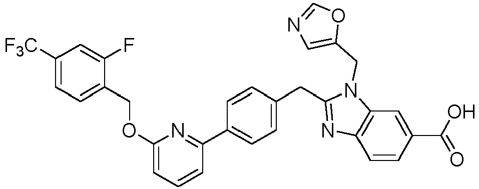

### 2-(4-(6-((2-fluoro-4-(trifluoromethyl)benzyl)oxy)pyridin-2-yl)benzyl)-1-(oxazol-5-ylmethyl)-1H-benzo[d]imidazole-6-carboxylic acid

2-(4-(6-((2-fluoro-4-(trifluoromethyl)benzyl)oxy)pyridin-2-yl)phenyl)acetic acid (170 mg, 0.419 mmol) was dissolved in DMF (2.5 mL), and the methyl 4-amino-3-((oxazol-5-ylmethyl)amino)benzoate (110 mg, 0.445 mmol) obtained in Preparation Example 23, HATU (478 mg, 1.258 mmol), and TEA (0.175 mL, 1.258 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(4-(6-((2-fluoro-4-(trifluoromethyl)benzyl)oxy)pyridin-2-yl)phenyl)acetamido)-3-((oxazol-5-ylmethyl)amino)benzoate (260 mg, 0.410 mmol). The intermediate was dissolved in AcOH (4.69 mL, 82.0 mmol) without additional purification, and then stirred for 3 hours at 120 °C. After the reaction, concentration was performed under reduced pressure. Water was added, and the resulting solution was extracted with EtOAc, dried with MgSO₄ and concentrated under reduced pressure, thereby obtaining an intermediate, methyl 2-(4-(6-((2-fluoro-4-(trifluoromethyl)benzyl)oxy)pyridin-2-yl)benzyl)-1-(oxazol-5-ylmethyl)-1H-benzo[d]imidazole-6-carboxylate (248 mg, 0.402 mmol). The intermediate was dissolved in THF/H₂O (1 mL/1 mL) without additional purification, and NaOH (48 mg, 1.207 mmol) was added, followed by stirring for 24 hours at room temperature. After the reaction was completed, water was added, and IN HCl was added to acidify the resulting solution to pH ~2. Extraction was performed by using EtOAc, and drying was performed with MgSO₄, followed by concentration under reduced pressure. The resulting concentrate was purified by using MPLC, thereby obtaining 2-(4-(6-((2-fluoro-4-(trifluoromethyl)benzyl)oxy)pyridin-2-yl)benzyl)-1-(oxazol-5-ylmethyl)-1*H-*benzo[d]imidazole-6-carboxylic acid (60 mg, 0.100 mmol, 25%).

¹H NMR (500 MHz, MeOD) δ 8.33 (s, 1H), 7.94-8.05 (m, 4H), 7.68-7.78 (m, 3H), 7.48-7.54 (m, 3H), 7.33-7.37 (m, 2H), 6.91-6.95 (m, 1H), 6.83 *(d, J* = 8.2 Hz, 1H), 5.59-5.70 (m, 4H), 4.56 (s, 2H).

### [Example 22: Preparation of 2-(3-chloro-4-(6-((4-chloro-2-fluorobenzyl)oxy)pyridin-2-yl)benzyl)-1-(oxazol-5-ylmethyl)-1H-benzo[d]imidazole-6-carboxylic acid]

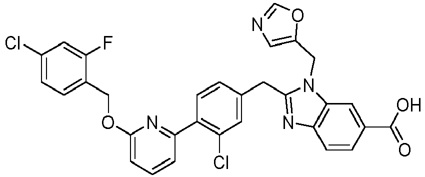

### 2-(3-chloro-4-(6-((4-chloro-2-fluorobenzyl)oxy)pyridin-2-yl)benzyl)-1-(oxazol-5-ylmethyl)-1H-benzo[d]imidazole-6-carboxylic acid

The 2-(3-chloro-4-(6-((4-chloro-2-fluorobenzyl)oxy)pyridin-2-yl)phenyl) acetic acid (110 mg, 0.271 mmol) obtained in Preparation Example 56 was dissolved in DMF (1.5 mL), and the methyl 4-amino-3-((oxazol-5-ylmethyl)amino)benzoate (67 mg, 0.271 mmol) obtained in Preparation Example 23, EDC (104 mg, 0.542 mmol) and HOBt (83 mg, 0.542 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(3-chloro-4-(6-((4-chloro-2-fluorobenzyl)oxy)pyridin-2-yl)phenyl)acetamido)-3-((oxazol-5-ylmethyl)amino)benzoate (147 mg, 0.231 mmol). The intermediate was dissolved in AcOH (2.65 mL, 46.3 mmol) without additional purification, and then stirred for 3 hours at 120 °C. After the reaction was completed, concentration was performed under reduced pressure. Water was added, and the resulting solution was extracted with EtOAc, dried with MgSO₄, and concentrated under reduced pressure, thereby obtaining an intermediate, methyl 2-(3-chloro-4-(6-((4-chloro-2-fluorobenzyl)oxy)pyridin-2-yl)benzyl)-1-(oxazol-5-ylmethyl)-1H-benzo[d]imidazole-6-carboxylate (112 mg, 0.181 mmol). The intermediate was dissolved in THF/H₂O (1 mL/1 mL) without additional purification, and NaOH (22 mg, 0.544 mmol) was added, followed by stirring for 24 hours at room temperature. After the reaction was completed, water was added, and the resulting solution was acidified with IN HCl to pH ~2. Extraction was performed by using EtOAc, and drying was performed with MgSO₄, followed by concentration under reduced pressure. The resulting concentrate was purified by using MPLC, thereby obtaining 2-(3-chloro-4-(6-((4-chloro-2-fluorobenzyl)oxy)pyridin-2-yl)benzyl)-1-(oxazol-5-ylmethyl)-1H-benzo[d]imidazole-6-carboxylic acid (20 mg, 0.033 mmol, 18%).

¹H NMR (500 MHz, MeOD) δ 8.39 (d, *J =* 40.9 Hz, 1H), 8.08-8.14 (m, 1H), 8.03 (dd, *J=* 8.5, 1.5 Hz, 1H), 7.73-7.83 (m, 2H), 7.51-7.58 (m, 2H), 7.36-7.42 (m, 1H), 7.20-7.31 (m, 4H), 6.99 (d, *J =* 32.3 Hz, 1H), 6.85 (d, *J =* 8.2 Hz, 1H), 5.64-5.77 (m, 2H), 5.48 (d, *J=* 24.4 Hz, 2H), 4.56 (d, *J=* 7.6 Hz, 2H).

### [Example 23: Preparation of 2-(4-(6-((4-chloro-2-fluorobenzyl)oxy)pyridin-2-yl)-3-fluorobenzyl)-1-(oxazol-5-ylmethyl)-1H-benzo[d]imidazole-6-carboxylic acid]

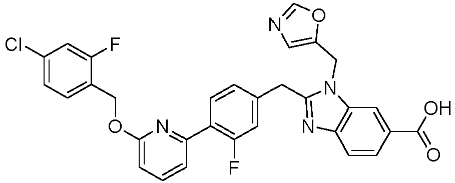

### 2-(4-(6-((4-chloro-2-fluorobenzyl)oxy)pyridin-2-yl)-3-fluorobenzyl)-1-(oxazol-5-ylmethyl)-1H-benzo[d]imidazole-6-carboxylic acid

2-(4-(6-((4-chloro-2-fluorobenzyl)oxy)pyridin-2-yl)-3-fluorophenyl)acetic acid (110 mg, 0.282 mmol) was dissolved in DMF (1.5 mL), and the methyl 4-amino-3-((oxazol-5-ylmethyl)amino)benzoate (70 mg, 0.282 mmol) obtained in Preparation Example 23, EDC (108 mg, 0.564 mmol) and HOBt (86 mg, 0.564 mmol) were added. The reactants were stirred for 24 hours under nitrogen at room temperature. After the reaction was completed, water was added, extraction was performed with EtOAc, and the resulting extract was dried with MgSO₄ and concentrated under reduced pressure, thereby obtaining an intermediate, methyl 4-(2-(4-(6-((4-chloro-2-fluorobenzyl)oxy)pyridin-2-yl)-3-fluorophenyl)acetamido)-3-((oxazol-5-ylmethyl)amino)benzoate (98 mg, 0.158 mmol). The intermediate was dissolved in AcOH (1.81 mL, 31.7 mmol) without additional purification, and stirred for 3 hours at 120 °C. After the reaction was completed, concentration was performed under reduced pressure. Water was added, extraction was performed with EtOAc, and the resulting extract was dried with MgSO₄ and concentrated under reduced pressure, thereby obtaining an intermediate, methyl 2-(4-(6-((4-chloro-2-fluorobenzyl)oxy)pyridin-2-yl)-3-fluorobenzyl)-1-(oxazol-5-ylmethyl)-1*H-*benzo[d]imidazole-6-carboxylate (93 mg, 0.155 mmol). The intermediate was dissolved in THF/H₂O (1 mL/1 mL) without additional purification, and NaOH (19 mg, 0.464 mmol) was added, followed by stirring for 24 hours at room temperature. After the reaction was completed, water was added, and the resulting solution was acidified to pH ~2 by using IN HCl. Extraction was performed with EtOAc, and drying was performed with MgSO₄, followed by concentration under reduced pressure. The resulting concentrate was purified by using MPLC, thereby obtaining 2-(4-(6-((4-chloro-2-fluorobenzyl)oxy)pyridin-2-yl)-3-fluorobenzyl)-1-(oxazol-5-ylmethyl)-1*H*-benzo[*d*]imidazole-6-carboxylic acid (34 mg, 0.058 mmol, 37%).

¹H NMR (400 MHz, MeOD) δ 8.29 (s, 1H), 7.93-8.03 (m, 3H), 7.70 (q, *J* = 7.6 Hz, 2H), 7.50 (t, *J =* 8.0 Hz, 1H), 7.42 (d, *J =* 5.9 Hz, 1H), 7.16-7.21 (m, 3H), 7.08 (d, *J=* 12.3 Hz, 1H), 6.98 (s, 1H), 6.78 (d, *J=* 8.2 Hz, 1H), 5.64 (s, 2H), 5.46 (s, 2H), 4.53 (s, 2H).

### [Example 24: Preparation of 2-(4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid]

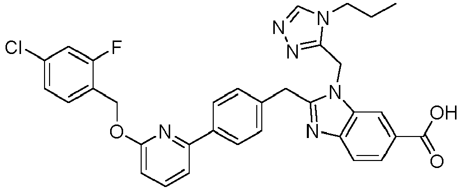

### 2-(4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid

The 2-(4-(6-((4-chloro-2-fluorobenzyl)oxy)pyridin-2-yl)phenyl)acetic acid (116 mg, 0.311 mmol) obtained in Preparation Example 2 was dissolved in DMF (1.5 mL), and the methyl 4-amino-3-(((4-propyl-4*H*-1,2,4-triazol-3-yl)methyl)amino)benzoate (90 mg, 0.311 mmol) obtained in Preparation Example 43, EDC (119 mg, 0.622 mmol) and HOBt (95 mg, 0.622 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(4-(6-((4-chloro-2-fluorobenzyl)oxy)pyridin-2-yl)phenyl)acetamido)-3-(((4-propyl-4*H*-1,2,4-triazol-3-yl)methyl)amino)benzoate (158 mg, 0.246 mmol). The intermediate was dissolved in AcOH (2.81 mL, 49.1 mmol) without additional purification, and stirred for 3 hours at 120 °C. After the reaction, concentration was performed under reduced pressure. Water was added, and the resulting solution was extracted with EtOAc, dried with MgSO₄ and concentrated under reduced pressure, thereby obtaining an intermediate, methyl 2-(4-(6-((4-chloro-2-fluorobenzyl)oxy)pyridin-2-yl)benzyl)-1-((4-propyl-4*H*-1,2,4-triazol-3-yl)methyl)-1H-benzo[d]imidazole-6-carboxylate (118 mg, 0.189 mmol). The intermediate was dissolved in THF/H₂O (1 mL/1 mL) without additional purification, and NaOH (38 mg, 0.944 mmol) was added, followed by stirring for 24 hours at room temperature. After the reaction was completed, water was added, and the resulting solution was acidified to pH ~2 using IN HCl. Extraction was performed using EtOAc, and drying was performed using MgSO₄, followed by concentration under reduced pressure. The resulting concentrate was purified by using MPLC, thereby obtaining 2-(4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid (33 mg, 0.054 mmol, 29%).

¹H NMR (500 MHz, MeOD) δ 8.38-8.41 (m, 1H), 8.19 (s, 1H), 8.03 (dd, *J* = 8.4, 1.4 Hz, 1H), 7.95 (d, *J=* 8.2 Hz, 2H), 7.72-7.76 (m, 2H), 7.55 (t, *J=* 8.1 Hz, 1H), 7.45 (d, *J=* 7.6 Hz, 1H), 7.31 (d, *J=* 8.2 Hz, 2H), 7.21-7.27 (m, 2H), 6.78 (d, *J* = 8.2 Hz, 1H), 5.76 (s, 2H), 5.48-5.60 (m, 2H), 4.50 (s, 2H), 3.83 (t, *J=* 7.5 Hz, 2H), 1.49-1.55 (m, 2H), 0.75 (t, *J=* 7.5 Hz, 3H).

### [Example 25: Preparation of 2-(4-(6-((2-fluoro-4-(trifluoromethyl)benzyl)oxy)pyridin-2-yl)benzyl)-1-((4-propyl-4H-1,2,4-triazol-3-yl)methyl)-1H-benzo[d]imidazole-6-carboxylic acid]

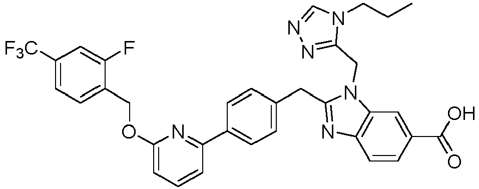

### 2-(4-(6-((2-fluoro-4-(trifluoromethyl)benzyl)oxy)pyridin-2-yl)benzyl)-1-((4-propyl-4H-1,2,4-triazol-3-yl)methyl)-1H-benzo[d]imidazole-6-carboxylic acid

2-(4-(6-((2-fluoro-4-(trifluoromethyl)benzyl)oxy)pyridin-2-yl)phenyl)acetic acid (126 mg, 0.311 mmol) was dissolved in DMF (1.5 mL), and the methyl 4-amino-3-(((4-propyl-4H-1,2,4-triazol-3-yl)methyl)amino)benzoate (90 mg, 0.311 mmol) obtained in Preparation Example 43, EDC (119 mg, 0.622 mmol) and HOBt (95 mg, 0.622 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(4-(6-((2-fluoro-4-(trifluoromethyl)benzyl)oxy)pyridin-2-yl)phenyl)acetamido)-3-(((4-propyl-4*H*-1,2,4-triazol-3-yl)methyl)amino)benzoate (135 mg, 0.200 mmol). The intermediate was dissolved in AcOH (2.28 mL, 39.9 mmol) without additional purification, and stirred for 3 hours at 120 °C. After the reaction was completed, concentration was performed under reduced pressure. Water was added, and the resulting solution was extracted with EtOAc, dried with MgSO₄ and concentrated under reduced pressure, thereby obtaining an intermediate, methyl 2-(4-(6-((2-fluoro-4-(trifluoromethyl)benzyl)oxy)pyridin-2-yl)benzyl)-1-((4-propyl-4*H*-1,2,4-triazol-3-yl)methyl)-1*H*-benzo[*d*]imidazole-6-carboxylate (126 mg, 0.191 mmol). The intermediate was dissolved in THF/H₂O (1 mL/1 mL) without additional purification, and NaOH (23 mg, 0.574 mmol) was added, followed by stirring for 24 hours at room temperature. After the reaction was completed, water was added, and the resulting solution was acidified to pH ~2 by using IN HCl. Extraction was performed using EtOAc, and drying was performed using MgSO₄, followed by concentration under reduced pressure. The resulting concentrate was purified by using MPLC, thereby obtaining 2-(4-(6-((2-fluoro-4-(trifluoromethyl)benzyl)oxy)pyridin-2-yl)benzyl)-1-((4-propyl-4*H*-1,2,4-triazol-3-yl)methyl)-1H-benzo[d]imidazole-6-carboxylic acid (60 mg, 0.093 mmol, 49%).

¹H NMR (500 MHz, MeOD) δ 8.39-8.42 (m, 1H), 8.19 (s, 1H), 8.02-8.04 (m, 1H), 7.94 (d, *J=* 8.2 Hz, 2H), 7.74-7.78 (m, 3H), 7.50-7.53 (m, 2H), 7.47 (d, *J=* 7.3 Hz, 1H), 7.31 *(d, J* = 8.2 Hz, 2H), 6.83 (d, *J =* 8.2 Hz, 1H), 5.76 (s, 2H), 5.60-5.70 (m, 2H), 4.49 (s, 2H), 3.83 (t, *J=* 7.5 Hz, 2H), 1.49-1.54 (m, 2H), 0.74 (t, *J=* 7.5 Hz, 3H).

### [Example 26: Preparation of 2-(3-chloro-4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxlic acid]

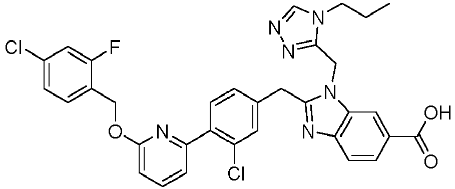

### 2-(3-chloro-4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid

The 2-(3-chloro-4-(6-((4-chloro-2-fluorobenzyl)oxy)pyridin-2-yl)phenyl) acetic acid (110 mg, 0.271 mmol) obtained in Preparation Example 56 was dissolved in DMF (1.5 mL), and the methyl 4-amino-3-(((4-propyl-4H-1,2,4-triazol-3-yl)methyl)amino)benzoate (78 mg, 0.271 mmol) obtained in Preparation Example 43, EDC (104 mg, 0.542 mmol) and HOBt (83 mg, 0.542 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(3-chloro-4-(6-((4-chloro-2-fluorobenzyl)oxy)pyridin-2-yl)phenyl)acetamido)-3-(((4-propyl-4*H*-1,2,4-triazol-3-yl)methyl)amino)benzoate (178 mg, 0.263 mmol). The intermediate was dissolved in AcOH (3.00 mL, 52.5 mmol) without additional purification, and stirred for 3 hours at 120 °C. After the reaction, concentration was performed under reduced pressure. Water was added, and the resulting solution was extracted with EtOAc, dried with MgSO₄ and concentrated under reduced pressure, thereby obtaining an intermediate, methyl 2-(3-chloro-4-(6-((4-chloro-2-fluorobenzyl)oxy)pyridin-2-yl)benzyl)-1-((4-propyl-4*H*-1,2,4-triazol-3-yl)methyl)-1*H*-benzo[*d*]imidazole-6-carboxylate (158 mg, 0.240 mmol). The intermediate was dissolved in THF/H₂O (1 mL/1 mL) without additional purification, and NaOH (29 mg, 0.719 mmol) was added, followed by stirring for 24 hours at room temperature. After the reaction was completed, water was added, and the resulting solution was acidified to pH ~2 by using IN HCl. Extraction was performed using EtOAc, and drying was performed using MgSO₄, followed by concentration under reduced pressure. The resulting concentrate was purified by using MPLC, thereby obtaining 2-(3-chloro-4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid (63 mg, 0.098 mmol, 41%).

¹H NMR (500 MHz, MeOD) δ 8.45 (s, 1H), 8.23 (s, 1H), 8.04 (d, *J =* 8.5 Hz, 1H), 7.75-7.78 (m, 2H), 7.53 (t, *J =* 8.1 Hz, 1H), 7.46 (d, *J =* 7.9 Hz, 1H), 7.38 (s, 1H), 7.21-7.25 (m, 4H), 6.85 (d, *J=* 8.2 Hz, 1H), 5.83 (s, 2H), 5.46 (s, 2H), 4.50 (s, 2H), 3.92 (t, *J =* 7.5 Hz, 2H), 1.58 (q, *J =* 7.3 Hz, 2H), 0.81 (t, *J =* 7.3 Hz, 3H).

### [Example 27: Preparation of 2-(4-(6-((4-chloro-2-fluorobenzyl)oxy)pyridin-2-yl)-3-fluorobenzyl)-1-((4-propyl-4H-1,2,4-triazol-3-yl)methyl)-1H-benzo[d]imidazole-6-carboxylic acid]

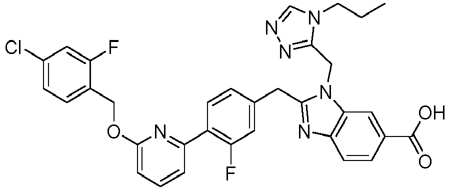

### 2-(4-(6-((4-chloro-2-fluorobenzyl)oxy)pyridin-2-yl)-3-fluorobenzyl)-1-((4-propyl-4H-1,2,4-triazol-3-yl)methyl)-1H-benzo[d]imidazole-6-carboxylic acid

2-(4-(6-((4-chloro-2-fluorobenzyl)oxy)pyridin-2-yl)-3-fluorophenyl)acetic acid (110 mg, 0.282 mmol) was dissolved in DMF (1.5 mL), and the methyl 4-amino-3-(((4-propyl-4H-1,2,4-triazol-3-yl)methyl)amino)benzoate (82 mg, 0.282 mmol) obtained in Preparation Example 43, EDC (108 mg, 0.564 mmol) and HOBt (86 mg, 0.564 mmol) were added. The reactants were stirred for 24 hours under nitrogen at room temperature. After the reaction was completed, water was added, and the resulting solution was extracted with EtOAc, dried with MgSO₄ and concentrated under reduced pressure, thereby obtaining an intermediate, methyl 4-(2-(4-(6-((4-chloro-2-fluorobenzyl)oxy)pyridin-2-yl)-3-fluorophenyl)acetamido)-3-(((4-propyl-4H-1,2,4-triazol-3-yl)methyl)amino)benzoate (180 mg, 0.272 mmol). The intermediate was dissolved in AcOH (3.10 mL, 54.5 mmol) without additional purification, and stirred for 3 hours at 120 °C. After the reaction, concentration was performed under reduced pressure. Water was added, and the resulting solution was extracted with EtOAc, dried with MgSO₄ and concentrated under reduced pressure, thereby obtaining an intermediate, methyl 2-(4-(6-((4-chloro-2-fluorobenzyl)oxy)pyridin-2-yl)-3-fluorobenzyl)-1-((4-propyl-4*H*-1,2,4-triazol-3-yl)methyl)-1H-benzo[d]imidazole-6-carboxylate (170 mg, 0.264 mmol). The intermediate was dissolved in THF/H₂O (1 mL/1 mL) without additional purification, and NaOH (32 mg, 0.793 mmol) was added, followed by stirring for 24 hours at room temperature. After the reaction was completed, water was added, and the resulting solution was acidified to pH ~2 using IN HCl. Extraction was performed using EtOAc, and drying was performed using MgSO₄, followed by concentration under reduced pressure. The resulting concentrate was purified by using MPLC, thereby obtaining 2-(4-(6-((4-chloro-2-fluorobenzyl)oxy)pyridin-2-yl)-3-fluorobenzyl)-1-((4-propyl-4H-1,2,4-triazol-3-yl)methyl)-1*H*-benzo[*d*]imidazole-6-carboxylic acid (35 mg, 0.056 mmol, 21%).

¹H NMR (400 MHz, MeOD) δ 8.40 (s, 1H), 8.19 (d, *J =* 10.1 Hz, 1H), 7.98-8.01 (m, 1H), 7.89 (t, *J =* 8.2 Hz, 1H), 7.69-7.73 (m, 2H), 7.50 (t, *J =* 8.0 Hz, 1H), 7.40 (d, *J=* 5.9 Hz, 1H), 7.17-7.22 (m, 2H), 7.03-7.12 (m, 2H), 6.78 (d, *J=* 7.8 Hz, 1H), 5.77 (s, 2H), 5.46 (s, 2H), 4.46 (s, 2H), 3.87 (t, *J=* 7.5 Hz, 2H), 1.52 (q, *J=* 7.5 Hz, 2H), 0.75 (t, *J =* 7.3 Hz, 3H).

### [Example 28: Preparation of 2-(4-(6-((4-chloro-2-fluorobenzyl)oxy)pyridin-2-yl)phenoxy)-1-((tetrahydrofuran-2-yl)methyl)-1H-benzo[d]imidazole-6-carboxylic acid]

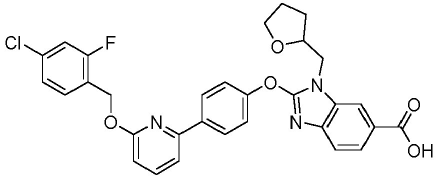

### 2-(4-(6-((4-chloro-2-fluorobenzyl)oxy)pyridin-2-yl)phenoxy)-1-((tetrahydrofuran-2-yl)methyl)-1H-benzo[d]imidazole-6-carboxylic acid

The methyl 2-(4-(6-((4-chloro-2-fluorobenzyl)oxy)pyridin-2-yl)phenoxy)-1-((tetrahydrofuran-2-yl)methyl)-1H-benzo[d]imidazole-6-carboxylate (43.8 mg, 0.074 mmol) obtained in Preparation Example 47 was dissolved in MeOH/THF (5 mL/5 mL), and 0.5 mL of IN NaOH was added to the reactants and stirred for 14 hours at 50 °C. After the reaction was completed, water was added, and the resulting solution was acidified to pH 4 by using IN HCl, extracted with EtOAc, dried with Na₂SO₄, and concentrated under reduced pressure. The resulting concentrate was purified by using MPLC, thereby obtaining 2-(4-(6-((4-chloro-2-fluorobenzyl)oxy)pyridin-2-yl)phenoxy)-1-((tetrahydrofuran-2-yl)methyl)-1*H-*benzo[d]imidazole-6-carboxylic acid (2 mg, 0.0034 mmol, 4.4%).

¹H NMR (500 MHz, CDCl₃) δ 8.17 (s, 1H), 8.10 (d, *J =* 8.8 Hz, 2H), 7.99 (d, *J =* 8.2 Hz, 1H), 7.67 (t, *J =* 7.8 Hz, 1H), 7.60 (d, *J =* 8.5 Hz, 1H), 7.46 - 7.50 (m, 3H), 7.36 (d, *J* = 7.3 Hz, 1H), 7.12 - 7.15 (m, 2H), 6.76 (d, *J =* 7.9 Hz, 1H), 5.54 (s, 2H), 4.41 (q, *J* = 6.3 Hz, 1H), 4.30 (d, *J =* 5.5 Hz, 2H), 3.90 (dd, *J =* 15.0, 6.7 Hz, 1H), 3.80 (dd, *J=* 15.1, 6.9 Hz, 1H), 2.10 - 2.15 (m, 1H), 1.91 - 1.97 (m, 2H), 1.75 - 1.83 (m, 1H); LC-MS(ESI): 574.26 [M+H]⁺

### [Example 29: Preparation of (S)-2-(4-(6-((2-fluoro-4-(trifluoromethyl)benzyl)oxy)pyridin-2-yl)benzyl)-1-((tetrahydrofuran-2-yl)methyl)-1H-benzo[d]imidazole-6-carboxylic acid]

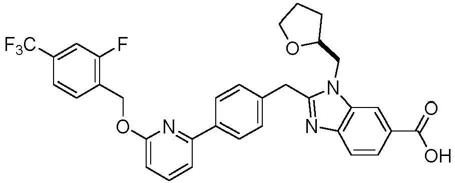

### (S)-2-(4-(6-((2-fluoro-4-(trifluoromethyl)benzyl)oxy)pyridin-2-yl)benzyl)-1-((tetrahydrofuran-2-yl)methyl)-1H-benzo[d]imidazole-6-carboxylic acid

The (S)-4-amino-3-(((tetrahydrofuran-2-yl)methyl)amino)benzoate (138 mg, 0.55 mmol) obtained in Preparation Example 30 and 2-(4-(6-((2-fluoro-4-(trifluoromethyl)benzyl)oxy) pyridin-2-yl)phenyl)acetic acid (203 mg, 0.5 mmol) were dissolved in 2 mL of DMF, and EDC (192 mg, 1.0 mmol) and HOBt (153 mg, 1.0 mmol) were added, followed by stirring for 16 hours at room temperature. After the reaction was completed, water added, followed by extraction with EtOAc. An organic layer was dried with Na₂SO₄, concentrated under reduced pressure and purified by using MPLC, thereby obtaining an intermediate, methyl (S)-4-(2-(4-(6-((2-fluoro-4-(trifluoromethyl)benzyl)oxy)pyridin-2-yl)phenyl)acetamido)-3-(((tetrahydrofuran-2-yl)methyl)amino)benzoate. The intermediate was dissolved in 10 mL of acetic acid without additional purification and stirred for 2 hours at 120 °C. The acetic acid was concentrated under reduced pressure, and MPLC was performed, thereby obtaining an intermediate, methyl (*S*)-2-(4-(6-((2-fluoro-4-(trifluoromethyl)benzyl)oxy)pyridin-2-yl)benzyl)-1-((tetrahydrofuran-2-yl)methyl)-1H-benzo[d]imidazole-6-carboxylate. The intermediate was dissolved in 10 mL of THF and 2 mL of MeOH, 2.5 mL of IN NaOH was added to the reactants, and stirred for 24 hours at 40 °C. IN HCl was added to adjust the resulting solution to pH 4, followed by extraction with EtOAc. An organic layer was dried with Na₂SO₄, concentrated under reduced pressure and purified by using MPLC, thereby obtaining (*S*)-2-(4-(6-((2-fluoro-4-(trifluoromethyl)benzyl)oxy)pyridin-2-yl)benzyl)-1-((tetrahydrofuran-2-yl)methyl)-1H-benzo[d]imidazole-6-carboxylic acid (138 mg, 0.228 mmol, 45.5%).

¹H NMR (500 MHz, CDCl₃) δ 8.16 (s, 1H), 8.06 (d, *J* = 8.5 Hz, 1H), 7.94 (d, *J =* 8.2 Hz, 2H), 7.84 (d, *J =* 8.5 Hz, 1H), 7.63 - 7.68 (m, 2H), 7.40 (d, *J =* 7.9 Hz, 1H), 7.32 - 7.37 (m, 4H), 6.76 (d, *J =* 7.9 Hz, 1H), 5.60 (s, 2H), 4.55 (dd , *J* = 43.3, 15.9 Hz, 2H), 4.22 (d, *J* = 12.5 Hz, 1H), 4.09 - 4.18 (m, 2H), 3.87 (q, *J* = 7.3 Hz, 1H), 3.74 (q, *J =* 7.3 Hz, 1H), 2.03 (td, *J =* 12.7, 6.2 Hz, 1H), 1.83 - 1.90 (m, 2H), 1.51 - 1.58 (m, 1H); LC-MS(ESI): 606.35 [M+H]⁺

### [Example 30: (S)-2-(4-(6-((4-chloro-2-fluorobenzyl)oxy)pyridin-2-yl)-2-fluorobenzyl)-1-((tetrahydrofuran-2-yl)methyl)-1H-benzo[d]imidazole-6-carboxylic acid]

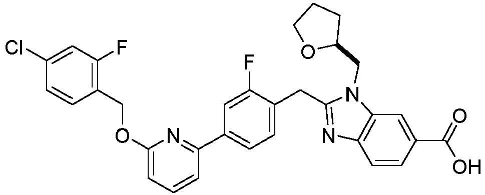

### (S)-2-(4-(6-((4-chloro-2-fluorobenzyl)oxy)pyridin-2-yl)-2-fluorobenzyl)-1-((tetrahydrofuran-2-yl)methyl)-1H-benzo[d]imidazole-6-carboxylic acid

The (S)-4-amino-3-(((tetrahydrofuran-2-yl)methyl)amino)benzoate (138 mg, 0.55 mmol) obtained in Preparation Example 30 and the 2-(4-(6-((4-chloro-2-fluorobenzyl)oxy)pyridin-2-yl)-2-fluorophenyl)acetic acid (195 mg, 0.5 mmol) obtained in Preparation Example 28 were dissolved in 2 mL of DMF, and EDC (192 mg, 1.0 mmol) and HOBt (153 mg, 1.0 mmol) were added, followed by stirring for 16 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl(*S*)-4-(2-(4-(6-((4-chloro-2-fluorobenzyl)oxy)pyridin-2-yl)-2-fluorophenyl)acetamido)-3-(((tetrahydrofuran-2-yl)methyl)amino)benzoate. The intermediate was dissolved in 10 mL of acetic acid without additional purification, and stirred for 2 hours at 120 °C. The acetic acid was concentrated under reduced pressure, and MPLC was performed, thereby obtaining an intermediate, methyl (*S*)-2-(4-(6-((4-chloro-2-fluorobenzyl)oxy)pyridin-2-yl)-2-fluorobenzyl)-1-((tetrahydrofuran-2-yl)methyl)-1H-benzo[d]imidazole-6-carboxylate. The intermediate was dissolved in 10 mL of THF and 2 mL of MeOH, and 2.5 mL of IN NaOH was added to the reactant and stirred for 24 hours at 40 °C. IN HCl was added to adjust the resulting solution to pH 4, followed by extraction with EtOAc. An organic layer was dried with Na₂SO₄, concentrated under reduced pressure and purified by using MPLC, thereby obtaining (S)-2-(4-(6-((4-chloro-2-fluorobenzyl)oxy)pyridin-2-yl)-2-fluorobenzyl)-1-((tetrahydrofuran-2-yl)methyl)-1H-benzo[d]imidazole-6-carboxylic acid (9.3 mg, 0.015 mmol, 3%).

¹H NMR (500 MHz, CDCl₃) δ 8.19 (s, 1H), 8.05 (d, *J =* 8.5 Hz, 1H), 7.78 - 7.83 (m, 2H), 7.69 (d, *J=* 7.0 Hz, 1H), 7.63 (t, *J=* 7.9 Hz, 1H), 7.42 - 7.47 (m, 1H), 7.36 (t, *J=* 7.6 Hz, 1H), 7.28 - 7.30 (m, 1H), 7.11 - 7.16 (m, 2H), 6.74 - 6.76 (m, 1H), 5.50 (s, 2H), 4.53 (dd, *J =* 39.7, 16.2 Hz, 2H), 4.30 *(d, J* = 12.2 Hz, 1H), 4.16 - 4.20 (m, 2H), 3.88 (q, *J =* 7.3 Hz, 1H), 3.74 (q, *J* = 7.2 Hz, 1H), 2.05 - 2.09 (m, 1H), 1.86 - 1.92 (m, 2H), 1.55 - 1.57 - 1.63 (m, 1H); LC-MS(ESI): 590.31 [M+H]⁺

### [Example 31: Preparation of (S)-2-(4-(6-((4-chloro-2-fluorobenzyl)oxy)pyridin-2-yl)-2-fluorophenoxy)-1-((tetrahydrofuran-2-yl)methyl)-1H-benzo[d]imidazole-6-carboxylic acid]

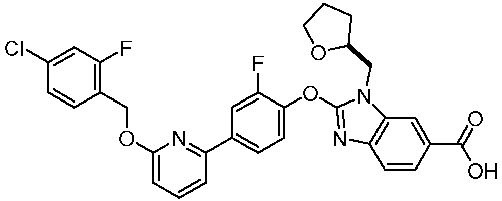

### (S)-2-(4-(6-((4-chloro-2-fluorobenzyl)oxy)pyridin-2-yl)-2-fluorophenoxy)-1-((tetrahydrofuran-2-yl)methyl)-1H-benzo[d]imidazole-6-carboxylic acid

The 4-(6-((4-chloro-2-fluorobenzyl)oxy)pyridin-2-yl)-2-fluorophenol (69.5 mg, 0.2 mmol) obtained in Preparation Example 40 was dissolved in DMF (3 mL), and t-BuOK (33.7 mg, 0.3 mmol) was added, followed by stirring for 10 minutes under nitrogen at room temperature. The methyl (*S*)-2-(benzylsulfonyl)-1-((tetrahydrofuran-2-yl)methyl)-1H-benzo[d]imidazole-6-carboxylate (82.9 mg, 0.20 mmol) obtained in Preparation Example 50 was added, followed by stirring for 12 hours at room temperature. After the reaction was completed, water was added, the resulting solution was extracted with EtOAc, dried with Na₂SO₄, concentrated under reduced pressure, and purified by using MPLC, thereby obtaining an intermediate, methyl (*S*)-2-(4-(6-((4-chloro-2-fluorobenzyl)oxy)pyridin-2-yl)-2-fluorophenoxy)-1-((tetrahydrofuran-2-yl)methyl)-1H-benzo[d]imidazole-6-carboxylate. The intermediate was dissolved in MeOH/THF (2 mL/2 mL) without additional purification, and 0.26 mL of IN NaOH was added to the reactants, followed by stirring for 15 hours at 40 °C. After the reaction was completed, water was added, and 1N-HCl was added to adjust the resulting solution to pH 4, extraction was performed with EtOAc, drying was performed with Na₂SO₄, followed by concentration under reduced pressure. The resulting concentrate was purified by using MPLC, thereby obtaining (*S*)-2-(4-(6-((4-chloro-2-fluorobenzyl)oxy)pyridin-2-yl)-2-fluorophenoxy)-1-((tetrahydrofuran-2-yl)methyl)-1*H*-benzo[*d*]imidazole-6-carboxylic acid (10.7 mg, 0.018 mmol, 9%).

¹H NMR (500 MHz, CDCl₃) δ 8.20 (s, 1H), 7.99 (d, *J=* 8.2 Hz, 1H), 7.94 (dd, *J =* 11.6, 1.8 Hz, 1H), 7.85 (d, *J =* 8.5 Hz, 1H), 7.68 (t, *J =* 7.8 Hz, 1H), 7.58 - 7.62 (m, 2H), 7.48 (t, *J=* 8.1 Hz, 1H), 7.33 - 7.36 (m, 1H), 7.13 - 7.15 (m, 2H), 6.79 (d, *J* = 8.2 Hz, 1H), 5.53 (s, 2H), 4.41 - 4.46 (m, 1H), 4.27 - 4.36 (m, 2H), 3.92 (q, *J=* 7.3 Hz, 1H), 3.79 - 3.83 (m, 1H), 2.14 (dt, *J =* 19.4, 6.7 Hz, 1H), 1.92 - 1.99 (m, 2H), 1.78 - 1.86 (m, 1H); LC-MS(ESI): 592.26 [M+H]⁺

### [Example 32: Preparation of (S)-2-(2-chloro-4-(6-((4-chloro-2-fluorobenzyl)oxy)pyridin-2-yl)phenoxy)-1-((tetrahydrofuran-2-yl)methyl)-1H-benzo[d]imidazole-6-carboxylic acid]

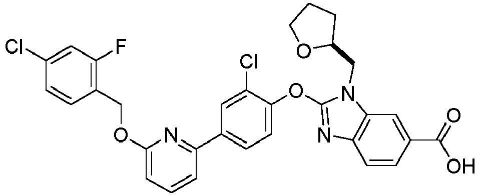

### (S)-2-(2-chloro-4-(6-((4-chloro-2-fluorobenzyl)oxy)pyridin-2-yl)phenoxy)-1-((tetrahydrofuran-2-yl)methyl)-1H-benzo[d]imidazole-6-carboxylic acid

The 2-chloro-4-(6-((4-chloro-2-fluorobenzyl)oxy)pyridin-2-yl)phenol (72.8 mg, 0.2 mmol) obtained in Preparation Example 39 was dissolved in DMF (3 mL), and t-BuOK (33.7 mg, 0.3 mmol) was added, followed by stirring for 10 minutes under nitrogen at room temperature. The methyl (*S*)-2-(benzylsulfonyl)-1-((tetrahydrofuran-2-yl)methyl)-1H-benzo[d]imidazole-6-carboxylate (82.9 mg, 0.20 mmol) obtained in Preparation Example 50 was added, followed by stirring for 12 hours at room temperature. After the reaction was completed, water was added, and the resulting solution was extracted with EtOAc, dried with Na₂SO₄, and concentrated under reduced pressure. The resulting concentrate was purified by using MPLC, thereby obtaining an intermediate, methyl (S)-2-(2-chloro-4-(6-((4-chloro-2-fluorobenzyl)oxy)pyridin-2-yl)phenoxy)-1-((tetrahydrofuran-2-yl)methyl)-1H-benzo[d]imidazole-6-carboxylate. The intermediate was dissolved in MeOH/THF (2 mL/2 mL) without additional purification, and 0.22 mL of IN NaOH was added to the reactants, followed by stirring for 15 hours at 40 °C. After the reaction was completed, water was added, 1N-HCl was added to adjust the resulting solution to pH 4, extraction was performed with EtOAc, and drying was performed with Na₂SO₄, followed by concentration under reduced pressure. The resulting concentrate was purified by using MPLC, thereby obtaining (*S*)-2-(2-chloro-4-(6-((4-chloro-2-fluorobenzyl)oxy)pyridin-2-yl)phenoxy)-1-((tetrahydrofuran-2-yl)methyl)-1H-benzo[d]imidazole-6-carboxylic acid (10 mg, 0.016 mmol, 8%).

¹H NMR (500 MHz, CDCl₃) δ 8.20 (d, *J=* 1.2 Hz, 1H), 8.16 (d, *J=* 2.1 Hz, 1H), 7.95 - 8.01 (m, 2H), 7.66 (t, *J =* 8.5 Hz, 2H), 7.59 (d, *J =* 8.2 Hz, 1H), 7.46 - 7.49 (m, 1H), 7.34 (d, *J* = 7.3 Hz, 1H), 7.13 - 7.16 (m, 2H), 6.79 (d, *J =* 8.2 Hz, 1H), 5.53 (s, 2H), 4.44 - 4.49 (m, 1H), 4.34 (t, *J =* 4.0 Hz, 2H), 3.93 (dd, *J=* 15.0, 6.7 Hz, 1H), 3.81 (dd, *J=* 15.1, 6.9 Hz, 1H), 2.12 - 2.18 (m, 1H), 1.93 - 1.98 (m, 2H), 1.80 - 1.87 (m, 1H) ; LC-MS(ESI): 608.24 [M+H]⁺

### [Example 33: Preparation of (R)-2-(4-(6-((4-chloro-2-fluorobenzyl)oxy)ypridin-2-yl)benzyl)-1-((tetrahydrofuran-3-yl)methyl)-1H-benzo[d]imidazole-6-carboxylic acid]

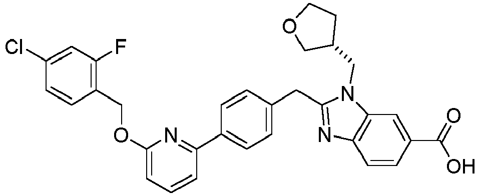

### (R)-2-(4-(6-((4-chloro-2-fluorobenzyl)oxy)pyridin-2-yl)benzyl)-1-((tetrahydrofuran-3-yl)methyl)-1H-benzo[d]imidazole-6-carboxylic acid

The methyl (R)-4-amino-3-(((tetrahydrofuran-3-yl)methyl)amino)benzoate (202 mg, 0.807 mmol) obtained in Preparation Example 52 and the 2-(4-(6-((4-chloro-2-fluorobenzyl)oxy)pyridin-2-yl)phenyl)acetic acid (300 mg, 0.807 mmol) obtained in Preparation Example 2 were dissolved in 5 mL of DMF, and EDC (309 mg, 1.614 mmol) and HOBt (247 mg, 1.614 mmol) were added, followed by stirring for 4 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl(R)-4-(2-(4-(6-((4-chloro-2-fluorobenzyl)oxy)pyridin-2-yl)phenyl)acetamido)-3-(((tetrahydrofuran-3-yl)methyl)amino)benzoate. The intermediate was dissolved in 5 mL of acetic acid without additional purification and stirred for 12 hours at 120 °C. The acetic acid was concentrated under reduced pressure, and MPLC was performed, thereby obtaining an intermediate, methyl (*R*)-2-(4-(6-((4-chloro-2-fluorobenzyl)oxy)pyridin-2-yl)benzyl)-1-((tetrahydrofuran-3-yl)methyl)-1*H*-benzo[*d*]imidazole-6-carboxylate. The intermediate was dissolved in 10 mL of THF, 0.9 mL of IN NaOH was added to the reactants, followed by stirring for 24 hours at room temperature. IN HCl was added to adjust the resulting solution to pH 4, followed by extraction with EtOAc. An organic layer was dried with Na₂SO₄, concentrated under reduced pressure and purified by using MPLC, thereby obtaining (*R*)-2-(4-(6-((4-chloro-2-fluorobenzyl)oxy)pyridin-2-yl)benzyl)-1-((tetrahydrofuran-3-yl)methyl)-1*H-*benzo[d]imidazole-6-carboxylic acid (40 mg, 0.070 mmol, 8.7%).

¹H NMR (400 MHz, CDCl₃) δ 8.19 (s, 1H), 8.10 (dd, *J =* 8.2, 1.4 Hz, 1H), 7.97 (d, *J=* 8.2 Hz, 2H), 7.89 (d, *J=* 8.2 Hz, 1H), 7.61 (t, *J=* 8.0 Hz, 1H), 7.44 (t, *J* = 8.2 Hz, 1H), 7.35 (d, *J =* 8.2 Hz, 2H), 7.31 (d, *J =* 7.8 Hz, 1H), 7.09 - 7.12 (m, 2H), 6.72 (d, *J* = 8.2 Hz, 1H), 5.50 (s, 2H), 4.49 (dd, *J=* 26.5, 16.0 Hz, 2H), 3.95 - 4.10 (m, 3H), 3.68 - 3.80 (m, 1H), 3.55 - 3.64 (m, 2H), 2.77 - 2.83 (m, 1H), 1.95 - 2.04 (m, 1H), 1.58 - 1.66 (m, 1H); LC-MS(ESI): 572.29 [M+H]⁺

### [Example 34: Preparation of (S)-2-(3-chloro-4-(6-((4-chloro-2-fluorobenzyl)oxy)pyridin-2-yl)benzyl)-1-((tetrahydrofuran-2-yl)methyl)-1H-benzo[d]imidazole-6-carboxylic acid]

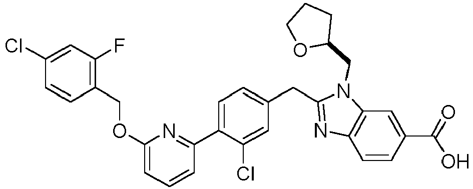

### (S)-2-(3-chloro-4-(6-((4-chloro-2-fluorobenzyl)oxy)pyridin-2-yl)benzyl)-1-((tetrahydrofuran-2-yl)methyl)-1H-benzo[d]imidazole-6-carboxylic acid

The (*S*)-4-amino-3-(((tetrahydrofuran-2-yl)methyl)amino)benzoate (138 mg, 0.55 mmol) obtained in Preparation Example 30 and the 2-(3-chloro-4-(6-((4-chloro-2-fluorobenzyl)oxy)pyridin-2-yl)phenyl)acetic acid (203 mg, 0.5 mmol) obtained in Preparation Example 56 were dissolved in 3 mL of DMF, and EDC (192 mg, 1.0 mmol) and HOBt (153 mg, 1.0 mmol) were added, followed by stirring for 16 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl (S)-4-(2-(3-chloro-4-(6-((4-chloro-2-fluorobenzyl)oxy)pyridin-2-yl)phenyl)acetamido)-3-(((tetrahydrofuran-2-yl)methyl)amino)benzoate. The intermediate was dissolved in 5 mL of acetic acid without additional purification and stirred for 2 hours at 120 °C. The acetic acid was concentrated under reduced pressure, and MPLC was performed, thereby obtaining an intermediate, methyl (S)-2-(3-chloro-4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylate. The intermediate was dissolved in 5 mL of THF, and 1.0 mL of IN NaOH was added to the reactants, followed by stirring for 15 hours at room temperature. IN HCl was added to adjust the resulting solution to pH 4, followed by extraction with EtOAc. An organic layer was dried with Na₂SO₄, concentrated under reduced pressure and purified by using MPLC, thereby obtaining (S)-2-(3-chloro-4-(6-((4-chloro-2-fluorobenzyl)oxy)pyridin-2-yl)benzyl)-1-((tetrahydrofuran-2-yl)methyl)-1*H-*benzo[d]imidazole-6-carboxylic acid (63 mg, 0.104 mmol, 20.8%).

¹H NMR (400 MHz, CDCl₃) δ 8.18 (d, *J* = 0.9 Hz, 1H), 8.07 (dd, *J* = 8.7, 1.4 Hz, 1H), 7.85 (d, *J* = 8.2 Hz, 1H), 7.63 (t, *J =* 8.0 Hz, 1H), 7.54 (d, *J* = 7.8 Hz, 1H), 7.39 - 7.46 (m, 2H), 7.22 - 7.28 (m, 2H), 7.08 - 7.13 (m, 2H), 6.77 (d, *J =* 8.2 Hz, 1H), 5.42 (s, 2H), 4.53 (dd, *J* = 36.6, 16.0 Hz, 2H), 4.30 (d, *J* = 11.9 Hz, 1H), 4.11 - 4.22 (m, 2H), 3.85 - 3.91 (m, 1H), 3.75 (q, *J=* 7.5 Hz, 1H), 2.04 - 2.12 (m, 1H), 1.84 - 1.94 (m, 2H), 1.53 - 1.62 (m, 1H); LC-MS(ESI): 606.28 [M+H]⁺

### [Example 35: Preparation of (S)-2-(4-(6-((4-chloro-2-fluorobenzyl)oxy)pyridin-2-yl)benzyl)-1-((tetrahydrofuran-3-yl)methyl)-1H-benzo[d]imidazole-6-carboxylic acid]

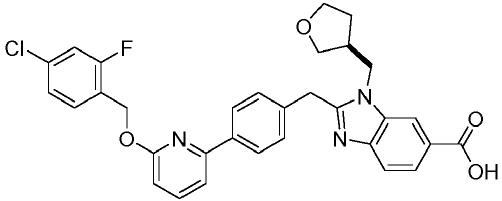

### (S)-2-(4-(6-((4-chloro-2-fluorobenzyl)oxy)pyridin-2-yl)benzyl)-1-((tetrahydrofuran-3-yl)methyl)-1H-benzo[d]imidazole-6-carboxylic acid

The methyl (*S*)-4-amino-3-(((tetrahydrofuran-3-yl)methyl)amino)benzoate (138 mg, 0.55 mmol) obtained in Preparation Example 58 and the 2-(4-(6-((4-chloro-2-fluorobenzyl)oxy)pyridin-2-yl)phenyl)acetic acid (186 mg, 0.5 mmol) obtained in Preparation Example 2 were dissolved in 2 mL of DMF, and EDC (192 mg, 1.0 mmol) and HOBt (153 mg, 1.0 mmol) were added, followed by stirring for 16 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl (*S*)-4-(2-(4-(6-((4-chloro-2-fluorobenzyl)oxy)pyridin-2-yl)phenyl)acetamido)-3-(((tetrahydrofuran-3-yl)methyl)amino)benzoate. The intermediate was dissolved in 5 mL of acetic acid without purification and stirred for 2 hours at 120 °C. The acetic acid was concentrated under reduced pressure, and MPLC was performed, thereby obtaining an intermediate, methyl (*S*)-2-(4-(6-((4-chloro-2-fluorobenzyl)oxy)pyridin-2-yl)benzyl)-l-((tetrahydrofuran-3-yl)methyl)-1*H*-benzo[d]imidazole-6-carboxylate. The intermediate was dissolved in 5 mL of THF, and 1.0 mL of IN NaOH was added to the reactants, followed by stirring for 15 hours at 40 °C. IN HCl was added to adjust the resulting solution to pH 4, followed by extraction with EtOAc. An organic layer was dried with Na₂SO₄, concentrated under reduced pressure and purified by using MPLC, thereby obtaining (*S*)-2-(4-(6-((4-chloro-2-fluorobenzyl)oxy)pyridin-2-yl)benzyl)-1-((tetrahydrofuran-3-yl)methyl)-1H-benzo[d]imidazole-6-carboxylic acid (49 mg, 0.086 mmol, 17%).

¹H NMR (500 MHz, CDCl₃) δ 8.19 (s, 1H), 8.10 (d, *J* = 8.5 Hz, 1H), 7.97 (d, *J =* 8.2 Hz, 2H), 7.89 (d, *J =* 8.5 Hz, 1H), 7.62 (t, *J =* 7.9 Hz, 1H), 7.45 (t, *J =* 8.1 Hz, 1H), 7.35 (d, *J =* 8.2 Hz, 2H), 7.31 (d, *J =* 7.3 Hz, 1H), 7.10 - 7.12 (m, 2H), 6.72 (d, *J* = 7.9 Hz, 1H), 5.50 (s, 2H), 4.49 (q, *J =* 15.8 Hz, 2H), 4.03 - 4.12 (m, 2H), 3.95 - 4.01 (m, 1H), 3.76 (dd, *J =* 15.3, 8.2 Hz, 1H), 3.56 - 3.64 (m, 2H), 2.65 - 2.88 (m, 1H), 1.96 - 2.05 (m, 1H), 1.63 (td, *J =* 12.4, 7.0 Hz, 1H); LC-MS(ESI): 572.29 [M+H]⁺

### [Example 36: Preparation of (S)-2-(4-(6-((2-fluoro-4-(trifluorobenzyl)benzyl)oxy)pyridin-2-yl)benzyl)-1-((tetrahydrofuran-3-yl)methyl)-1H-benzo[d]imidazole-6-carboxylic acid]

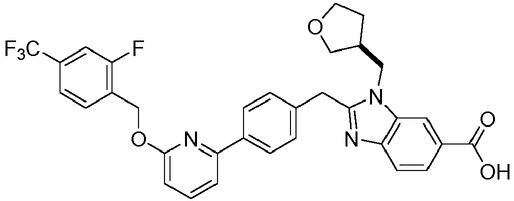

### (S)-2-(4-(6-((2-fluoro-4-(trifluoromethyl)benzyl)oxy)pyridin-2-yl)benzyl)-1-((tetrahydrofuran-3-yl)methyl)-1H-benzo[d]imidazole-6-carboxylic acid

The methyl (*S*)-4-amino-3-(((tetrahydrofuran-3-yl)methyl)amino)benzoate (138 mg, 0.55 mmol) obtained in Preparation Example 58 and 2-(4-(6-((2-fluoro-4-(trifluoromethyl)benzyl)oxy)pyridin-2-yl)phenyl)acetic acid (202 mg, 0.5 mmol) were dissolved in 2 mL of DMF, and EDC (192 mg, 1.0 mmol) and HOBt (153 mg, 1.0 mmol) were added, followed by stirring for 16 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl (S)-4-(2-(4-(6-((2-fluoro-4-(trifluoromethyl)benzyl)oxy)pyridin-2-yl)phenyl)acetamido)-3-(((tetrahydrofuran-3-yl)methyl)amino)benzoate. The intermediate was dissolved in 5 mL of acetic acid without additional purification, and stirred for 2 hours at 120 °C. The acetic acid was concentrated under reduced pressure, and MPLC was performed, thereby obtaining an intermediate, methyl (*S*)-2-(4-(6-((2-fluoro-4-(trifluoromethyl)benzyl)oxy)pyridin-2-yl)benzyl)-1-((tetrahydrofuran-3-yl)methyl)-1*H*-benzo[*d*]imidazole-6-carboxylate. The intermediate was dissolved in 5 mL of THF, and 0.7 mL of IN NaOH was added to the reactants, followed by stirring for 15 hours at 40 °C. IN HCl was added to adjust the resulting solution to pH 4, followed by extraction with EtOAc. An organic layer was dried with Na₂SO₄, concentrated under reduced pressure and purified by using MPLC, thereby obtaining (*S*)-2-(4-(6-((2-fluoro-4-(trifluoromethyl)benzyl)oxy)pyridin-2-yl)benzyl)-1-((tetrahydrofuran-3-yl)methyl)-1*H*-benzo[*d*]imidazole-6-carboxylic acid (23.2 mg, 0.038 mmol, 7.6%).

¹H NMR (500 MHz, CDCl₃) δ 8.18 (s, 1H), 8.09 (d, *J =* 8.5 Hz, 1H), 7.95 (d, *J =* 8.2 Hz, 2H), 7.87 (d, *J =* 8.5 Hz, 1H), 7.63 - 7.67 (m, 2H), 7.40 (d, *J =* 7.9 Hz, 1H), 7.32 - 7.36 (m, 4H), 6.76 (d, *J =* 7.9 Hz, 1H), 5.60 (s, 2H), 4.48 (dd, *J =* 29.6, 15.9 Hz, 2H), 4.03 - 4.11 (m, 2H), 3.98 (dd, *J* = 14.0, 8.2 Hz, 1H), 3.75 (dd, *J* = 15.3, 8.5 Hz, 1H), 3.56 - 3.64 (m, 2H), 2.73 - 2.91 (m, 1H), 1.92 - 2.04 (m, 1H), 1.61 - 1.66 (m, 1H); LC-MS(ESI): 606.35 [M+H]⁺

### [Example 37: Preparation of (S)-2-(3-chloro-4-(6-((4-chloro-2-fluorobenzyl)oxy)pyridin-2-yl)benzyl)-1-((tetrahydrofuran-3-yl)methyl)-1H benzo[d]imidazole-6-carboxylic acid]

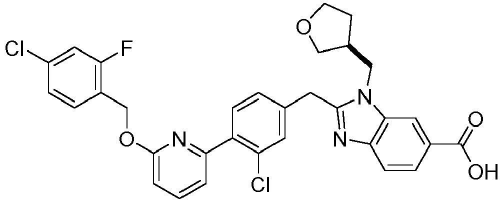

### (S)-2-(3-chloro-4-(6-((4-chloro-2-fluorobenzyl)oxy)pyridin-2-yl)benzyl)-1-((tetrahydrofuran-3-yl)methyl)-1H-benzo[d]imidazole-6-carboxylic acid

The methyl (*S*)-4-amino-3-(((tetrahydrofuran-2-yl)methyl)amino)benzoate (138 mg, 0.55 mmol) obtained in Preparation Example 58 and the 2-(3-chloro-4-(6-((4-chloro-2-fluorobenzyl)oxy)pyridin-2-yl)phenyl)acetic acid (203 mg, 0.5 mmol) obtained in Preparation Example 56 were dissolved in 2 mL of DMF, EDC (192 mg, 1.0 mmol) and HOBt (153 mg, 1.0 mmol) were added, followed by stirring for 16 hours at room temperature. After the reaction was completed, water was added, followed by extraction with EtOAc. An organic layer was dried with Na₂SO₄, concentrated under reduced pressure and purified by using MPLC, thereby obtaining an intermediate, methyl (*S*)-4-(2-(3-chloro-4-(6-((4-chloro-2-fluorobenzyl)oxy)pyridin-2-yl)phenyl)acetamido)-3-(((tetrahydrofuran-3-yl)methyl)amino)benzoate. The intermediate was dissolved in 5 mL of acetic acid without additional purification and stirred for 2 hours at 120 °C. The acetic acid was concentrated under reduced pressure, and MPLC was performed, thereby obtaining an intermediate, methyl (*S*)-2-(3-chloro-4-(6-((4-chloro-2-fluorobenzyl)oxy)pyridin-2-yl)benzyl)-1-((tetrahydrofuran-3-yl)methyl)-1H-benzo[*d*]imidazole-6-carboxylate. The resulting intermediate was dissolved in 5 mL of THF, and 0.6 mL of IN NaOH was added to the reactants, followed by stirring for 15 hours at 40 °C. IN HCl was added to adjust the resulting solution to pH 4, followed by extraction with EtOAc. An organic layer was dried with Na₂SO₄, concentrated under reduced pressure and purified by using MPLC, thereby obtaining (*S*)-2-(3-chloro-4-(6-((4-chloro-2-fluorobenzyl)oxy)pyridin-2-yl)benzyl)-1-((tetrahydrofuran-3-yl)methyl)-1H-benzo[d]imidazole-6-carboxylic acid (35.7 mg, 0.059 mmol, 12%).

¹H NMR (500 MHz, CDCl₃) δ 8.20 (s, 1H), 8.10 *(d, J =* 8.5 Hz, 1H), 7.87 (d, *J =* 8.5 Hz, 1H), 7.64 (t, *J =* 7.8 Hz, 1H), 7.56 (d, *J =* 7.9 Hz, 1H), 7.43 (t, *J =* 7.9 Hz, 1H), 7.40 (s, 1H), 7.24 - 7.26 (m, 2H), 7.08 - 7.12 (m, 2H), 6.78 *(d, J =* 8.2 Hz, 1H), 5.42 (s, 2H), 4.44 (dd, *J =* 30.7, 16.0 Hz, 2H), 4.11 - 4.14 (m, 2H), 4.03 (td, *J =* 8.3, 5.9 Hz, 1H), 3.79 (dd, *J =* 15.3, 8.5 Hz, 1H), 3.58 - 3.66 (m, 2H), 2.83 - 2.86 (m, 1H), 2.02 - 2.09 (m, 1H), 1.65 - 1.71 (m, 1H); LC-MS(ESI): 606.28 [M+H]⁺.

### [Experimental Example 1: Measurement of activity of GLP-1R agonist (Cell-based Luciferase assay)]

15,000 cells of a CHO-K1/hGLP-1R/CRE-luciferase cell line were seeded in a 96-well plate. A medium used herein was Ham's F-12 Nutrient medium. The cells were incubated for 18 hours in a 5% CO₂ incubator which was maintained at 37 °C, and then treated with drugs. Each drug dispensed at 9 different concentrations was dissolved in a DMEM/F-12 + 1% FBS medium, and the cell line was treated with each of 100 µl thereof. After four hours of incubation, a Bright-Glo^{™} luciferase assay system kit was used to add 30 µl of an assay reagent per well. A plate was stored at room temperature for 15 minutes, and then luminescence was measured using a SpectraMax M5 reader. The GLP-1R activity of the example compounds obtained by the experiment is shown in Table 1 in units of EC₅₀(nM).

**[Table 1]**

| **Example** | **EC₅₀** | **Example** | **EC₅₀** |
|---|---|---|---|
| 1 | 0.205 | 2 | 0.007 |
| 3 | 0.79 | 4 | 0.015 |
| 5 | 0.016 | 6 | 21 |
| 7 | 0.066 | 8 | 2.928 |
| 9 | 0.7 | 10 | 7.372 |
| 11 | 0.0746 | 12 | 0.0706 |
| 13 | 2.02 | 14 | >80 |
| 15 | 0.0035 | 16 | >80 |
| 17 | 0.357 | 18 | 0.438 |
| 19 | 1.328 | 20 | 0.121 |
| 21 | 0.3166 | 22 | 0.635 |
| 23 | 0.061 | 24 | 0.167 |
| 25 | 0.402 | 26 | 0.775 |
| 27 | 0.13 | 28 | 1.121 |
| 29 | 0.208 | 30 | 0.0327 |
| 31 | >80 | 32 | 3.426 |
| 33 | 0.365 | 34 | 0.087 |
| 35 | 0.784 | 36 | 2.086 |
| 37 | 1.344 | | |

As confirmed in Table 1, the compounds corresponding to Formula 1 according to the present invention, as excellent GLP-1 receptor agonists, were able to be confirmed as having an excellent effect. In addition, through drug metabolism-related experiments such as a Cytochrome P (CYP) suppression/induction experiment and a metabolic stability (MS) experiment and pharmacokinetics-related experiments, it can be confirmed that the compounds of Formula 1 have excellent DMPK profiles.

## Claims

1. A compound of Formula 1, an isomer or a pharmaceutically acceptable salt thereof: 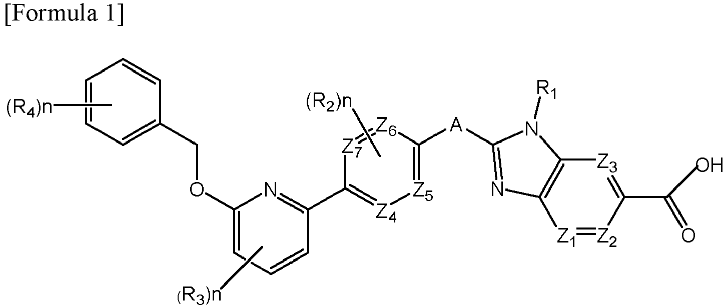 wherein,
A is -(CH₂)m-, -O- or -N(Rₐ)-, wherein m is an integer ranging from 1 to 3, and Rₐ is hydrogen or alkyl;
R₁ is (cycloalkyl)alkyl, (heterocycloalkyl)alkyl, (aryl)alkyl or (heteroaryl)alkyl;
R₂, R₃ or R₄ is each independently hydrogen, deuterium, halo, alkyl, alkoxy, alkylamine or a nitrile group;
n is an integer ranging from 1 to 4, wherein when n is an integer of 2 or more, each of R₂, R₃ and R₄ may be the same or different from each other; and
Z₁, Z₂, Z₃, Z₄, Z₅, Z₆ or Z₇ each independently represents CH, CF, CCl, CBr, CI or N;
wherein the alkyl, alkoxy, alkylamine, cycloalkyl, heterocycloalkyl, aryl, or heteroaryl is unsubstituted or substituted.

2. The compound, the isomer or the pharmaceutically acceptable salt thereof of claim 1, wherein the A is -CH₂-, -O- or -N(Rₐ)-, wherein the Rₐ is hydrogen or C₁₋₃ alkyl.

3. The compound, the isomer or the pharmaceutically acceptable salt thereof of claim 1, wherein the R₁ may be (C₃₋₈ cycloalkyl)C₁₋₃ alkyl, (4- to 10-membered heterocycloalkyl)C₁₋₃ alkyl, (C₆₋₁₀ aryl)alkyl or (4- to 10-membered heteroaryl)C₁₋₃ alkyl, wherein the heterocycloalkyl or heteroaryl is one containing 1 to 3 heteroatoms selected from the group consisting of N, O and S; and
the R₂, R₃ or R₄ is each independently hydrogen, deuterium, F, Cl, Br, I, C₁₋₃ alkyl, C₁₋₃ alkoxy, C₁₋₃ alkylamine or a nitrile group, n is an integer ranging from 1 to 3, wherein when n is an integer of 2 or more, each of R₂, R₃ and R₄ may be the same or different from each other.

4. The compound, the isomer or the pharmaceutically acceptable salt thereof of claim 1, wherein Z₁, Z₂, Z₃, Z₄, Z₅, Z₆ or Z₇ is each independently CH, CF or CCl.

5. The compound, the isomer or the pharmaceutically acceptable salt thereof of claim 1, wherein the R₁ is cyclopropylmethyl, cyclobutylmethyl, cyclopentylmethyl, cyclohexylmethyl, oxetanylmethyl, tetrahydrofuranylmethyl, tetrahydropyranylmethyl, oxazolylmethyl, benzyl, unsubstituted or propyl-substituted triazolylmethyl, or unsubstituted or ethyl-substituted imidazolylmethyl.

6. The compound, the isomer or the pharmaceutically acceptable salt thereof of claim 1, wherein the compound represented by Formula 1 is selected from the group consisting of the following compounds:
1] 2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
2] (*S*)-2-(4-(6-((4-chloro-2-fluorobenzyl)oxy)pyridin-2-yl)benzyl)-1-(oxetan-2-ylmethyl)-1H-benzo[*d*]imidazole-6-carboxylic acid;
3] (S)-2-(4-(6-((4-cyano-2-fluorobenzyl)oxy)pyridin-2-yl)benzyl)-1-(oxetan-2-ylmethyl)-1H-benzo[*d*]imidazole-6-carboxylic acid;
4] 2-(4-(6-((4-cyano-2-fluorobenzyl)oxy)pyridin-2-yl)benzyl)-1-((1-ethyl-1*H*-imidazol-5-yl)methyl)-1*H*-benzo[*d*]imidazole-6-carboxylic acid;
5] 2-(4-(6-((4-cyano-2-fluorobenzyl)oxy)pyridin-2-yl)phenoxy)-1-((1-ethyl-1*H*-imidazol-5-yl)methyl)-1*H*-benzo[*d*]imidazole-6-carboxylic acid;
6] (S)-2-(4-(6-((4-cyano-2-fluorobenzyl)oxy)pyridin-2-yl)phenoxy)-1-(oxetan-2-ylmethyl)-1H-benzo[*d*]imidazole-6-carboxylic acid;
7] (S)-2-(4-(6-((4-chloro-2-fluorobenzyl)oxy)pyridin-2-yl)phenoxy)-1-(oxetan-2-ylmethyl)-1H-benzo[*d*]imidazole-6-carboxylic acid;
8] 2-(4-(6-((4-chloro-2-fluorobenzyl)oxy)pyridin-2-yl)benzyl)-1-(oxazol-5-ylmethyl)-1H-benzo[*d*]imidazole-6-carboxylic acid;
9] 2-(4-(6-((4-cyano-2-fluorobenzyl)oxy)pyridin-2-yl)phenoxy)-1-(oxazol-5-ylmethyl)-1H-benzo[*d*]imidazole-6-carboxylic acid;
10] (S)-2-(4-(6-((4-chloro-2-fluorobenzyl)oxy)pyridin-2-yl)-2-fluorobenzyl)-1-(oxetan-2-ylmethyl)-1H-benzo[*d*]imidazole-6-carboxylic acid;
11] 2-(4-(6-((4-chloro-2-fluorobenzyl)oxy)pyridin-2-yl)-2-fluorobenzyl)-1-(oxazol-5-ylmethyl)-1H-benzo[*d*]imidazole-6-carboxylic acid;
12] (S)-2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
13] (R)-2-(4-(6-((4-chloro-2-fluorobenzyl)oxy)pyridin-2-yl)benzyl)-1-((tetrahydrofuran-2-yl)methyl)-1*H*-benzo[*d*]imidazole-6-carboxylic acid;
14] 2-(4-(6-((4-chloro-2-fluorobenzyl)oxy)pyridin-2-yl)benzyl)-1-((tetrahydro-2*H*-pyran-2-yl)methyl)-1H-benzo[*d*]imidazole-6-carboxylic acid;
15] (S)-2-(4-(6-((4-chloro-2-fluorobenzyl)oxy)pyridin-2-yl)-3-fluorobenzyl)-1-(oxetan-2-ylmethyl)-1H-benzo[*d*]imidazole-6-carboxylic acid;
16] (S)-2-(4-(6-((2-fluoro-4-(trifluoromethyl)benzyl)oxy)pyridin-2-yl)benzyl)-1-(oxetan-2-ylmethyl)-1H-benzo[d]imidazole-6-carboxylic acid;
17] (S)-2-(4-(6-((4-chloro-2-fluorobenzyl)oxy)pyridin-2-yl)-2-fluorophenoxy)-1-(oxetan-2-ylmethyl)-1H-benzo[d]imidazole-6-carboxylic acid;
18] (S)-2-(2-chloro-4-(6-((4-chloro-2-fluorobenzyl)oxy)pyridin-2-yl)phenoxy)-1-(oxetan-2-ylmethyl)-1H-benzo[d] imidazol-6-carboxylic acid;
19] (S)-2-(4-(6-((4-chloro-2-fluorobenzyl)oxy)pyridin-2-yl)-2,6-difluorophenoxy)-1-(oxetan-2-ylmethyl)-1H-benzo[d]imidazole-6-carboxylic acid;
20] (S)-2-(4-(6-((4-chloro-2-fluorobenzyl)oxy)pyridin-2-yl)-3-fluorophenoxy)-1-(oxetan-2-ylmethyl)-1H-benzo[d]imidazole-6-carboxylic acid;
21] 2-(4-(6-((2-fluoro-4-(trifluoromethyl)benzyl)oxy)pyridin-2-yl)benzyl)-1-(oxazol-5-ylmethyl)-1H-benzo[*d*]imidazole-6-carboxylic acid;
22] 2-(3-chloro-4-(6-((4-chloro-2-fluorobenzyl)oxy)pyridin-2-yl)benzyl)-1-(oxazol-5-ylmethyl)-1H-benzo[*d*]imidazole-6-carboxylic acid;
23] 2-(4-(6-((4-chloro-2-fluorobenzyl)oxy)pyridin-2-yl)-3-fluorobenzyl)-1-(oxazol-5-ylmethyl)-1H-benzo[*d*]imidazole-6-carboxylic acid;
24] 2-(4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid;
25] 2-(4-(6-((2-fluoro-4-(trifluoromethyl)benzyl)oxy)pyridin-2-yl)benzyl)-1-((4-propyl-4H-1,2,4-triazol-3-yl)methyl)-1H-benzo[d]imidazole-6-carboxylic acid;
26] 2-(3-chloro-4-(6-((4-chloro-2-fluorobenzyl)oxy)pyridin-2-yl)benzyl)-1-((4-propyl-4H-1,2,4-triazol-3-yl)methyl)-1H-benzo[d]imidazole-6-carboxylic acid;
27] 2-(4-(6-((4-chloro-2-fluorobenzyl)oxy)pyridin-2-yl)-3-fluorobenzyl)-1-((4-propyl-4H-1,2,4-triazol-3-yl)methyl)-1H-benzo[d]imidazole-6-carboxylic acid;
28] 2-(4-(6-((4-chloro-2-fluorobenzyl)oxy)pyridin-2-yl)phenoxy)-1-((tetrahydrofuran-2-yl)methyl)-1*H*-benzo[*d*]imidazole-6-carboxylic acid;
29] (S)-2-(4-(6-((2-fluoro-4-(trifluoromethyl)benzyl)oxy)pyridin-2-yl)benzyl)-1-((tetrahydrofuran-2-yl)methyl)-1H-benzo[d]imidazole-6-carboxylic acid;
30] (S)-2-(4-(6-((4-chloro-2-fluorobenzyl)oxy)pyridin-2-yl)-2-fluorobenzyl)-1-((tetrahydrofuran-2-yl)methyl)-1H-benzo[*d*]imidazole-6-carboxylic acid;
31] (S)-2-(4-(6-((4-chloro-2-fluorobenzyl)oxy)pyridin-2-yl)-2-fluorophenoxy)-1-((tetrahydrofuran-2-yl)methyl)-1H-benzo[*d*]imidazole-6-carboxylic acid;
32] (S)-2-(2-chloro-4-(6-((4-chloro-2-fluorobenzyl)oxy)pyridin-2-yl)phenoxy)-1 -((tetrahydrofuran-2-yl)methyl)- 1H-benzo[*d*]imidazole-6-carboxylic acid;
33] (R)-2-(4-(6-((4-chloro-2-fluorobenzyl)oxy)pyridin-2-yl)benzyl)-1-((tetrahydrofuran-3-yl)methyl)-1H-benzo[*d*]imidazole-6-carboxylic acid;
34] (S)-2-(3-chloro-4-(6-((4-chloro-2-fluorobenzyl)oxy)pyridin-2-yl)benzyl)-1-((tetrahydrofuran-2-yl)methyl)-1H-benzo[*d*]imidazole-6-carboxylic acid;
35] (S)-2-(4-(6-((4-chloro-2-fluorobenzyl)oxy)pyridin-2-yl)benzyl)-1-((tetrahydrofuran-3-yl)methyl)-1H-benzo[*d*]imidazole-6-carboxylic acid;
36] (S)-2-(4-(6-((2-fluoro-4-(trifluoromethyl)benzyl)oxy)pyridin-2-yl)benzyl)-1-((tetrahydrofuran-3-yl)methyl)-1H-benzo[d]imidazole-6-carboxylic acid; and
37] (S)-2-(3-chloro-4-(6-((4-chloro-2-fluorobenzyl)oxy)pyridin-2-yl)benzyl)-1-((tetrahydrofuran-3-yl)methyl)-1H-benzo[d]imidazole-6-carboxylic acid.

7. A pharmaceutical composition for preventing or treating a metabolic disease or a neurodegenerative disease, comprising the compound of any one of claims 1 to 6, or an isomer or a pharmaceutically acceptable salt thereof, and a pharmaceutically acceptable excipient, diluent or carrier.

8. The pharmaceutical composition of claim 7, wherein the metabolic disease is selected from the group consisting of diabetes, hypertension, hypoglycemia, hyperlipidemia (dyslipidemia), atherosclerosis, coronary artery disease, cardiovascular disorders, abnormal blood clotting, obesity, diabetic complications, diabetic retinopathy, liver disease, hepatobiliary disease, fatty liver, alcoholic steatohepatitis, chronic kidney disease, insulin resistance and impaired glucose tolerance.

9. The pharmaceutical composition of claim 7, wherein the neurodegenerative disease is selected from the group consisting of Parkinson's disease and Alzheimer's disease.

10. A method of preparing a compound of Formula 1 below, comprising:
1) reacting a compound of Formula 2 below with a compound of Formula 3 below in the presence of a palladium catalyst to obtain a compound of Formula 4 below;
2) reacting the compound of Formula 4 below obtained in step 1) with a compound of Formula 5 below in the presence of a palladium catalyst, followed by hydrolyzing the resulting reaction product to obtain a compound of Formula 6 below; and
3) coupling the compound of Formula 6 below obtained in step 2) with a compound of Formula 7 below, followed by condensing and hydrolyzing the resulting reaction product to obtain the compound of Formula 1 below: 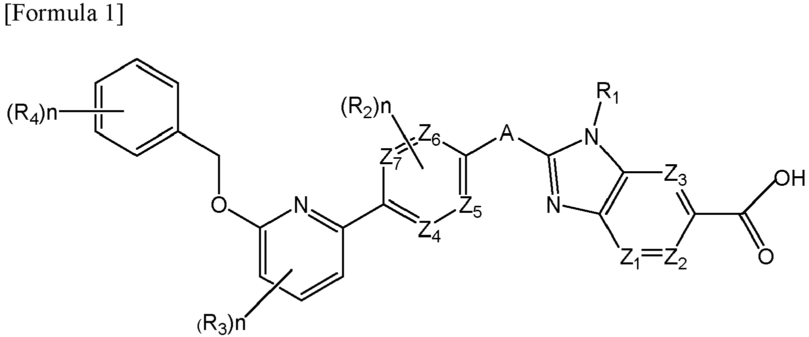 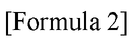 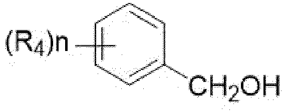 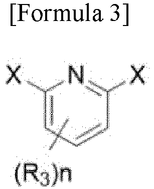 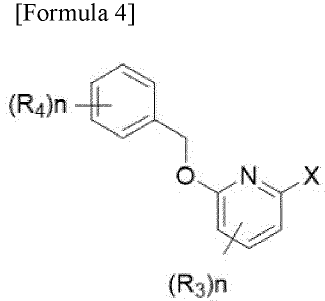 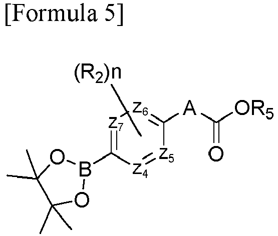 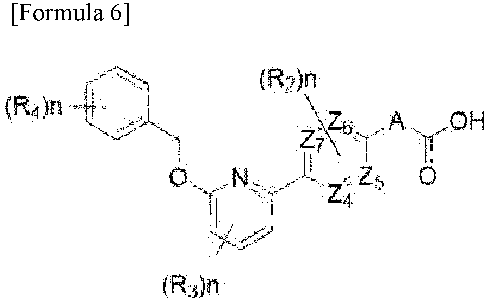 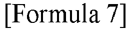 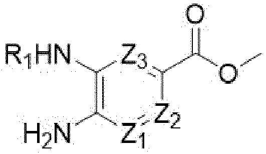 wherein,
A is carbon;
m, n, R₁, R₂, R₃, R₄, Z₁, Z₂, Z₃, Z₄, Z₅, Z₆ and Z₇ are the same as defined in claim 1;
R₅ is alkyl; and
X is halo.

11. A method of preparing a compound of Formula 1 below, comprising:
1') coupling a compound of Formula 4 below with a compound of Formula 8 below to obtain a compound of Formula 9 below; and
2') conducting a substitution reaction of the compound of Formula 9 below obtained in step 1') with a compound of Formula 10 below in the presence of a base, followed by hydrolyzing the resulting reaction product to obtain the compound of Formula 1 below: 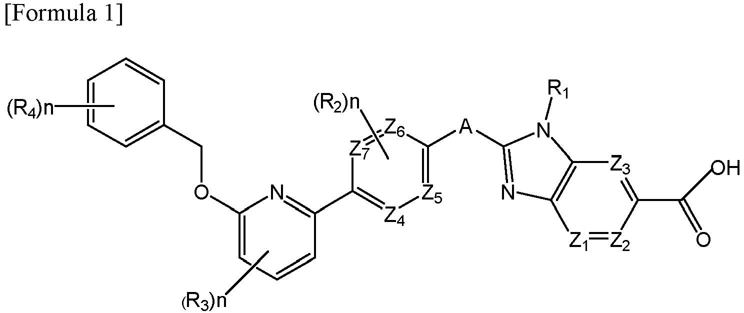 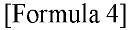 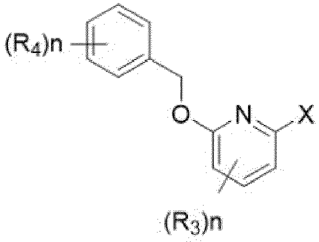 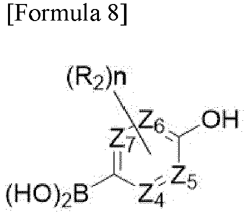 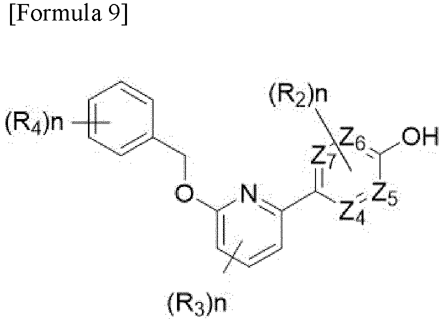 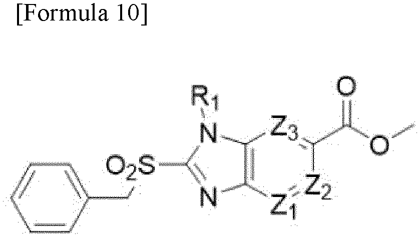 wherein,
A is oxygen;
n, R₁, R₂, R₃, R₄, Z₁, Z₂, Z₃, Z₄, Z₅, Z₆ and Z₇ are the same as defined in claim 1; and
X is halo.

12. The method of claim 11, wherein the compound of Formula 10 is prepared by a preparation method comprising following steps:
a) conducting a cyclization reaction of the compound of Formula 6 below, and then a substitution reaction with a benzyl halide to obtain a compound of Formula 11 below; and
b) conducting an oxidation reaction of the compound of Formula 11 obtained in step a) to obtain the compound of Formula 10;

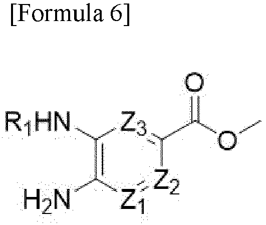

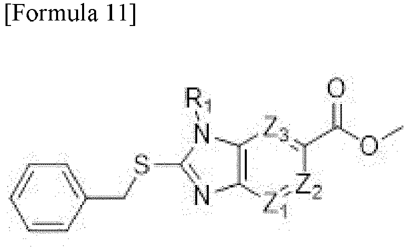

13. A method of preparing a compound of Formula 1 below, comprising:
1") coupling a compound of Formula 4 below with a compound of Formula 12 below to obtain a compound of Formula 13 below; and
2") conducting a substitution reaction of the compound of Formula 13 below obtained in step 1") with a compound of Formula 14 in the presence of base, followed by hydrolyzing the resulting reaction product to obtain the compound of Formula 1 below; 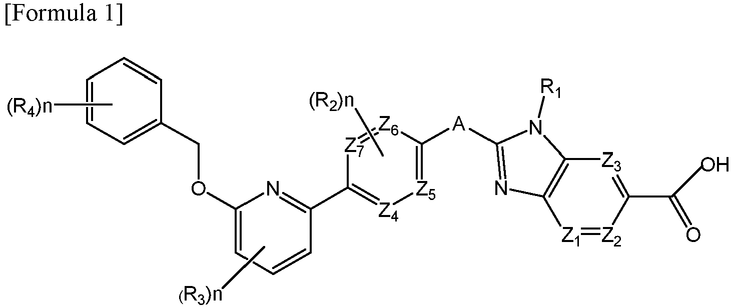 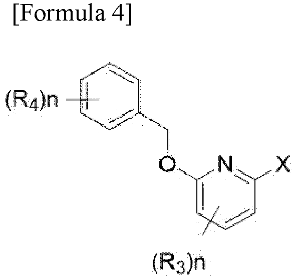 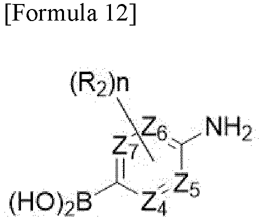 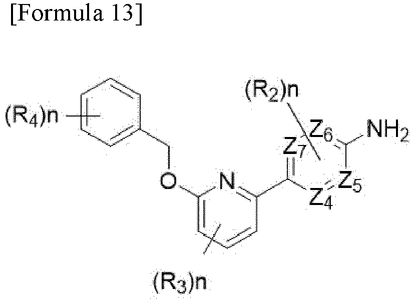 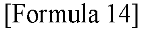 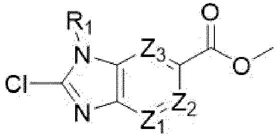
wherein,
A is nitrogen;
n, Rₐ, R₁, R₂, R₃, Z₁, Z₂, Z₃, Z₄, Z₅, Z₆ and Z₇ are the same as defined in claim 1; and
X is halo.
